(19) Europäisches Patentamt
European Patent Office
Office européen des brevets

(11) EP 3 766 977 A1

(12) EUROPEAN PATENT APPLICATION
published in accordance with Art. 153(4) EPC

(43) Date of publication:
**20.01.2021 Bulletin 2021/03**

(21) Application number: **19766785.0**

(22) Date of filing: **15.03.2019**

(51) Int Cl.:
***C12N 15/62*** *(2006.01)* ***A61K 35/12*** *(2015.01)*
***A61P 35/02*** *(2006.01)* ***C12N 5/10*** *(2006.01)*
***C12N 15/12*** *(2006.01)* ***C12N 15/13*** *(2006.01)*
***C12N 15/27*** *(2006.01)* ***C12N 15/85*** *(2006.01)*

(86) International application number:
**PCT/JP2019/010854**

(87) International publication number:
**WO 2019/177151 (19.09.2019 Gazette 2019/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.03.2018 JP 2018050266**

(71) Applicant: **Shinshu University**
**Matsumoto-shi, Nagano 390-8621 (JP)**

(72) Inventors:
- **NAKAZAWA, Yozo**
**Nagano 390-8621 (JP)**
- **HASEGAWA, Aiko**
**Nagano 390-8621 (JP)**
- **TANAKA, Miyuki**
**Nagano 390-8621 (JP)**
- **NAKANO, Shigeru**
**Nagano 399-8304 (JP)**
- **NARIMATSU, Shogo**
**Nagano 399-8304 (JP)**

(74) Representative: **Vossius & Partner**
**Patentanwälte Rechtsanwälte mbB**
**Siebertstrasse 3**
**81675 München (DE)**

# (54) GENETICALLY MODIFIED CELL AND PRODUCING METHOD THEREFOR

(57) It is intended to produce a cell expressing a mutant chimeric antigen receptor (CAR) having excellent cytotoxicity to target cells. The present invention provides a genetically modified cell having introduced thereinto a polynucleotide encoding a chimeric antigen receptor (CAR) protein having a target binding domain that specifically binds to a human granulocyte-macrophage colony stimulating factor (GM-CSF) receptor, a transmembrane domain, and an intracellular signaling domain, wherein the target binding domain is a mutant having the substitution of glutamic acid at position 21 in the amino acid sequence shown in SEQ ID NO: 1 with another amino acid.

Fig. 1

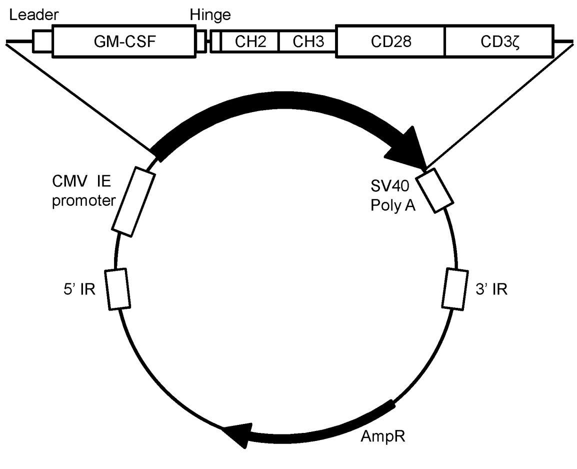


EP 3 766 977 A1

## Description

### Technical Field

The present invention relates to a genetically modified cell expressing a chimeric antigen receptor and useful in the field of adoptive immunotherapy, and also relates to a method for producing the cell.

More specifically, the present invention relates to a genetically modified cell having introduced thereinto a polynucleotide encoding a mutant chimeric antigen receptor specific for a human granulocyte-macrophage colony stimulating factor (GM-CSF) receptor, which has excellent targeted cytotoxicity, and methods for producing these cells and medicinal use of the cells.

### Background Art

Adoptive immunotherapy using T cells engineered to express a chimeric antigen receptor (CAR) (CAR-T) targeting a tumor-related antigen reportedly has a potent antitumor effect, and its development has advanced rapidly in recent years. Particularly, CD19 which is expressed on B cells is expressed on B lineage tumor cells without being expressed on hematopoietic stem cells and is therefore an ideal target of adoptive immunotherapy. Hence, CAR aimed at treating B cell tumor is under development. This CAR has exhibited remarkable effects in clinical trials and received great attention.

Meanwhile, CAR-T therapy for other tumor cells has been less developed. For example, juvenile myelomonocytic leukemia (JMML) or acute myelocytic leukemia (AML) is known as leukemia with poor prognosis, and the development of CAR-T therapy therefor is expected. Clinical trials have been conducted so far, targeting CD33, CD123, and the like. Also, a GM-CSF receptor-specific chimeric antigen receptor (hereinafter, referred to as GMR.CAR) has been reported, focusing on high expression of the GM-CSF receptor on the tumor cell surface of juvenile myelomonocytic leukemia (JMML) or acute myelocytic leukemia (AML) (Non Patent Literatures 1 and 2). However, clinically effective CAR-T therapy for AML has not yet been found, and sufficient effects of such therapy have not been confirmed in animal experiments using living organisms with engrafted tumor cells (Non Patent Literatures 3 and 4). A reason for the halting development of CAR-T therapy for JMML or AML is, for example, difficult selection of a target antigen.

In the case of using a myeloid antigen as a target antigen of CAR-T therapy, this antigen is expressed not only in tumor cells but in normal cells of myelocyte series, dendritic cells, and the like and it is known that it may cause problems associated with on-target/off-tumor reaction (Non Patent Literatures 3, 4, and 5). Hence, there is a demand for search for novel target antigens for the diseases and provision of CAR having safety and excellent *in vivo* effects.

### Citation List

#### Non Patent Literature

Non Patent Literature 1: Journal of Hematology & Oncology, 2016, 9:27, DOI 10.1186/s13045-016-0256-3
Non Patent Literature 2: The 22nd Annual Meeting JSGCT2016: Japan Society of Gene and Cell Therapy Program and Abstracts, 2016. 07. 13, P0-70
Non Patent Literature 3: Journal of Hematology & Oncology, 2017, 10: 151, DOI 10.1186/s13045-017-0519-7
Non Patent Literature 4: Blood, 2013, 122 (18), 3138-3148
Non Patent Literature 5: Blood Cancer Journal, 2016, 6, e458; doi: 10.1038/bcj.2016.61

### Summary of Invention

#### Technical Problem

As described above, CAR-T technology targeting a myeloid antigen or the like is still under development. Thus, it is desired to develop CAR that has high safety and exerts excellent *in vivo* effects.

#### Solution to Problem

The present inventors have conducted diligent studies to solve the problems described above. As a result, the present inventors have found that a T cell having introduced thereinto a gene for a GM-CSF receptor-specific mutant chimeric antigen receptor, in which a mutant GM-CSF having the substitution of glutamic acid at amino acid residue 21 of a GM-CSF polypeptide with other amino acid is used as a target binding domain (hereinafter, referred to as "mutant GMR.CAR"), (hereinafter, this T cell is referred to as a "mutant GMR.CAR-T cell") has both excellent cytotoxic activity and safety in the treatment of AML, etc.

Specifically, the present invention is as follows.

[1] A genetically modified cell having introduced thereinto a polynucleotide encoding a chimeric antigen receptor (CAR) protein comprising a target binding domain that specifically binds to a human granulocyte-macrophage colony stimulating factor (GM-CSF) receptor, a transmembrane domain, and an intracellular signaling domain, wherein the target binding domain is a mutant polypeptide having a sequence in which glutamic acid at position 21 in the amino acid sequence shown in SEQ ID NO: 1 is substituted with another amino acid.
[2] The cell according to [1] above, wherein the cell expresses the CAR protein that binds to a GM-CSF receptor on its cell membrane.
[3] The cell according to [1] or [2] above, wherein the CAR protein further comprises a co-stimulatory domain and/or an extracellular spacer domain.
[4] The cell according to any of [1] to [3] above, wherein the intracellular signaling domain is human CD3ζ.
[5] The cell according to [3] or [4] above, wherein the co-stimulatory domain is human CD28 or human 4-1BB.
[6] The cell according to any of [3] to [5] above, wherein the extracellular spacer domain comprises a hinge, CH2 and/or CH3 region of human IgG1 or a portion thereof, and/or an artificial spacer sequence.
[7] The cell according to any of [1] to [6] above, wherein the target binding domain is a mutant polypeptide having a sequence in which glutamic acid at position 21 in the amino acid sequence shown in SEQ ID NO: 1 is substituted with arginine or lysine.
[8] A method for producing a CAR protein expressing cell, comprising introducing a polynucleotide encoding a chimeric antigen receptor (CAR) protein that specifically binds to a human GM-CSF receptor to a cell using a vector, wherein the protein comprises an amino acid sequence in which glutamic acid at position 21 in the amino acid sequence shown in SEQ ID NO: 1 is substituted with another amino acid.
[9] A vector comprising a polynucleotide encoding a chimeric antigen receptor (CAR) protein that specifically binds to a human GM-CSF receptor, wherein the protein comprises an amino acid sequence in which glutamic acid at position 21 in the amino acid sequence shown in SEQ ID NO: 1 is substituted with another amino acid.
[10] A therapeutic agent for a disease involving a GM-CSF receptor expressing cell, comprising the cell according to any of [1] to [7] above.
[11] A pharmaceutical composition comprising the therapeutic agent according to [10] above and a pharmaceutically acceptable carrier.
[12] The therapeutic agent according to [10] above or the composition according to [11] above, wherein the disease involving a GM-CSF receptor expressing cell is selected from juvenile myelomonocytic leukemia (JMML), acute myelocytic leukemia (AML), glioma, neuroblastoma, glioblastoma, brain tumor, colorectal adenocarcinoma, prostate cancer, kidney cancer, melanoma and small cell lung cancer.

The present specification encompasses the contents disclosed in Japanese Patent Application No. 2018-050266 on which the priority of the present application is based.

Advantageous Effects of Invention

The GMR.CAR-T cell of the present invention is excellent in safety and exhibits a potent cytotoxic effect on tumor cells in adoptive immunotherapy targeting various cells expressing a GM-CSF receptor on a cell surface including acute myelocytic leukemia (AML), etc.

Brief Description of Drawings

[Figure 1] Figure 1 shows a vector map of CAR001.
[Figure 2] Figure 2 shows an exemplary vector map of GMR.CAR having a modified spacer domain (CH2CH3 partial deletion mutant, GMR.CAR dCH2CH3).
[Figure 3] Figure 3 shows an exemplary vector map of GMR.CAR having a modified spacer domain (CH2CH3 partial deletion-(G4S)3 insertion mutant, GMR.CAR dCH2CH3+(G4S)3).
[Figure 4] Figure 4 shows an exemplary vector map of (E21R)GMR.CAR having a modified spacer domain (CH2CH3 partial deletion mutant, E21RGMR.CAR dCH2CH3).
[Figure 5] Figure 5 shows an exemplary vector map of (E21K)GMR.CAR having a modified spacer domain (CH2CH3 partial deletion mutant, E21KGMR.CAR dCH2CH3).

[Figure 6] Figure 6 shows an exemplary vector map of (E21R)GMR.CAR having a modified spacer domain (CH2CH3 partial deletion-(G4S)3 insertion mutant, E21RGMR.CAR dCH2CH3+(G4S)3).

[Figure 7] Figure 7 shows an exemplary vector map of (E21K)GMR.CAR having a modified spacer domain (CH2CH3 partial deletion-(G4S)3 insertion mutant, E21KGMR.CAR dCH2CH3+(G4S)3).

[Figure 8] Figure 8 shows results of GM-CSF expression analysis on day 14 of CAR-T cells having introduced thereinto a polynucleotide encoding CAR (CAR-T 001 to CAR-T 008).

[Figure 9] Figure 9 shows the anti-tumor cell activity % of CAR-T 001 to CAR-T 008 against THP-1 cells.

[Figure 10] Figure 10 shows results of GM-CSF expression analysis on day 14 of CAR-T cells having introduced thereinto a polynucleotide encoding CAR (CAR-T 001-A and CAR-T 009 to CAR-T 014).

[Figure 11] Figure 11 shows the anti-tumor cell activity % of CAR-T 001-A and CAR-T 009 to CAR-T 014 against THP-1 cells.

[Figure 12] Figure 12 shows results of GM-CSF expression analysis on day 14 of CAR-T cells having introduced thereinto a polynucleotide encoding CAR (CAR-T 010-A to CAR-T 014-A).

[Figure 13] Figure 13 shows the sustained tumor cell killing ability of CAR-T 010-A to CAR-T 014-A against THP-1 cells at an E:T ratio of 1:1.

[Figure 14] Figure 14 shows the sustained tumor cell killing ability of CAR-T 010-A to CAR-T 014-A against MV4-11 cells at an E:T ratio of 1:1.

[Figure 15] Figure 15 shows the sustained tumor cell killing ability of CAR-T 010-A to CAR-T 014-A against Kasumi-1 cells at an E:T ratio of 1:1.

[Figure 16] Figure 16 shows the sustained tumor cell killing ability of CAR-T 010-A to CAR-T 014-A against shinAML-1 cells at an E:T ratio of 1:1.

[Figure 17] Figure 17 shows tumor cell numbers in wells in Figures 13 to 16.

[Figure 18] Figure 18 shows results of GM-CSF expression analysis on day 14 of CAR-T cells having introduced thereinto a polynucleotide encoding CAR (CAR-T 001-B and CAR-T 009-B to CAR-T 014-B).

[Figure 19] Figure 19 shows the relative ratio of viable tumor cell number to MV4-11 cells and the relative ratio of viable cell number to peripheral blood cells at E:T ratios of 1:1 and 1:10.

[Figure 20] Figure 20 shows a sigmoid curve drawn from points plotted at respective E:T ratios for healthy human-derived bone marrow cells.

[Figure 21] Figure 21 shows a sigmoid curve drawn from points plotted at respective E:T ratios for MV4-11 cells.

[Figure 22] Figure 22 shows the margin of safety calculated on the basis of the IC50 value calculated from Figure 20 and the EC50 value calculated from Figure 21.

[Figure 23] Figure 23 shows results of GM-CSF expression analysis on day 14 of CAR-T cells having introduced thereinto a polynucleotide encoding CAR (CAR-T 009-C, CAR-T 011-C and CAR-T 012-C).

[Figure 24] Figure 24 shows experimental results about CAR-T 009-C, CAR-T 011-C and CAR-T 012-C for THP-1 cancer-bearing mouse models.

[Figure 25] Figure 25 shows results of GM-CSF expression analysis on day 14 of CAR-T cells having introduced thereinto a polynucleotide encoding CAR (CAR-T 009-D to CAR-T 014-D).

[Figure 26] Figure 26 shows experimental results about CAR-T 009-D, CAR-T 011-D and CAR-T 012-D for MV4-11 cancer-bearing mouse models.

[Figure 27] Figure 27 shows experimental results about CAR-T 010-D, CAR-T 013-D and CAR-T 014-D for MV4-11 cancer-bearing mouse models.

[Figure 28] Figure 28 shows results of quantitative PCR for CAR-T 009-D to CAR-T 014-D in a tumor in peripheral blood on day 52 (ND denotes not detected).

[Figure 29] Figure 29 shows results of GM-CSF expression analysis on day 14 of CAR-T cells having introduced thereinto a polynucleotide encoding CAR (CAR-T 001-C, CAR-T 014-F, and comparative control CD19 T cell).

[Figure 30] Figure 30 shows experimental results about CAR-T 001-C and CAR-T 014-F for MV4-11 cancer-bearing mouse models.

[Figure 31] Figure 31 shows results of GM-CSF expression analysis on day 14 of CAR-T cells having introduced thereinto a polynucleotide encoding CAR (CAR-T 014-E).

## Description of Embodiments

Hereinafter, embodiments of the present invention will be described in detail.

As used herein, the "chimeric antigen receptor (CAR)" refers to a modified receptor, which can impart its target specificity to cells such as T cells (e.g., T cells such as naive T cells, stem cell memory T cells, central memory T cells, effector memory T cells or a combination thereof). CAR is also known as an artificial T cell receptor, a chimeric T cell receptor or a chimeric immunoreceptor.

As used herein, the "domain" refers to a region within a polypeptide which is folded into a specific structure, independently of other regions.

As used herein, the "polynucleotide" includes, but is not limited to, a natural or synthetic DNA and RNA, for example, genomic DNA, cDNA (complementary DNA), mRNA (messenger RNA), rRNA (ribosome RNA), shRNA (small hairpin RNA), snRNA (small nuclear RNA), snoRNA (small nucleolar RNA), miRNA (microRNA), and/or tRNA.

As used herein, the term "encode (encodes, encoding)" means that a predetermined nucleotide sequence has a code for information of the amino acid sequence of a predetermined protein or (poly)peptide, as ordinarily used in the art, and both a sense strand and an antisense strand are used herein in the context of "encoding".

As described above, the present invention provides a genetically modified cell having introduced thereinto a polynucleotide encoding a chimeric antigen receptor (CAR) protein comprising a target binding domain that specifically binds to a human granulocyte-macrophage colony stimulating factor (GM-CSF) receptor, a transmembrane domain, and an intracellular signaling domain, wherein the target binding domain is a mutant polypeptide having a sequence in which glutamic acid at position 21 in the amino acid sequence shown in SEQ ID NO: 1 is substituted with another amino acid.

As used herein, the phrase "comprising (having) ... sequence" may encompass the case of comprising a sequence other than the described sequence and also encompass the case of comprising only the described sequence (i.e., "consisting of ... sequence").

The CAR protein of the present invention comprises a "target binding domain" that specifically binds to a human GM-CSF receptor. The target (GM-CSF receptor) binding domain shows a binding ability specific to the GM-CSF receptor, and can cause an immune response specific to target cells expressing the GM-CSF receptor on the cell surface.

Thus, a polypeptide having the substitution of glutamic acid at amino acid residue 21 of GM-CSF, which is a cytokine that binds to a GM-CSF receptor and has the amino acid sequence of SEQ ID NO: 1, with another amino acid can be used as the GM-CSF receptor binding domain. Alternatively, a mutant also having the substitution of other amino acid residue moieties can be used as long as the mutant retains the binding ability to the GM-CSF receptor. Furthermore, a fragment of any of these polypeptides, i.e., a binding fragment, may be used as long as the fragment retains the binding ability to the GM-CSF receptor.

The polypeptide having the substitution of the amino acid at amino acid residue 21 of GM-CSF with another amino acid is not particularly limited as long as the polypeptide retains binding activity against the GM-CSF receptor. For example, substitution with arginine (amino acid sequence of SEQ ID NO: 2), substitution with lysine (amino acid sequence of SEQ ID NO: 3), substitution with histidine (amino acid sequence of SEQ ID NO: 4), substitution with aspartic acid (amino acid sequence of SEQ ID NO: 5), substitution with serine (amino acid sequence of SEQ ID NO: 6), substitution with alanine (amino acid sequence of SEQ ID NO: 7), or substitution with phenylalanine (amino acid sequence of SEQ ID NO: 8) can be used.

In a preferred aspect, a polypeptide having the substitution of the amino acid residue 21 of GM-CSF with a basic amino acid arginine, lysine, or histidine can be used.

In a more preferred aspect, a polypeptide having the substitution of the amino acid residue 21 of GM-CSF with arginine or lysine can be used.

In this context, the phrase "having the ability to bind to the human GM-CSF receptor" means that the association constant to the human GM-CSF receptor is, for example, 1 to 1000 nM or less. The binding ability can be relatively weak as compared with antigen-antibody binding ability.

Any target binding domain can be used instead of the polypeptide as long as the domain retains the binding ability to the human GM-CSF receptor. For example, a single-chain antibody (scFv) that is a single-chain polypeptide derived from an antibody retaining the binding ability to the GM-CSF receptor can be used, and an antibody polypeptide having linked Fv regions of immunoglobulin heavy chain (H chain) and light chain (L chain) fragments can be used as the target binding domain.

Furthermore, a ligand that specifically binds to the receptor protein expressed on target cells, for example, an affibody, a ligand-binding domain derived from a natural receptor, a soluble protein/peptide ligand of a receptor (e.g., on tumor cells), a peptide, and a vaccine for promoting an immune response, may be used.

The CAR protein herein may optionally comprise an "extracellular spacer domain". The extracellular spacer domain is desirably a sequence promoting CAR-antigen binding to facilitate signal transduction into a cell. For example, an Fc fragment of an antibody or a fragment or a derivative thereof, a hinge region of an antibody or a fragment or a derivative thereof, a CH2 region of an antibody, a CH3 region of an antibody, an artificial spacer sequence, or a combination thereof can be used.

In one aspect of the present invention, as the extracellular spacer domain, (i) hinge, CH2 and CH3 regions of IgG4, (ii) a hinge region of IgG4, (iii) a hinge and CH2 of IgG4, (iv) a hinge region of CD8a, (v) a hinge, CH2 and CH3 regions of IgG1, (vi) a hinge region of IgG1, or (vii) a hinge and CH2 of IgG1, or a combination thereof can be used. For example, the following region having the amino acid sequence (SEQ ID NO: 10) encoded by the nucleotide sequence represented by SEQ ID NO: 9 can be suitably used as the hinge region of IgG1, though the hinge region is not limited thereto.
**UniProt No.: P01857 (99-110)**

EPKSCDKTHTCP PCDPA EPKSPDKTHTCP
**Hinge Spacer Hinge**

As the CH2 region of IgG1, the region having the amino acid sequence (SEQ ID NO: 12) encoded by the nucleotide sequence represented by SEQ ID NO: 11 can be suitably used. As the CH3 region of IgG1, the region having the amino acid sequence (SEQ ID NO: 14) encoded by the nucleotide sequence represented by SEQ ID NO: 13 can be suitably used.

In a preferred aspect, human IgG1 hinge, CH2 and CH3 regions or a part thereof can be used as the extracellular spacer domain.

In a preferred aspect, (i) a human IgG1 hinge region (SEQ ID NO: 10) alone, (ii) a combination of a human IgG1 hinge region (SEQ ID NO: 10), CH2 region (SEQ ID NO: 12) and CH3 region (SEQ ID NO: 14), (iii) a combination of a human IgG1 hinge region (SEQ ID NO: 10) and CH3 region (SEQ ID NO: 14), or (iv) a CH3 region (SEQ ID NO: 14) alone can be used as the extracellular spacer domain.

In one aspect of the present invention, as the artificial spacer sequence to be used as the extracellular spacer domain, a spacer sequence represented by the formula (G4S)n can be used. In the formula, n represents an integer of 1 to 10, and preferably n = 3. For example, the spacer sequence represented by SEQ ID NO: 15 can be suitably used. The spacer having such a spacer sequence is sometimes referred to as a peptide linker. Peptide linkers suitably used in the art can be appropriately used in the present invention. In this case, the constitution and chain length of the peptide linker can be properly selected as long as the function of the resulting CAR protein is not impaired.

In a further preferred aspect, a combination of human IgG1 hinge region (SEQ ID NO: 10) and a spacer sequence (SEQ ID NO: 15) represented by (G4S)3 can be used as the extracellular spacer domain.

The extracellular spacer domain can be appropriately selected from those listed above or further modified based on common technical knowledge in the art, and used for the present invention.

Nucleotide sequences encoding the respective amino acid sequences of domains are ligated, inserted into a vector, and expressed in a host cell, such that the extracellular spacer domain can exist between the target binding domain and the transmembrane domain. Alternatively, the extracellular spacer domain can be modified using a polynucleotide encoding a plasmid CAR protein produced in advance as a template.

The modification of the extracellular spacer domain is useful when considering, for example, improvement of a CAR gene expression rate in host cells of a CAR-T cell having a polynucleotide encoding CAR introduced thereinto, signal transduction, aging of a cell, distribution in a tumor, antigen recognition or influence on *in vivo* activity.

The CAR protein of the present invention resides on a cell membrane and comprises an extracellular domain comprising a target binding domain and optionally an extracellular spacer domain, a transmembrane domain, and an intracellular domain comprising an intracellular signaling domain and optionally a co-stimulatory domain. As well known in the art, the "transmembrane domain" is a domain having an affinity to a lipid bilayer constituting cell membrane, whereas the extracellular domain and intracellular domain are both hydrophilic domains. The transmembrane domain in the GMR.CAR of the present invention is not particularly limited as long as the CAR protein can reside on the cell membrane without impairing the functions of the target binding domain and the intracellular signaling domain. A polypeptide derived from the same protein as that of a co-stimulatory domain mentioned later may function as the transmembrane domain.

In one aspect of the present invention, a transmembrane domain such as CD28, CD3$\varepsilon$, CD8$\alpha$, CD3, CD4 or 4-1BB can be used as the transmembrane domain.

In a preferred aspect, human CD28 (Uniprot No.: P10747 (153-179)) can be used as the transmembrane domain. More specifically, human CD28 having the amino acid sequence (SEQ ID NO: 17) encoded by the nucleotide sequence represented by SEQ ID NO: 16 (NCBI Accession No.: NM_006139.3 (679-759)) can be suitably used as the transmembrane domain.

The CAR protein of the present invention may optionally comprise a "co-stimulatory domain". The co-stimulatory domain specifically binds to a co-stimulation ligand, thereby mediating cellular co-stimulation responses such as growth of CAR-T cells, cytokine production, functional differentiation and target cell death, but the cellular co-stimulation responses are not limited thereto.

In one aspect of the present invention, the co-stimulatory domain that can be used include, for example, CD27, CD28, 4-1BB (CD137), CD134 (OX40), Dap10, CD27, CD2, CD5, CD30, CD40, PD-1, ICAM-1, LFA-1 (CD11a/CD18), TNFR-1, TNFR-II, Fas, and Lck.

In a preferred aspect, for example, human CD28 (Uniprot No.: P10747 (180-220)) or 4-1BB (GenBank:

U03397.1) can be used as the co-stimulatory domain. More specifically, those having the amino acid sequence (SEQ ID NO: 19) encoded by the nucleotide sequence represented by SEQ ID NO: 18 (NCBI Accession No.: NM_006139.3 (760-882) can be suitably used as the co-stimulatory domain.

The CAR protein of the present invention comprises an "intracellular signaling domain". The intracellular signaling domain transmits a signal required for the effector function of immune cells.

In one aspect of the present invention, as the intracellular signaling domain, for example, a human CD3ζ chain, FcγRIII, FcεRI, a cytoplasmic end of an Fc receptor, a cytoplasmic receptor having an immunoreceptor tyrosine activation motif (ITAM) or a combination thereof can be used.

In a preferred aspect, as the intracellular signaling domain, a human CD3ζ chain (e.g., nucleotides 299-637 of NCBI Accession No. NM_000734.3) can be used. More specifically, a human CD3ζ chain having the amino acid sequence (SEQ ID NO: 21) encoded by the nucleotide sequence represented by SEQ ID NO: 20 can be suitably used as the intracellular signaling domain.

The polynucleotide of interest can be easily produced in accordance with a routine method. The Nucleotide sequences encoding the amino acid sequences of individual domains can be obtained from NCBI RefSeq ID or GenBank Accession numbers. The polynucleotide of the present invention can be produced using standard molecular biological and/or chemical procedures. For example, nucleic acids can be synthesized based on these nucleotide sequences. Also, DNA fragments obtained through the polymerase chain reaction (PCR) from a cDNA library can be combined to produce the polynucleotide of the present invention.

Accordingly, the polynucleotide encoding the GMR.CAR of the present invention can be produced by ligating polynucleotides encoding aforementioned respective domains. A GMR.CAR-T cell can be produced by introducing this polynucleotide into a proper cell. Alternatively, the GMR.CAR can be produced by using a polynucleotide encoding a known CAR protein having the same structural components except the target binding domain, as a template, and recombining the target binding domain in accordance with a routine method.

Furthermore, depending on the purpose, one or more domains, for example, the extracellular spacer domain, can be modified by use of inverse PCR (iPCR), etc. using a polynucleotide encoding a known CAR protein as a template. The technique for modifying the extracellular spacer domain is described in, for example, Oncoimmunology, 2016, Vol. 5, No. 12, e1253656.

In one embodiment of the present invention, a genetically modified cell having introduced thereinto a polynucleotide encoding a chimeric antigen receptor (CAR) protein comprising a target binding domain that specifically binds to a human granulocyte-macrophage colony stimulating factor (GM-CSF) receptor and comprising the amino acid sequence of SEQ ID NO: 2 or SEQ ID NO: 3, a transmembrane domain comprising the amino acid sequence of SEQ ID NO: 17, and an intracellular signaling domain comprising the amino acid sequence of SEQ ID NO: 21 can be used.

In one embodiment of the present invention, a genetically modified cell having introduced thereinto a polynucleotide encoding a chimeric antigen receptor (CAR) protein comprising a target binding domain that specifically binds to a human granulocyte-macrophage colony stimulating factor (GM-CSF) receptor and comprising the amino acid sequence of SEQ ID NO: 2 or SEQ ID NO: 3, an extracellular spacer domain comprising the amino acid sequence of SEQ ID NO: 10, SEQ ID NO: 12, SEQ ID NO: 14 and/or SEQ ID NO: 15, a transmembrane domain comprising the amino acid sequence of SEQ ID NO: 17, and an intracellular signaling domain comprising the amino acid sequence of SEQ ID NO: 21 can be used.

The phrase "comprising the amino acid sequence of SEQ ID NO: 10, SEQ ID NO: 12, SEQ ID NO: 14 and/or SEQ ID NO: 15" means comprising any one of or any combination of two or more of the amino acid sequences of SEQ ID NO: 10, SEQ ID NO: 12, SEQ ID NO: 14 and SEQ ID NO: 15. The same holds true for the description below in the present specification.

As the cell into which the above polynucleotide is to be introduced, cells derived from a mammal, such as human, or T cells derived from a non-human mammal, such as monkey, mouse, rat, pig, cow and dog, or a cell population comprising the T cells can be used. For example, cells collected, isolated, purified or induced from a body fluid, a tissue or an organ, such as blood (peripheral blood, umbilical cord blood, etc.) or bone marrow can be used. Peripheral blood mononuclear cells (PBMCs), immune cells [dendritic cells, B cells, hematopoietic stem cells, macrophages, monocytes, NK cells or hemocyte cells (neutrophils and basophils)], umbilical blood mononuclear cells, fibroblasts, preadipocytes, stem cells, skin keratinocytes, mesenchymal cells, fatty liver cells, various cancer cell lines or neural stem cells can be used.

In a preferred embodiment of the present invention, for example, cells releasing a cytotoxic protein (perforin, granzyme, etc.) can be used. More specifically, for example, T cells, precursor T cells (hematopoietic stem cells, lymphocyte precursor cells, etc.), NK cells, NK-T cells or a cell population containing these cells can be used. Further, cells capable of differentiating into these cells include various stem cells such as ES cells and iPS cells. T cells include CD8-positive T cells, CD4-positive T cells, regulatory T cells, cytotoxic T cells or tumor-infiltrating lymphocytes. The cell population containing T cells and precursor T cells includes PBMCs. The above cells may be collected from a living organism, obtained by the expansion culture of the cells, or established as a cell line. In the case of transplanting the produced cells expressing CAR or cells differentiated from the cells into a living organism, it is desirable to introduce a nucleic acid into cells collected from the living organism itself or a living organism of the same species thereas.

As T cells for use in adoptive immunotherapy to which the polynucleotide is to be introduced for the genetically modified cell of the present invention, T cells expected to have a sustained antitumor effect, for example, stem cell memory T cells, can be used. The stem cell memory T cells can be analyzed according to a routine method and easily confirmed as described in, for example, Yang Xu, et al., Blood. 2014; 123: 3750-3759.

In one embodiment of the present invention, CD45R0-, CD62L+, CD45RA+ and CCR7+ T cells transduced with a CAR expression plasmid can be used as cells to which the polynucleotide described above is to be introduced.

In a more preferred embodiment of the present invention, cells comprising at least 50% of CD8+, CD45RA+, CCR7+, and CD62L+ cells can be used as cells to which the polynucleotide described above is introduced.

A method for introducing the polynucleotide for the production of the genetically modified cell is not particularly limited as long as it is ordinarily used. In the case where a polynucleotide is introduced using a vector, examples of the vector that may be used include, but are not particularly limited to, a lentivirus vector, a retrovirus vector, a foamy virus vector and an adeno-associated virus vector. A plasmid transposon can be used as a non-viral vector. A sleeping beauty transposon system (described in, for example, Huang X, Guo H, et al., Mol Ther. 2008; 16: 580-9; Singh H, Manuri PR, et al., Cancer Res. 2008; 68: 2961-71; Deniger DC, Yu J, et al., PLoS One. 2015; 10: e0128151; Singh H, Moyes JS, et al., Cancer Gene Ther. 2015; 22: 95-100; Hou X, Du Y, et al., Cancer Biol Ther. 2015; 16: 8-16; Singh H, Huls H, et al., Immunol Rev. 2014; 257: 181-90; and Maiti SN, Huls H, et al., J Immunother. 2013; 36: 112-23) or a piggybac transposon system (described in Nakazawa Y, Huye LE, et al., J Immunother. 2009; 32: 826-36; Galvan DL, Nakazawa Y, et al., J Immunother. 2009; 32: 837-44; Nakazawa Y, Huye LE, et al., Mol Ther. 2011; 19: 2133-43; Huye LE, Nakazawa Y, et al., Mol Ther. 2011; 19: 2239-48; Saha S, Nakazawa Y, et al., J Vis Exp. 2012; (69): e4235; Nakazawa Y, Saha S, et al., J Immunother. 2013; 36: 3-10; and Saito S, Nakazawa Y, et al., Cytotherapy. 2014; 16: 1257-69) can be suitably used.

In a preferred aspect of the present invention, a non-viral vector, particularly, a PiggyBac transposon system can be used. Specific examples will be described in Examples.

The cell of the present invention causes a receptor-specific immune response to target cells expressing a GM-CSF receptor on the surface and is thereby activated through signal transduction into the cell. Activation of a cell expressing CAR varies depending on the type of host cells or the intracellular domain of CAR and can be confirmed based on, for example, cytokine release, improvement in the cell growth rate, or change in the cell surface molecule as an index. The release of a cytotoxic protein (perforin, granzyme, etc.) brings about the destruction of cells expressing the receptor.

The present invention further provides a vector comprising a polynucleotide encoding a chimeric antigen receptor (CAR) protein that specifically binds to a human GM-CSF receptor. The vector of the present invention can be suitably used for producing the genetically modified cell of the present invention described above.

Cells expressing the GMR.CAR of the present invention are desirably cells excellent in safety. It is desirable that the cells excellent in safety should enable, for example, a specific immune response to target cells without influencing normal hematopoietic stem cells, though the cells are not particularly limited thereto. Diseases involving bone marrow-related antigens are known to manifest, for example, bone marrow suppression, as a severe adverse reaction.

Thus, the cells expressing the GMR.CAR of the present invention are desirably cells that do not completely expel bone marrow cells at a dose at which the GMR.CAR-expressing cells kill target cells.

In one aspect of the present invention, cells expressing GMR.CAR having no influence on normal cells can be suitably used as the cells expressing the GMR.CAR of the present invention. When comparing the ability to kill susceptible peripheral cells and/or bone marrow cells and the ability to kill tumor cells in terms of IC50 values, a sufficiently large value of the ratio can be used as an index to confirm safety, but the index is not particularly limited.

The cell expressing the GMR.CAR of the present invention can be used as a therapeutic agent for a disease. Accordingly, the present invention provides a therapeutic agent for a disease involving a GM-CSF receptor expressing cell, comprising the cell of the present invention. The therapeutic agent comprises the cell expressing the CAR protein as an active ingredient and may further comprise an appropriate excipient. The disease that is expected to be treated with the therapeutic agent of the present invention is not limited as long as it exhibits sensitivity to the cell. Examples thereof include a disease involving a cell expressing, particularly highly expressing a GM-CSF receptor, for example, blood cancer (leukemia). Specific examples thereof include juvenile myelomonocytic leukemia (JMML) and acute myelocytic leukemia (AML). Further examples thereof include solid cancer. Specific examples thereof include glioma, neuroblastoma, glioblastoma, brain tumor, colorectal adenocarcinoma, prostate cancer, kidney cancer, melanoma and small cell lung cancer.

In the case of applying the therapeutic agent to solid cancer, the disease includes diseases in which the GM-CSF receptor is expressed on cells forming a microenvironment of target cells, for example, bone marrow-derived immunosuppressive cells (MDSCs). For such a disease, the therapeutic agent can be used in combination with an additional therapeutic agent for the purpose of enhancing, for example, cytotoxic activity against tumor cells, even if the GM-CSF receptor is not expressed on target cells.

In this context, the phrase "expressing, particularly, highly expressing a GM-CSF receptor" may mean that expression intensity is high (e.g., median fluorescence intensity (MFI) is high) or may mean that the expression rate (positive rate) is high. Cells intended to have a high expression rate mean, for example, cells having the CD33/CD116 expression rate (positive rate) of at least 40% or more. Cells having the CD33/CD116 expression rate of 80% or more are preferred from the viewpoint of homogeneous expression in tumor cells. Cells having the CD33/CD116 expression rate of 90% or more are more preferred.

In one aspect, the therapeutic agent of the present invention is an anticancer drug against tumor cells expressing a GM-CSF receptor. The therapeutic agent or the anticancer drug of the present invention can be used alone or in combination with an agent and/or a therapy having different action mechanism.

Thus, the therapeutic agent of the present invention can be used, either alone or in combination with an additional active ingredient, in the form of a pharmaceutical composition. The therapeutic agent or the pharmaceutical composition of the present invention can be topically or systemically administered and is not limited by its dosage form. For example, in the case of treating leukemia, intravenous administration is preferred. The pharmaceutical composition can comprise, in addition to the therapeutic agent of the present invention and the additional active ingredient, a carrier, an excipient, a buffer, a stabilizer, and the like which are usually used in the art, according to the dosage form. The dose of the therapeutic agent of the present invention varies depending on the body weight and age of the patient, the severity of the disease, etc. and is not particularly limited. For example, the dose falls within the range of 0.0001 to 1 mg/kg body weight and can be administered once to several times per day, every 2 days, every 3 days, weekly, every 2 weeks, monthly, every 2 months or every 3 months.

One aspect of the therapeutic agent of the present invention relates to a method for conditioning a patient before cell transplantation, comprising administering an effective amount of cells comprising the CAR protein as the genetically modified cell of the present invention to the patient. In a certain aspect, the cell transplantation is stem cell transplantation. Hematopoietic stem cell transplantation (bone marrow transplantation, umbilical cord blood transplantation, or peripheral blood stem cell transplantation) is suitably used as the stem cell transplantation.

One aspect of the therapeutic agent of the present invention includes decreasing the number of GM-CSF receptor-expressing cells in a patient as the conditioning of the patient before cell transplantation. The GM-CSF receptor-expressing cells in a patient are GM-CSF receptor-expressing normal cells or GM-CSF receptor-expressing cancer cells. The conditioning of the patient may be decreasing in the numbers of both the GM-CSF receptor-expressing normal cells and cancer cells before cell transplantation.

## Examples

Hereinafter, the present invention will be described further specifically with reference to Examples. However, the present invention is not limited by Examples given below.

### [Example 1 Production of GMR.CAR expression plasmid (CAR 001)]

A PCR product of human GM-CSF (coding region of NCBI Accession No.: NM_000758 (SEQ ID NO: 22, nucleotides 33 to 464: prior to stop codon)) with an EcoRI site at its 5' side and an IgG1 hinge-encoding nucleotide sequence and a BclI site at its 3' side was subcloned to a pTA2 cloning vector (Toyobo Co., Ltd.). The pTA2 cloning vector to which the human GM-CSF and IgG1 hinge sequences were subcloned was double-digested with EcoRI and BclI to cleave the human GM-CSF and IgG1 hinge sequences. Meanwhile, a pSL1190 vector (Amersham plc) was ligated with an anti-CD19 scFv (FMC63; Zola H. et al., Immunol Cell Biol. 1991; 69 (Pt6): 411-412)-CD28-CD3zeta fragment obtained by double-digesting SFG-CD19.CAR (Savoldo et al., J Clin Invest 2011; 121 (5): 1822-1826) with NcoI and SphI, to produce a pSL1190-CD19.CAR vector.

The pSL1190-CD19.CAR vector was double-digested with EcoRI and BamHI and then ligated with the cleaved human GM-CSF and IgG1 hinge sequences mentioned above using compatible sites to produce a pSL1190-GMR.CAR vector. Further, a pIRII-CD19.CAR vector (Huye et al., Mol Ther 2011; 19 (12): 2239-2248) was double-digested with EcoRI and NotI to remove a CD19.CAR fragment, instead of which a fragment obtained by double-digesting the pSL1190-GMR.CAR vector with EcoRI and NotI was ligated to obtain a GMR.CAR expression plasmid (CAR 001). Figure 1 shows the vector map of the produced CAR 001.

### [Example 2 Production of mutant GMR.CAR expression plasmids (E21R, E21H, E21S, and E21A)]

Mutant GMR.CAR expression plasmids were produced by inverse-PCR using CAR 001 as a template.

The 5' PCR primers used in inverse-PCR were designed and synthesized such that glutamic acid (E) at position 21 of a GM-CSF polypeptide (SEQ ID NO: 1) was substituted with arginine (R), histidine (H), serine (S) or alanine (A) (SEQ ID NOs: 23 to 26, respectively, outsourced to Eurofins Genomics K.K.). The 3' primer was designed (SEQ ID NO: 27) and synthesized in common among all the mutants (outsourced to Eurofins Genomics K.K.).

Forward primer for E21R production: AGAGCCCGGCGTCTCCTGAACCTGAGTA (SEQ ID NO: 23)
Forward primer for E21H production: CACGCCCGGCGTCTCCTGAACCTGAGTA (SEQ ID NO: 24)
Forward primer for E21S production: AGCGCCCGGCGTCTCCTGAACCTGAGTA (SEQ ID NO: 25)
Forward primer for E21A production: GCCGCCCGGCGTCTCCTGAACCTGAGTAGA (SEQ ID NO: 26)
Reverse primer: CTGGATGGCATTCACATCGAGGGTC (SEQ ID NO: 27)

CAR 001 was adjusted to 50 ng/μL and used as a template. Each PCR primer was adjusted to a concentration of 0.2 or 0.3 μM in a reaction solution. Inverse-PCR employed KOD-Plus-Mutagenesis Kit (Toyobo Co., Ltd.), and the reaction composition abided the protocol attached to the kit. The reaction conditions involved (i) 94°C for 2 minutes, (ii) 98°C for 10 seconds, and (iii) 68°C for 7 minutes with 10 cycles of (ii) and (iii).

After the PCR reaction, an aliquot of the sample was separated by 1 to 1.2% agarose gel electrophoresis to confirm the production of a linear plasmid having the size of interest. The remaining sample after the PCR reaction was treated with DpnI according to the protocol attached to the kit. Through this reaction, methylated template plasmid CAR 001 was cleaved and eliminated. Then, the linear plasmid was self-ligated with T4 Polynucleotide Kinase attached to the kit to form a circular plasmid. Then, *E. coli* DH5α (Toyobo Co., Ltd.) was transformed with the circular plasmid and cultured on an LB agar medium containing 50 μg/mL ampicillin for approximately 16 hours.

Colonies that appeared were further cultured in an LB liquid medium containing 50 μg/mL ampicillin for approximately 16 hours. Each plasmid was purified from the culture solution of the cultured *E. coli* using QIAprep Spin Miniprep Kit (Qiagen N.V.) and sequenced. Plasmids confirmed to have the substitution of the nucleotide sequence of interest were designated as CAR 002 (E21R GMR.CAR), CAR 004 (E21H GMR.CAR), CAR 006 (E21S GMR.CAR) and CAR 008 (E21A GMR.CAR).

[Example 3 Production of mutant GMR.CAR expression plasmids (E21K, E21D, and E21F)]

Plasmids were produced in the same way as in Example 2 such that glutamic acid (E) at position 21 of the GM-CSF polypeptide (SEQ ID NO: 1) was substituted with lysine (K), aspartic acid (D) or phenylalanine (F). Specifically, an XhoI-secretory signal sequence-GM-CSF mutant-hinge sequence-DraIII fragment having a sequence to be cleaved with a restriction enzyme XhoI at the 5' side and a sequence to be cleaved with a restriction enzyme DraIII at the 3' side was artificially synthesized (outsourced to Eurofins Genomics K.K.). This fragment was ligated with a fragment obtained by treating CAR 001 with XhoI and DraIII to produce CAR 003 (E21K GMR.CAR), CAR 005 (E21D GMR.CAR) or CAR 007 (E21F GMR.CAR).

[Example 4 Production of mutant spacer-modified GMR.CAR expression plasmids]

Mutant spacer-modified GMR.CAR expression plasmids were produced by producing spacer-modified GMR.CAR expression plasmids and then incorporating a mutation into these plasmids as templates.

GMR.CAR expression plasmids having a modified extracellular spacer domain moiety were produced by inverse-PCR using CAR 001 as a template. Specifically, a CH2CH3 partial deletion mutant (Figure 2) and a CH2CH3 partial deletion-(G4S)3 insertion mutant (Figure 3) were produced, the vector maps of which are shown in Figures 2 and 3, respectively.

The PCR primers used in inverse-PCR were designed and synthesized as primers for the CH2CH3 partial deletion mutant (SEQ ID NOs: 28 and 29) and primers for the CH2CH3 partial deletion-(G4S)3 insertion mutant (SEQ ID NOs: 30 and 31) (outsourced to Eurofins Genomics K.K.) as given below.

<Primer for CH2CH3 partial deletion mutant>

Forward primer: 5'- TTTTGGGTGCTGGTGGTGGTTGGTGGAGTC -3' (SEQ ID NO: 28)
Reverse primer: 5'- TGGGCATGTGTGAGTTTTGTCAGGAGAT -3' (SEQ ID NO: 29)

<Primer for CH2CH3 partial deletion-(G4S)3 insertion mutant>

Forward primer: 5'-GGTGGTGGTGGATCCGGCGGCGGCGGCTCCGGTGGTGGTGGTTCTAAAGATCCCA AATTTTGGGTGCTGG -3' (SEQ ID NO: 30)
Reverse primer: 5'- TGGGCATGTGTGAGTTTTGTCAGGAGATTTGGGC -3' (SEQ ID NO: 31)

CAR 001 was adjusted to 50 ng/μL and used as a template. Each PCR primer was adjusted to a concentration of 0.2 or 0.3 μM in a reaction solution. Inverse-PCR employed KOD-Plus-Mutagenesis Kit (Toyobo Co., Ltd.), and the reaction composition abided the protocol attached to the kit. The reaction conditions involved (i) 94°C for 2 minutes, (ii) 98°C for 10 seconds, and (iii) 68°C for 7 minutes with 10 cycles of (ii) and (iii).

After the PCR reaction, an aliquot of the sample was separated by 1 to 1.2% agarose gel electrophoresis to confirm the production of a linear plasmid having the size of interest. Subsequently, the remaining sample after the PCR reaction was treated with DpnI according to the protocol attached to the kit. Through this reaction, methylated template plasmid CAR 001 was cleaved and eliminated. Then, the linear plasmid was self-ligated with T4 Polynucleotide Kinase attached to the kit to form a circular plasmid. Then, *E. coli* DH5α (Toyobo Co., Ltd.) was transformed with each circular plasmid thus obtained and cultured on an LB agar medium containing 50 μg/mL ampicillin for approximately 16 hours.

Colonies that appeared were further cultured in an LB liquid medium containing 50 μg/mL ampicillin for approximately 16 hours. Each plasmid was purified from the culture solution of the cultured *E. coli* using QIAprep Spin Miniprep Kit (Qiagen N.V.) and sequenced. Mutant spacer-modified GMR.CAR expression plasmids confirmed to have the modification of the nucleotide sequence of interest were obtained: CAR 009 (GMR.CAR dCH2CH3) and CAR 010 (GMR.CAR dCH2CH3+(G4S)3).

Mutant spacer-modified GMR.CAR expression plasmids were produced by inverse-PCR using the produced CAR 009 and CAR 010 as templates. The vector maps of the produced plasmids are shown in Figures 4 to 7. A CH2CH3 partial deletion mutant with E at position 21 of the GM-CSF polypeptide (SEQ ID NO: 1) substituted with R (Figure 4), a CH2CH3 partial deletion mutant with E at position 21 substituted with K (Figure 5), a CH2CH3 partial deletion-(G4S)3 insertion mutant with E at position 21 substituted with R (Figure 6) and a CH2CH3 partial deletion-(G4S)3 insertion mutant with E at position 21 substituted with K (Figure 7) were produced.

In this Example, the PCR primers used in inverse-PCR were designed and synthesized as primers for the substitution of E with R (SEQ ID NOs: 32 and 33) and primers for the substitution of E with K (SEQ ID NOs: 34 and 33) (outsourced to Eurofins Genomics K.K.) as given below.

<Primer for substitution of E with R>

Forward primer: 5'- AGAGCCCGGCGTCTCCTGAACCTG -3' (SEQ ID NO: 32)
Reverse primer: 5'- CTGGATGGCATTCACATGCTCCCAGGG -3' (SEQ ID NO: 33)

<Primer for substitution of E with K>

Forward primer: 5'- AAGGCCCGGCGTCTCCTGAACCTG -3' (SEQ ID NO: 34)
Reverse primer: 5'- CTGGATGGCATTCACATGCTCCCAGGG -3' (SEQ ID NO: 33)

Each of CAR 009 and CAR 010 was adjusted to 50 ng/μL and used as a template. Each PCR primer was adjusted to a concentration of 0.2 or 0.3 μM in a reaction solution. Inverse-PCR employed KOD-Plus-Mutagenesis Kit (Toyobo Co., Ltd.), and the reaction composition abided the protocol attached to the kit. The reaction conditions involved (i) 94°C for 2 minutes, (ii) 98°C for 10 seconds, and (iii) 68°C for 7 minutes with 10 cycles of (ii) and (iii).

After the PCR reaction, an aliquot of the sample was separated by 1 to 1.2% agarose gel electrophoresis to confirm the production of a linear plasmid having the size of interest. Subsequently, the remaining sample after the PCR reaction was treated with DpnI according to the protocol attached to the kit. Through this reaction, a methylated template plasmid was cleaved and eliminated. Then, the linear plasmid was self-ligated with T4 Polynucleotide Kinase attached to the kit to form a circular plasmid. Then, *E. coli* DH5α (Toyobo Co., Ltd.) was transformed with each circular plasmid thus obtained and cultured on an LB agar medium containing 50 μg/mL ampicillin for approximately 16 hours.

Colonies that appeared were further cultured in an LB liquid medium containing 50 μg/mL ampicillin for approximately 16 hours. Each plasmid was purified from the culture solution of the cultured *E. coli* using QIAprep Spin Miniprep Kit (Qiagen N.V.) and sequenced. Mutant spacer-modified GMR.CAR expression plasmids confirmed to have the modification of the nucleotide sequence of interest were obtained: CAR 011 (E21R GMR.CAR dCH2CH3), CAR 012 (E21KGMR.CAR E dCH2CH3), CAR 013 (E21RGMR.CAR dCH2CH3+(G4S)3) and CAR 014 (E21KGMR.CAR dCH2CH3+(G4S)3).

[Example 5 Culture and amplification of GMR.CAR-T cells]

<Separation of PBMCs and production of nonspecificly stimulated OKT 3 blasts (Day 0)>

Peripheral blood was collected from a healthy adult donor and diluted 2-fold with D-PBS (Wako Pure Chemical Industries, Ltd.). Then, the diluted peripheral blood was overlayered on Ficoll-Paque PLUS and centrifuged at 400 × g for 30 minutes to separate PBMCs. The separated PBMCs were washed twice with D-PBS and isolated by centrifugation. The isolated PBMCs were suspended at 2 to 4 × $10^6$ cells/well/2 mL in TexMACS medium containing 5 ng/mL IL-15. A 24-well non-treatment culture plate was coated with D-PBS containing an anti-human CD3 antibody and an anti-human CD28 antibody at 37°C for 2 hours to prepare an antibody-coated plate. The cell suspension was seeded at 2 mL/well for specific stimulation of T cells to start the production of OKT3 blasts.

On day 3, an antibody-immobilized plate for re-stimulation of OKT3 blasts was produced. Specifically, an anti-human CD3 antibody and an anti-human CD28 antibody adjusted to 1 μg/mL with D-PBS were added at 500 μL/well to a 24-well non-treatment flat-bottomed plate and incubated overnight at 4°C to immobilize the antibodies onto the plate.

Meanwhile, OKT3 blasts stimulated from day 0 were transferred to an antibody-unimmobilized 24-well flat-bottomed multiwell plate for cell culture and thereby were not stimulated temporarily.

On day 4, a supernatant was removed from the plate subjected to the antibody immobilization on the previous day. OKT3 blasts were added thereto to start re-stimulation of the OKT3 blasts with the anti-human CD3 antibody and the anti-human CD28 antibody. The re-stimulation was carried out until day 7. During this period, medium replacement and addition of IL-15 were appropriately performed.

<Viral peptide pulse of PBMCs (Day 0)>

The isolated PBMCs were stimulated with viral peptides using PepTivator peptide pool® (Miltenyi Biotec GmbH). Specifically, PBMCs were suspended in the peptide pool of 50 μL of D-PBS supplemented with 0.05 μg/μL each of AdV5 Hexon, CMV pp65, EBV BZLF1 and EBV EBNA-1, and stimulated with the viral peptides at 37°C for 30 minutes. 30 minutes later, an appropriate amount of D-PBS was added to PBMCs for suspension, followed by UV irradiation for 4 minutes. The UV-irradiated PBMCs were recovered, and the cell number was counted. Then, the cells were suspended at 0.5 to 6 × $10^6$ cells/well/2 mL in TexMACS medium containing 10 ng/mL IL-7 and 5 ng/mL IL-15, and transferred as feeder cells to a 24-well treatment culture plate.

<Electrical introduction of GMR.CAR expression vectors (Day 0)>

For the introduction of the mutant GMR.CAR expression plasmid CAR 002 (E21R GMR.CAR), CAR 003 (E21K GMR.CAR), CAR 004 (E21H GMR.CAR), CAR 005 (E21D GMR.CAR), CAR 006(E21S GMR.CAR), CAR 007 (E21F GMR.CAR) or CAR 008 (E21A GMR.CAR) to PBMCs, 5 μg of each CAR, 5 μg of pCMV-piggyBac plasmid, and 100 μL of P3 Primary Cell solution attached to P3 Primary Cell 4D-Nucleofector™ X Kit (Lonza Japan Ltd.) were mixed and added to 10 × $10^6$ PBMCs.

Subsequently, the total amount of the cells was transferred to Nucleocuvette where electrical gene introduction was carried out by 4D-Nucleofection (Program No: FI-115). The cells after the electrical gene introduction were left at room temperature for 10 minutes. The total amount of the cells was added to the 24-well treatment culture plate containing the feeder cells to start culture. 1 to 15 ×$10^6$ cells were similarly cultured as Mock-T cells without gene introduction operation. Medium exchange operation was appropriately performed by discarding half the amount of the culture medium and adding half the amount of TexMACS medium containing 20 ng/mL IL-7 and 10 ng/mL IL-15 (double concentration). The Mock-T cells were continuously cultured on a 24-well treatment culture plate, and medium exchange and amplification were performed depending on the degree of cell growth.

<Amplification of CAR-T cells (Day 7)>

The total amount of the cell suspension was transferred to G-Rex 10 filled with 30 mL of TexMACS medium containing 10 ng/mL IL-7 and 5 ng/mL IL-15, OKT 3 blasts produced as described above, and 2 × $10^6$ feeder cells treated with peptide pulse and UV irradiation. The GMR.CAR-T cells were amplified until day 14. The expression rate of the GMR.CAR-T cells amplified until day 14 was measured by the following method.

<Evaluation of GMR.CAR expression rate (Day 14)>

The cell number was counted, and 1 to 2 $\times$ $10^5$ cells were evaluated for the expression rate of GMR.CAR-T by flow cytometry analysis. 1 to 2 $\times$ $10^5$ cells were collected, suspended by the addition of 2.5 μL of PE Rat Anti-Human GM-CSF antibody (BD Pharmingen) and 5 μL of APC Anti-Human CD3 antibody (Milteny Biotech GmbH), and subjected to antibody labeling reaction at 4°C for 20 minutes in the dark. 20 minutes later, the cells were washed with an appropriate amount of D-PBS and precipitated by centrifugation. After removal of the supernatant, the cells were resuspended in an appropriate amount of D-PBS. This sample was analyzed using FACSCalibur (Nippon Becton Dickinson Co., Ltd.) to determine the expression rate of GMR.CAR with IgG1/CD3 positivity or GM-CSF/CD3 positivity as an index.

GMR.CAR-T cells obtained by electrical introduction operation of the GMR.CAR expression plasmid (CAR 001) into PBMCs and T cell culture and amplification operation were designated as CAR-T 001. Likewise, GMR.CAR-T cells obtained by operation using the CAR 002 (E21R GMR.CAR), CAR 003 (E21K GMR.CAR), CAR 004 (E21H GMR.CAR), CAR 005 (E21D GMR.CAR), CAR 006(E21S GMR.CAR), CAR 007(E21F GMR.CAR) and CAR 008 (E21A GMR.CAR) plasmids were designated as CAR-T 002 (E21R), CAR-T 003 (E21K), CAR-T 004 (E21H), CAR-T 005 (E21D), CAR-T 006 (E21S), CAR-T 007 (E21F), and CAR-T 008 (E21A), respectively.

The expression analysis results about CAR-T 001 to 008 on day 14 are shown in Figure 8. The GMR.CAR expression rate in each cell group was CAR-T 001: 20.89%, CAR-T 002 (E21R): 16.68%, CAR-T 003 (E21K): 15.94%, CAR-T 004 (E21H): 17.89%, CAR-T 005 (E21D): 19.83%, CAR-T 006 (E21S): 21.00%, CAR-T 007 (E21F): 20.47%, and CAR-T 008 (E21A): 20.01%, all of which were high as compared with the expression rate in Mock-T cells (5.66%). Thus, T cells expressing a mutant GMR.CAR were successfully produced.

[Example 6 Anti-tumor cell activity of GMR.CAR-T]

In order to evaluate CAR-T 001 to 008 obtained in Example 5 for their cytotoxic activity against tumor cells, a coculture test with the tumor cells was conducted. Specifically, THP-1 cells (American Type Culture Collection (ATCC)) were used, and the tumor cells [target (T)] were adjusted to 5 $\times$ $10^5$ cells/mL with an RPMI 1640 medium containing 10% FBS (Thermo Fisher Scientific Inc.), and seeded at 500 μL/well in a 48-well treatment culture plate. Each of CAR-T 001 to CAR-T 008 [effector (E)] obtained in Example 5 was diluted with an RPMI 1640 medium containing 10% FBS so as to attain an E:T ratio of 1:5 to 1:100.

Specifically, in the case of the E:T ratio = 1:5, the GMR.CAR-T cells were adjusted to 1 $\times$ $10^5$ cells/mL and added at 500 μL/well to the 48-well treatment culture plate containing the seeded tumor cells. Likewise, in the case of the E:T ratio = 1:50 or 1:100, the GMR.CAR-T cells were adjusted to 0.1 $\times$ $10^5$ cells/mL or 0.05 $\times$ $10^5$ cells/mL and added at 500 μL/well to the 48-well treatment culture plate containing the seeded tumor cells. The coculture test was conducted for 5 days. Mock-T cells were also subjected to the same coculture test. A group of tumor cells alone having the same cell number was also established as a control group (CAR-T non-addition group).

On day 5 of the coculture test, the cells in each well were recovered and centrifuged. The cells were suspended by the addition of 5 μL of APC Anti-Human CD3 antibody (Miltenyi Biotec GmbH) and 5 μL of PE Anti-Human CD33 antibody (Miltenyi Biotec GmbH), and subjected to antibody labeling reaction at 4°C for 20 minutes in the dark. 20 minutes later, the cells were washed with an appropriate amount of D-PBS and precipitated by centrifugation. After complete removal of the supernatant, the cells were resuspended in 450 μL of D-PBS, and 50 μL of CountBright absolute counting beads (Invitrogen Corp.) was further added thereto. This sample was analyzed using FACSCanto II (Nippon Becton Dickinson Co., Ltd.) and FLOWJO (Nippon Becton Dickinson Co., Ltd.). CD33-positive cell number was calculated on the basis of the counting beads. The anti-tumor cell activity % of CAR-T 001 to CAR-T 008 and Mock-T cells was calculated according to the following formula.

<Calculation of anti-tumor cell activity %>

Formula:

Anti-tumor cell activity % = 100 - (CD33-positive cell number of CAR-T addition group) / (CD33-positive cell number of CAR-T non-addition group) $\times$ 100

Figure 9 shows the results of the anti-tumor cell activity % of CAR-T 001 to CAR-T 008 against THP-1 cells. All of CAR-T 002 to CAR-T 008 were found to have equivalent or higher anti-tumor cell activity against the THP-1 cells as compared with CAR-T 001.

[Example 7 Culture and amplification of mutant spacer-modified GMR.CAR-T cell]

<Separation of PBMCs and production of nonspecificly stimulated OKT 3 blasts (Day 0)>

Peripheral blood was collected from a healthy adult donor and diluted 2-fold with D-PBS (Wako Pure Chemical Industries, Ltd.). Then, the diluted peripheral blood was overlayered on Ficoll-Paque PLUS and centrifuged at 400 × g for 30 minutes to separate PBMCs. The separated PBMCs were washed twice with D-PBS and isolated by centrifugation. The isolated PBMCs were suspended at 2 to 4 × $10^6$ cells/well/2 mL in TexMACS medium containing 5 ng/mL IL-15. A 24-well non-treatment culture plate was coated with D-PBS containing an anti-human CD3 antibody and an anti-human CD28 antibody at 37°C for 2 hours to prepare an antibody-coated plate. The cell suspension was seeded at 2 mL/well for specific stimulation of T cells to start the production of OKT3 blasts.

On day 3, an antibody-immobilized plate for re-stimulation of OKT3 blasts was produced. Specifically, an anti-human CD3 antibody and an anti-human CD28 antibody each adjusted to 1 μg/mL with D-PBS were added at 500 μL/well to a 24-well non-treatment flat-bottomed plate and incubated overnight at 4°C to immobilize the antibodies onto the plate.

Meanwhile, OKT3 blasts stimulated from day 0 were transferred to an antibody-unimmobilized 24-well flat-bottomed multiwell plate for cell culture and thereby were not stimulated temporarily.

On day 4, a supernatant was removed from the plate subjected to the antibody immobilization on the previous day. OKT3 blasts were added thereto to start the re-stimulation of the OKT3 blasts with the anti-human CD3 antibody and the anti-human CD28 antibody. The re-stimulation was carried out until day 7. During this period, medium replacement and addition of IL-15 were appropriately performed.

<Viral peptide pulse of PBMCs (Day 0)>

The isolated PBMCs were stimulated with viral peptides using PepTivator peptide pool® (Miltenyi Biotec GmbH). Specifically, PBMCs were suspended in the peptide pool of 50 μL of D-PBS supplemented with 0.05 μg/μL each of AdV5 Hexon, CMV pp65, EBV BZLF1 and EBV EBNA-1, and stimulated with the viral peptides at 37°C for 30 minutes. 30 minutes later, an appropriate amount of D-PBS was added to PBMCs for suspension, followed by UV irradiation for 4 minutes. The UV-irradiated PBMCs were recovered, and the cell number was counted. Then, the cells were suspended at 0.5 to 6 × $10^6$ cells/well/2 mL in TexMACS medium containing 10 ng/mL IL-7 and 5 ng/mL IL-15, and transferred as feeder cells to a 24-well treatment culture plate.

<Gene introduction operation (Day 0)>

For the introduction of the GMR.CAR expression plasmids to PBMCs, 5 μg of any of CAR 001 and CAR 009 to CAR 014, 5 μg of pCMV-piggyBac plasmid, and 100 μL of P3 Primary Cell solution attached to P3 Primary Cell 4D-Nucleofector™ X Kit (Lonza Japan Ltd.) were mixed and added to 10 to 15 × $10^6$ PBMCs.

Subsequently, the total amount of the cells was transferred to Nucleocuvette where electrical gene introduction was carried out by 4D-Nucleofection (Program No: FI-115). The cells after the electrical gene introduction were left at room temperature for 10 minutes. The total amount of the cells was added to the 24-well treatment culture plate containing the feeder cells to start culture. 1 to 15 × $10^6$ cells were similarly cultured as Mock-T cells without gene introduction operation. Medium exchange operation was appropriately performed by discarding half the amount of the culture medium and adding half the amount of TexMACS medium containing 20 ng/mL IL-7 and 10 ng/mL IL-15 (double concentration). The Mock-T cells were continuously cultured on a 24-well treatment culture plate, and medium exchange and amplification were performed depending on the degree of cell growth.

<Amplification of CAR-T cells (Day 7)>

The total amount of the cell suspension was transferred to G-Rex 10 filled with 30 mL of TexMACS medium containing 10 ng/mL IL-7 and 5 ng/mL IL-15, OKT 3 blasts produced as described above, and 2 to 10 × $10^6$ feeder cells treated with peptide pulse and UV irradiation. The GMR.CAR-T cells were amplified until day 14. The expression rate of the GMR.CAR-T cells amplified until day 14 was measured by the following method.

<Evaluation of GMR.CAR expression rate (Day 14)>

The cell number was counted, and 1 to 2 × $10^5$ cells were evaluated for the expression rate of GMR.CAR-T by flow cytometry analysis. 1 to 2 × $10^5$ cells were collected and centrifuged. The cells were suspended by the addition of 5 μL of PE Rat Anti-Human GM-CSF antibody (Milteny Biotech GmbH) and 5 μL of APC mouse Anti-Human CD3 antibody (Milteny Biotech GmbH), and subjected to antibody labeling reaction at 4°C for 20 minutes in the dark. 20 minutes later, the cells were washed with an appropriate amount of D-PBS and precipitated by centrifugation. After removal of the supernatant, the cells were resuspended in an appropriate amount of D-PBS. This sample was analyzed using FACSCanto II (Nippon Becton Dickinson Co., Ltd.) and FLOWJO (Nippon Becton Dickinson Co., Ltd.) to determine the expression rate of GMR.CAR with GM-CSF/CD3 positivity as an index.

In this Example, GMR.CAR-T cells obtained by electrical introduction operation of the expression plasmids (CAR 001 and CAR 009 to CAR 014) into PBMCs and T cell culture and amplification operation were designated as CAR-T 001-A, CAR-T 009, CAR-T 010, CAR-T 011, CAR-T 012, CAR-T 013 and CAR-T 014.

The expression analysis results about CAR-T 001-A and CAR-T 009 to CAR-T 014 on day 14 are shown in Figure 10. The GMR.CAR expression rate in each cell group was CAR-T 001-A: 28.0%, CAR-T 009: 31.7%, CAR-T 010: 31.0%, CAR-T 011: 30.1%, CAR-T 012: 31.7%, CAR-T 013: 35.4%, and CAR-T 014: 30.7%, all of which were high as compared with the expression rate of 0.54% in Mock-T cells. Thus, T cells expressing GMR.CAR differing in spacer site were successfully produced. The produced GMR.CAR-expressing T cells were diluted with Mock-T cells to adjust the expression rate to 28.0%, and evaluated for their anti-tumor cell activity and safety in Examples given below.

[Example 8 Anti-tumor cell activity of mutant spacer-modified GMR.CAR-T]

In order to evaluate CAR-T 001-A and CAR-T 009 to CAR-T 014 obtained in Example 7 for their cytotoxic activity against tumor cells, a coculture test with the tumor cells was conducted. Specifically, THP-1 cells (American Type Culture Collection (ATCC)) were used, and the THP-1 cells [target (T)] were adjusted to 4 × $10^5$ cells/mL with an RPMI 1640 medium containing 10% FBS (Thermo Fisher Scientific Inc.), and seeded at 500 μL/well in a 48-well treatment culture plate (cell number in a well was 2.0 × $10^5$ cells).

Each of CAR-T 001-A and CAR-T 009 to CAR-T 014 [effector (E)] was diluted with an RPMI 1640 medium containing 10% FBS so as to attain an E:T ratio of 1:25 and 1:125. Specifically, in the case of the E:T ratio = 1:25, CAR-T was adjusted to 0.16 × $10^5$ cells/mL and added at 500 μL/well to the 48-well treatment culture plate containing the seeded tumor cells (CAR-T cell number in a well was 0.08 × $10^5$ cells). Likewise, in the case of the E:T ratio = 1:125, the cells were adjusted to 0.032 × $10^5$ cells/mL and added at 500 μL/well to the 48-well treatment culture plate containing the seeded tumor cells (CAR-T cell number in a well was 0.016 × $10^5$ cells).

Anti-tumor cell activity % was calculated in the same way as in Example 6. Figure 11 shows the results of the anti-tumor cell activity % of CAR-T 001-A, CAR-T 009 to CAR-T 014 and Mock-T cells against THP-1 cells. All of CAR-T 009 to CAR-T 014 were found to have equivalent or higher anti-tumor cell activity against the THP-1 cells as compared with CAR-T 001-A.

[Example 9 Evaluation of sustained anti-tumor cell activity of mutant spacer-modified GMR.CAR-T]

CAR-T cells expected to be effective in adoptive immunotherapy are required to have sustained cytotoxic activity. Thus, in order to evaluate the sustained anti-tumor cell activity of GMR.CAR-T, the following experiment was conducted.

Electrical introduction operation of the expression plasmids (CAR 010 to CAR 014) into PBMCs and T cell culture and amplification operation were performed in the same way as in Example 7 to newly produce GMR.CAR-T cells (CAR-T 010-A to CAR-T 014-A).

The expression analysis results about CAR-T 010-A to 014-A used in this Example on day 14 are shown in Figure 12. The GMR.CAR expression rate in each cell group was CAR-T 010-A: 26.2%, CAR-T 011-A: 27.6%, CAR-T 012-A: 18.5%, CAR-T 013-A: 29.8%, and CAR-T 014-A: 26.9%, all of which were higher as compared with the expression rate in Mock-T cells (0.50%). Thus, T cells expressing GMR.CAR differing in spacer site were successfully produced.

Subsequently, the produced GMR.CAR-expressing T cells were diluted with Mock-T cells to adjust the expression rate to 18.5%, and evaluated for their sustained anti-tumor cell activity.

In order to evaluate CAR-T 010-A to CAR-T 014-A for their sustained cytotoxic activity against tumor cells, a long-term coculture test with the tumor cells was conducted. Specifically, THP-1, MV4-11, Kasumi-1 cells (American Type Culture Collection (ATCC)) and shinAML-1 cells (cell line established from AML patients at Shinshu University Hospital) were used, and the tumor cells of each group [target (T)] were adjusted to 5 × $10^5$ cells/mL with an RPMI 1640 medium containing 10% FBS (Thermo Fisher Scientific Inc.), and seeded at 500 μL/well in a 48-well treatment culture plate (tumor cell number in each well was 2.5 × $10^5$ cells).

Each of CAR-T 010-A to 014-A [effector (E)] was diluted with an RPMI 1640 medium containing 10% FBS so as to attain an E:T ratio of 1:1. Specifically, CAR-T was adjusted to 5 × $10^5$ cells/mL and added at 500 μL/well to the 48-well treatment culture plate containing the seeded tumor cells of each group (CAR-T cell number in a well was 2.5

$\times 10^5$ cells). Two wells were provided for each group. One of the wells was used "for analysis" using FACSCanto II (Nippon Becton Dickinson Co., Ltd.), and the other well was used "for continuation" for addition to new tumor cells in the next term.

The coculture test of term 1 was conducted for 4 days. Mock-T cells without gene introduction were also subjected to the same coculture test. A group of tumor cells alone having the same cell number was also established as a control group (CAR-T non-addition group).

On day 4 of the coculture test, the cells in the well "for analysis" were recovered and centrifuged. The cells were suspended by the addition of 5 μL of APC Anti-Human CD3 antibody (Miltenyi Biotec GmbH) and 5 μL of PE Anti-Human CD33 antibody (Miltenyi Biotec GmbH), and subjected to antibody labeling reaction at 4°C for 20 minutes in the dark. 20 minutes later, the cells were washed with an appropriate amount of D-PBS and precipitated by centrifugation. After complete removal of the supernatant, the cells were resuspended in 450 μL of D-PBS, and 50 μL of CountBright absolute counting beads (Invitrogen Corp.) was further added thereto. This sample was analyzed using FACSCanto II and FLOWJO (Nippon Becton Dickinson Co., Ltd.). A CD33-positive cell number was calculated on the basis of the counting beads. These procedures were included in term 1.

A group in which the tumor cells were killed (tumor cell number was decreased) compared to the start of coculture proceeded to the following term 2. Specifically, two wells containing tumor cells seeded in advance at $2.5 \times 10^5$ cells/500 μL/well were prepared. To the wells, the culture solution of the cells cocultured "for continuation" in term 1 was equally added at 500 μL/well and further cocultured for 4 days. This 4-day or 3-day culture operation was repeated. A group in which the tumor cell number was increased as compared with that at the start of coculture did not proceed to the following term.

Figures 13 to 17 show the results of anti-tumor cell activity evaluation on days 4, 8, 12, 15, 19, and 22 targeting tumor cells of each group. CAR-T 010-A to CAR-T 014-A were confirmed to have a sustained killing effect on all the tumor cell lines.

[Example 10 Effect of GMR.CAR-T on peripheral cells]

The GMR.CAR-T cells used in this Example (CAR-T 001-B and CAR-T 009-B to CAR-T 014-B) were newly produced by the following method.

<Gene introduction operation (Day 0)>

OKT3 blasts were produced in the same way as in Example 7. Then, for the introduction of the GMR.CAR expression plasmids to PBMCs, 5 μg of any of CAR 001 and CAR 009 to CAR 014, 5 μg of pCMV-piggyBac plasmid, and 100 μL of P3 Primary Cell solution attached to P3 Primary Cell 4D-Nucleofector™ X Kit (Lonza Japan Ltd.) were mixed and added to 15 to $20 \times 10^6$ PBMCs.

The total amount of the cells was transferred to Nucleocuvette where electrical gene introduction was carried out by 4D-Nucleofection (Program No: FI-115). The cells after the electrical gene introduction were left at room temperature for 10 minutes and added to a 24-well non-treatment culture plate (which was antibody-coated with D-PBS containing an anti-CD28 antibody at 37°C for 2 hours and then supplemented with feeder cells) to start culture. 1 to $20 \times 10^6$ cells were similarly cultured as Mock-T cells without gene introduction operation. On day 3, the cells were transferred from the 24-well non-treatment plate to a 24-well treatment culture plate and continuously cultured.

Medium exchange operation was appropriately performed by discarding half the amount of the culture medium and adding half the amount of TexMACS medium containing 20 ng/mL IL-7 and 10 ng/mL IL-15 (double concentration). The Mock-T cells were continuously cultured on the 24-well treatment culture plate, and medium exchange and amplification were performed depending on the degree of cell growth.

Subsequently, the evaluation of amplification of the CAR-T cells and the evaluation of GMR.CAR expression rate was performed in the same way as in Example 7.

The expression analysis results about CAR-T 001-B and CAR-T 009-B to CAR-T 014-B used in this Example on day 14 are shown in Figure 18. The GMR.CAR expression rate in each cell group was CAR-T 001-B: 45.9%, CAR-T 009-B: 37.2%, CAR-T 010-B: 42.4%, CAR-T 011-B: 46.4%, CAR-T 012-B: 41.1%, CAR-T 013-B: 48.3%, and CAR-T 014-B: 51.2%, all of which were higher as compared with the expression rate of 0.56% in Mock-T cells. Thus, GMR.CAR-expressing T cells were successfully produced.

Next, the produced GMR.CAR-expressing T cells were diluted with Mock-T cells to adjust the expression rate to 37.2%, and their influence on peripheral blood cells was confirmed. In order to confirm the influence of CAR-T 001-B and CAR-T 009-B to CAR-T 014-B on peripheral blood cells, a coculture test with the peripheral blood cells was conducted.

<Separation of polymorphonuclear leukocyte (PMN) and PBMC>

Peripheral blood was collected from a healthy adult donor and diluted 2-fold with D-PBS. Then, the diluted peripheral blood was overlayered on polymorph prep (AXS) and centrifuged at 450 × g for 35 minutes to separate PMNs and PBMCs. The separated PMNs and PBMCs were washed twice with D-PBS and isolated by centrifugation.

A coculture test with the isolated PMNs or PBMCs or a tumor cell line MV4-11 was conducted using CAR-T 001-B and CAR-T 009-B to CAR-T 014-B. Specifically, PMNs, PBMCs, or MV4-11 cells [target (T)] were adjusted to 2 × $10^5$ cells/mL with an RPMI 1640 medium containing 10% FBS (Thermo Fisher Scientific Inc.), and seeded at 500 μL/well in a 48-well treatment culture plate (cell number in each well was 1 × $10^5$ cells).

Each of CAR-T 001-B and CAR-T 009-B to CAR-T 014-B [effector (E)] was diluted with an RPMI 1640 medium containing 10% FBS so as to attain an E:T ratio of 1:1 and 1:10. Specifically, CAR-T for a sample with E:T ratio =1:1 was adjusted to 2 × $10^5$ cells/mL and added at 500 μL/well to the 48-well treatment culture plate containing the seeded PMNs, PBMCs, or MV4-11 cells (CAR-T cell number in a well was 1 × $10^5$ cells).

Likewise, CAR-T for a sample with E:T ratio = 1:10 was adjusted to 0.2 × $10^5$ cells/mL and added at 500 μL/well to the 48-well treatment culture plate containing the seeded PMNs, PBMCs, or MV4-11 cells (CAR-T cell number in a well was 0.1 × $10^5$ cells).

<Coculture test with PMNs>

The coculture test was conducted for 3 days. Mock-T cells were also subjected to the same coculture test. A group of cultured PMNs having the same cell number was also established as a control group (CAR-T non-addition group).

On day 3 of the coculture test, the cells in each well were recovered and centrifuged. The cells were suspended by the addition of 0.1 μL of FITC Anti-Human CD3 antibody (BioLegend, Inc.), 5 μL of PE Anti-Human GM-CSF antibody (Miltenyi Biotec GmbH), and 0.1 μL of APC Anti-Human CD11b antibody (BioLegend, Inc.) and subjected to antibody labeling reaction at 4°C for 20 minutes in the dark. 20 minutes later, the cells were washed with an appropriate amount of D-PBS and precipitated by centrifugation. After removal of the supernatant, the cells were resuspended in 450 μL of D-PBS, and 50 μL of counting beads was added thereto. This sample was analyzed using FACSCanto II. The number of living cells was measured with CD3-CD11b+ cells as neutrophils. The measured cell number was decupled to calculate a numerical value as a viable cell number. The relative ratio to a value obtained from the culture of PMNs alone was calculated.

<Coculture test with PBMCs>

The coculture test was conducted for 3 days. Mock-T cells were also subjected to the same coculture test. A group of cultured PBMCs having the same cell number was also established as a control group (CAR-T non-addition group).

On day 3 of the coculture test, the cells in each well were recovered and centrifuged. The cells were suspended by the addition of 0.1 μL of FITC Anti-Human CD3 antibody, 5 μL of PE Anti-Human GM-CSF antibody, 0.1 μL of APC Anti-Human CD11b antibody, 0.1 μL of Pacific blue Anti-Human CD16 antibody (BioLegend, Inc.), and 0.1 μL of APC-cy7 Anti-Human CD19 antibody (BioLegend, Inc.) and subjected to antibody labeling reaction at 4°C for 20 minutes in the dark. 20 minutes later, the cells were washed with an appropriate amount of D-PBS and precipitated by centrifugation. After removal of the supernatant, the cells were resuspended in 450 μL of D-PBS, and 50 μL of counting beads was added thereto. This sample was analyzed using FACSCanto II. The number of living cells was measured with CD3-CD11b+ cells as monocytes, CD3-CD16+ cells as NK cells, and CD3-CD19+ cells as B cells. The measured cell number was decupled to calculate a numerical value as a viable cell number. The relative ratio to a value obtained from the culture of PBMCs alone was calculated.

<Coculture test with MV4-11 cells>

The coculture test was conducted for 3 days. Mock-T cells were also subjected to the same coculture test. A group of cultured MV4-11 cells having the same cell number was also established as a control group (CAR-T non-addition group).

On day 3 of the coculture test, the cells in each well were recovered and centrifuged. The cells were suspended by the addition of 5 μL of APC Anti-Human CD33, 0.2 μL of FITC Anti-Human CD3, and 5 μL of PE Anti-Human GM-CSF and subjected to antibody labeling reaction at 4°C for 20 minutes in the dark. 20 minutes later, the cells were washed with an appropriate amount of D-PBS and precipitated by centrifugation. After removal of the supernatant, the cells were resuspended in 450 μL of D-PBS, and 50 μL of counting beads was added thereto. This sample was analyzed using FACSCanto II. A CD33+ live tumor cells were measured. The measured cell number was decupled to calculate a numerical value as a viable tumor cell number. The relative ratio to a value obtained from the culture of MV4-11 cells alone was calculated.

Figure 19 shows the relative ratio of viable tumor cell number targeting MV4-11 and the relative ratio of viable cell number targeting peripheral blood cells PMNs and PBMCs at E:T ratios of 1:1 and 1:10. All of CAR-T 001-B and CAR-T009-B to CAR-T014-B killed almost all of tumor cells at the E:T ratio of 1:1 without influencing peripheral blood cells such as B cells, NK cells, and neutrophils. At the E:T ratio of 1:10, all of CAR-T 001-B and CAR-T 009-B to CAR-T 014-B killed neither tumor cells nor peripheral blood cells. These results demonstrated that CAR-T 001-B and CAR-T 009-B to CAR-T 014-B at a cell number having the ability to kill tumor cells have no influence on peripheral blood cells such as B cells, NK cells, and neutrophils.

[Example 11 Effect of GMR.CAR-T on bone marrow cells]

In order to evaluate CAR-T 001-A and CAR-T 009 to CAR-T 014 produced in Example 7 for their influence on normal bone marrow cells, a colony formation test using bone marrow cells was conducted.

Specifically, a CD34-positive fraction of healthy human-derived bone marrow cells (VERITAS Corp.) [target (T)] was adjusted to $1 \times 10^4$ cells/mL with an RPMI 1640 medium containing 10% FBS, and 20 ng/mL each of IL-3, SCF and TPO (Thermo Fisher Scientific Inc.). Likewise, MV4-11 cells [target (T)] were adjusted to $6 \times 10^3$ cells/mL with an RPMI 1640 medium containing 10% FBS, and 20 ng/mL each of IL-3, SCF and TPO (Thermo Fisher Scientific Inc.).

The target cells of each group thus adjusted were seeded at 50 μL/well to a 96-well round-bottomed plate. Each of CAR-T 001-A and CAR-T 009 to CAR-T 014 [effector (E)] was diluted with an RPMI 1640 medium containing 10% FBS so as to attain an E:T ratio of 20:1, 6:1, 2:1, 0.6:1, and 0.2:1 for the CD34-positive fraction of healthy human-derived bone marrow cells and an E:T ratio of 1:1, 0.5:1, 0.25:1, and 0.125:1 for MV4-11, and seeded at 50 μL/well to the plate. Specifically, the effector and the target were cocultured at 100 μL/well at the E:T ratios described above in an RPMI 1640 medium containing 10% FBS, and 10 ng/mL each of IL-3, SCF and TPO in the 96-well round-bottomed plate.

The coculture was conducted for 2 days. Mock-T cells without gene introduction were also subjected to the same coculture test. A group of the CD34-positive fraction of healthy human-derived bone marrow cells alone or MV4-11 cells alone cultured at the same cell number was also established as a control group (CAR-T non-addition group).

2 days later, 100 μL of the coculture solution and 1 mL of MethoCult Classic (VERITAS Corp.) were mixed and seeded to SmartDish 6-Well Plates (VERITAS Corp.). 7 days or later after seeding, colony formation was confirmed, followed by counting. For the CD34-positive fraction, plural types of colonies are observed and are difficult to discriminate. For this reason, the colonies were divided into colonies of erythroid series and colonies of myeloid series, which were then counted using an automatic colony counting apparatus STEMvision (VERITAS Corp.). On the other hand, the colonies of MV4-11 were manually counted under a microscope because there existed only one type of colony without the need of discrimination.

Figure 20 shows a sigmoid curve drawn from points plotted at the respective E:T ratios targeting the CD34-positive fraction of healthy human-derived bone marrow cells. The plot employed means from 3 wells for the groups other than the control group and means from 6 wells for the control group. The sigmoid curve was output using GraphPad Prism 4 ver. 4.03 (GraphPad), and fitting was carried out according to the following formula with parameters set to TOP = 100 and BOTTOM ≠ 0.

[Formula 1]

Formula: Y=Bottom+(Top-Bottom)/(1+10^((logEC50-X)*HillSlope))

Figure 21 shows a sigmoid curve drawn from points plotted at the respective E:T ratios targeting MV4-11. The plot employed means from 2 wells. For the sigmoid curve, fitting was carried out according to the formula described above with parameters set to TOP = 100 and BOTTOM = 0.

Figure 22 shows the IC50 value calculated from Figure 20, the EC50 value calculated from Figure 21, and the margin of safety calculated therefrom. As a result, CAR-T 014 had the greatest margin of safety for largely susceptible myeloid series.

[Example 12 *In vivo* effect of GMR.CAR-T - 1]

Electrical introduction operation of the expression plasmids (CAR 009, CAR 011 and CAR 012) into PBMCs and T cell culture and amplification operation were performed in the same way as in Example 10 to newly produce GMR.CAR-T cells (CAR-T 009-C, CAR-T 011-C and CAR-T 012-C, respectively). The expression analysis results about CAR-T 009-C, CAR-T 011-C and CAR-T 012-C used in this Example on day 14 are shown in Figure 23. The GMR.CAR expression rate in each cell group was CAR-T 009-C: 25.7%, CAR-T 011-C: 32.1%, and CAR-T 012-C: 25.2%.

In order to confirm the effects of these GMR.CAR-T cells in THP-1 cancer-bearing mouse models, an *in vivo* experiment was conducted as described below. The mice used were 7-week-old female NSG mice NOD.Cg-Prkdc<scid >Il2rg< tmlWjl >/SzJ (Charles River Laboratories Japan, Inc.), which were delivered at the age of 6 weeks, then acclimatized for 6 days, and then used in the experiment.

On day 0, THP-1 cell line was suspended in 150 μL of D-PBS to be 1 × $10^6$ cells/mouse and intravenously administered to the necks of the NSG mice under isoflurane anesthesia.

On day 3, the CAR-T cells on day 16 of culture were suspended in 150 μL of D-PBS to be 5 × $10^6$ total T cells/mouse and intravenously administered to the necks of the NSG mice under isoflurane anesthesia.

The states of the mice were observed at intervals of 1 to 2 days until day 148, and Kaplan-Meier analysis was conducted. Statistical analysis was conducted by Log-rank (Cochran-Mantel-Haenszel) tests.

The states of the mice were also observed, and mice considered to reach the following humane endpoints were euthanized and regarded as being dead.

Humane endpoints:

Dragging inferior limbs
Having a tumor mass diameter of 20 mm
Body weight decreasing by 25% or more in 7 days

As a result, as shown in Figure 24, CAR-T 009-C (P value = 0.0086), CAR-T 011-C (P value = 0.0011) and CAR-T 012-C (P value = 0.0011) were found to significantly prolong the survival periods of the mice as compared with PBS administration group.

The statistical analysis between the mice given CAR-T 011-C or CAR-T 012-C and the mice given CAR-T 009-C showed that CAR-T 011-C (P value = 0.0042) and CAR-T 012-C (P value = 0.0136) significantly prolong the survival periods of the mice as compared with CAR-T 009-C.

These results demonstrated that in THP-1 cancer-bearing mouse models, CAR-T 011-C and CAR-T 012-C have stronger antitumor effect than that of CAR-T 009-C.

[Example 13 *In vivo* effect of GMR.CAR-T - 2]

Electrical introduction operation of the expression plasmids (CAR 009 to CAR 014) into PBMCs and T cell culture and amplification operation were performed in the same way as in Example 10 to newly produce GMR.CAR-T cells (CAR-T 009-D to CAR-T 014-D). The expression analysis results about CAR-T 009-D to CAR-T 014-D used in this Example on day 14 are shown in Figure 25. The GMR.CAR expression rate in each cell group was CAR-T 009-D: 33.3%, CAR-T 010-D: 39.6%, CAR-T 011-D: 35.1%, CAR-T 012-D: 37.4%, CAR-T 013-D: 43.0%, and CAR-T 014-D: 40.9%.

CAR-positive cell numbers were calculated from the obtained expression rates and cell numbers, and CAR-positive cells to be administered to each group were adjusted to 1.2 × $10^6$ cells. In order to confirm effects in MV4-11 cancer-bearing mouse models, an *in vivo* experiment was conducted as described below in the same way as in Example 12. Eight-week-old female NSG mice were used, and the mice were delivered at the age of 6 weeks, then acclimatized for 13 days, and then used in the experiment.

On day 0, MV4-11 cell line was suspended in 150 μL of D-PBS to be 1 × $10^6$ cells/mouse and intravenously administered to the necks of the NSG mice under isoflurane anesthesia.

On day 3, the CAR-T cells on day 16 of culture were suspended in 150 μL of D-PBS to be 1.2 × $10^6$ CAR-positive cells/mouse and intravenously administered to the necks of the NSG mice under isoflurane anesthesia.

The states of the mice were observed at intervals of 1 to 2 days until day 116, and Kaplan-Meier analysis was conducted. Statistical analysis was conducted by Log-rank (Cochran-Mantel-Haenszel) tests. The states of the mice were also observed, and mice considered to reach the humane endpoints were euthanized and regarded as being dead in the same way as in Example 12.

On day 52, approximately 100 μL of blood was collected using a hematocrit capillary coated with EDTA-2K (Tokyo Garasu Kikai Co., Ltd.), and the amount of residual tumor cells in the blood was quantified by commissioned quantitative PCR (MLL-AF4 chimeric mRNA quantification (SRL)).

As a result, as shown in Figures 26 and 27, CAR-T 009-D (P value = 0.0042), CAR-T 011-D (P value = 0.0216), CAR-T 012-D (P value = 0.0042)), CAR-T 010-D (P value = 0.0042), CAR-T 013-D (P value = 0.0042) and CAR-T 014-D (P value = 0.0042) were found to significantly prolong the survival periods of the mice as compared with PBS administration group.

The results of Kaplan-Meier analysis on day 116 and quantitative PCR of tumor in peripheral blood on day 52 shown in Figure 28 demonstrated that in MV4-11 cancer-bearing mouse models, CAR-T 010-D and CAR-T 014-D have stronger antitumor effect than that of CAR-T 013-D.

[Example 14 *In vivo* effect of GMR.CAR-T - 3]

Electrical introduction operation of the expression plasmids (CAR 001 and CAR 014) into PBMCs and T cell culture and amplification operation were performed in the same way as in Example 10 to newly produce GMR.CAR-T cells (CAR-T 001-C and CAR-T 014-F). CD19.CAR-T was similarly produced as a comparative control for expression rate calculation using the CH2CH3-free CD19.CAR plasmid described in Morita et al., Molecular Therapy: Methods & Clinical Development Vol. 8 March 2018.

The expression analysis results about CAR-T 001-C and CAR-T 014-F used in this Example on day 14 are shown in Figure 29. The GMR.CAR expression rate in each cell group was CAR-T 001-C: 32.3% and CAR-T 014-F: 14.1%.

CAR-positive cell numbers were calculated from the obtained expression rates and cell numbers, and CAR-positive cells to be administered to each group were adjusted to $0.7 \times 10^6$ cells. In order to confirm effects in MV4-11 cancer-bearing mouse models, an *in vivo* experiment was conducted as described below in the same way as in Example 12. Seven-week-old female NSG mice were used, and the mice were delivered at the age of 6 weeks, then acclimatized for 6 days, and then used in the experiment.

On day 0, MV4-11 cell line was suspended in 150 μL of D-PBS to be $1 \times 10^6$ cells/mouse and intravenously administered to the tails of the NSG mice without anesthesia.

On day 3, the CAR-T cells on day 16 of culture were suspended in 150 μL of D-PBS to be $0.7 \times 10^6$ CAR-positive cells/mouse and intravenously administered to the tails of the NSG mice without anesthesia.

The states of the mice were observed at intervals of 1 to 2 days until day 105, and Kaplan-Meier analysis was conducted. Statistical analysis was conducted by Log-rank (Cochran-Mantel-Haenszel) tests. The states of the mice were also observed, and mice considered to reach the humane endpoints were euthanized and regarded as being dead in the same way as in Example 12.

As a result, as shown in Figure 30, CAR-T 014-F (P value = 0.0050) was found to significantly prolong the survival periods of the mice as compared with PBS administration group, and CAR-T 014-F (P value = 0.0081) was also found to significantly prolong the survival periods of the mice as compared with CAR-T 001-C.

[Example 15 Expression analysis of GMRα (CD116) on tumor cell surface]

The expression rate of GMRα (CD116) on the cell surface of the tumor cell lines used in anti-tumor cell activity evaluation in Examples described above was analyzed by flow cytometry. Acute myelocytic leukemia (AML) cell lines THP-1, Kasumi-1, shinAML-1, and MV4-11 were analyzed. Specifically, 1 to $2 \times 10^5$ cells were collected, suspended by the addition of 5 μL of APC Anti-Human CD116 antibody (Miltenyi Biotec GmbH) and 5 μL of PE Anti-Human CD33 antibody (Miltenyi Biotec GmbH), and subjected to antibody labeling reaction at 4°C for 20 minutes in the dark. 20 minutes later, the cells were washed with an appropriate amount of D-PBS and precipitated by centrifugation. After removal of the supernatant, the cells were resuspended in an appropriate amount of D-PBS. This sample was analyzed using FACSCalibur to determine the expression rate of CD33/CD116.

Table 1 shows the CD33/CD116 expression rate on the surface of each tumor cell. The CD33/CD116 positive rate was 90% or higher for all of THP-1, Kasumi-1, shinAML-1 and MV4-11.

[Table 1]

| Cell line | CD33-positive/CD116-positive (%) |
|---|---|
| THP-1 | 99.21 |
| Kasumi-1 | 94.24 |
| shinAML-1 | 98.93 |
| MV4-11 | 99.84 |

[Example 16 Phenotype analysis of GMR.CAR-expressing T cells]

Electrical introduction operation of the expression plasmid CAR 014 into PBMCs and T cell culture and amplification operation were performed in the same way as in Example 7 to newly produce GMR.CAR-T cells (CAR-T 014-E). The expression analysis results about CAR-T 014-E on day 14 are shown in Figure 31. The GMR.CAR expression rate was 9.75%, which was higher as compared with the expression rate in Mock-T cells (1.75%). Thus, GMR.CAR-expressing T cells were successfully produced.

The cell number of the produced GMR.CAR-expressing T cells was counted, and $1 \times 10^6$ GMR.CAR-T cells were evaluated for their phenotype by flow cytometry analysis. $1 \times 10^6$ cells were collected and centrifuged. The cells were subjected to block treatment of an Fc receptor by the addition of Human BD Fc Block (Nippon Becton Dickinson Co., Ltd.), then suspended by the addition of appropriate amounts of BB515 Mouse AntiHuman CD197 antibody (Nippon Becton Dickinson Co., Ltd.), PE Mouse Anti-Human CD27 antibody (Nippon Becton Dickinson Co., Ltd.), APC Mouse Anti-Human CD62L antibody (Nippon Becton Dickinson Co., Ltd.), APC-H7 Mouse Anti-Human CD45RO antibody (Nippon Becton Dickinson Co., Ltd.), Brilliant Violet421 Mouse Anti-Human CD56 antibody (Nippon Becton Dickinson Co., Ltd.), BV510 Mouse Anti-Human CD8 antibody (Nippon Becton Dickinson Co., Ltd.), BV605 Mouse Anti-Human CD45RA antibody (Nippon Becton Dickinson Co., Ltd.), BV650 Mouse Anti-Human CD3 antibody (Nippon Becton Dickinson Co., Ltd.) and BV786 Mouse Anti-Human CD4 antibody (Nippon Becton Dickinson Co., Ltd.), and subjected to antibody labeling reaction at 4°C for 20 minutes in the dark.

20 minutes later, the cells were washed with an appropriate amount of D-PBS containing 3% FBS and precipitated by centrifugation. After removal of the supernatant, the cells were resuspended in an appropriate amount of D-PBS containing 3% FBS. This sample was analyzed using FACSCelesta (Nippon Becton Dickinson Co., Ltd.) and FLOWJO (Nippon Becton Dickinson Co., Ltd.). The phenotype of the GMR.CAR was analyzed using CD3 positivity and CD45RA/CD62L positivity or CD3 positivity and CD45RA/CD197 positivity as indexes. As a result, 60% or more of the produced GMR.CAR-expressing T cells were stem cell memory T cells.

## Industrial Applicability

The present invention provides CAR-T cells that specifically binds to a target cell expressing a human granulocyte-macrophage colony stimulating factor (GM-CSF) receptor on the cell surface and providing an excellent cytotoxic activity. The cell of the present invention can be used for an adoptive immunotherapy for diseases such as AML.

All publications, patents and patent applications cited in the description are incorporated in their entirety by reference.

## Claims

1. A genetically modified cell having introduced thereinto, a polynucleotide encoding a chimeric antigen receptor (CAR) protein having a target binding domain that specifically binds to a human granulocyte-macrophage colony stimulating factor (GM-CSF) receptor, a transmembrane domain, and an intracellular signaling domain, wherein the target binding domain is a mutant polypeptide having a sequence in which glutamic acid at position 21 in the amino acid sequence shown in SEQ ID NO: 1 is substituted with another amino acid.

2. The cell according to claim 1, wherein the cell expresses the CAR protein that binds to a GM-CSF receptor on a cell membrane thereof.

3. The cell according to claim 1 or 2, wherein the CAR protein further comprises a costimulatory domain and/or an extracellular spacer domain.

4. The cell according to any one of claims 1 to 3, wherein the intracellular signaling domain is human CD3ζ.

5. The cell according to claim 3 or 4, wherein the co-stimulatory domain is human CD28 or human 4-1BB.

6. The cell according to any one of claims 3 to 5, wherein the extracellular spacer domain comprises a hinge, CH2 and/or CH3 region of human IgG1 or a part thereof, and/or an artificial spacer sequence.

7. The cell according to any one of claims 1 to 6, wherein the target-binding domain is a mutant polypeptide having a sequence in which glutamic acid at position 21 in the amino acid sequence shown in SEQ ID NO: 1 is substituted with arginine or lysine.

8. A method for producing a CAR protein-expressing cell, comprising introducing a polynucleotide encoding a chimeric antigen receptor (CAR) protein that specifically binds to a human GM-CSF receptor to a cell using a vector, wherein the protein comprises an amino acid sequence in which glutamic acid at position 21 in the amino acid sequence shown in SEQ ID NO: 1 is substituted with another amino acid.

9. A vector comprising a polynucleotide encoding a chimeric antigen receptor (CAR) protein that specifically binds to a human GM-CSF receptor, wherein the protein comprises an amino acid sequence in which glutamic acid at position 21 in the amino acid sequence shown in SEQ ID NO: 1 is substituted with another amino acid.

10. A therapeutic agent for a disease involving a GM-CSF receptor-expressing cell, comprising the cell according to any one of claims 1 to 7.

11. A pharmaceutical composition comprising the therapeutic agent according to claim 10 and a pharmaceutically acceptable carrier.

12. The therapeutic agent according to claim 10 or the composition according to claim 11, wherein the disease involving a GM-CSF receptor expressing cell is selected from juvenile myelomonocytic leukemia (JMML), acute myelocytic leukemia (AML), glioma, neuroblastoma, glioblastoma, brain tumor, colorectal adenocarcinoma, prostate cancer, kidney cancer, melanoma and small cell lung cancer.

Fig. 1

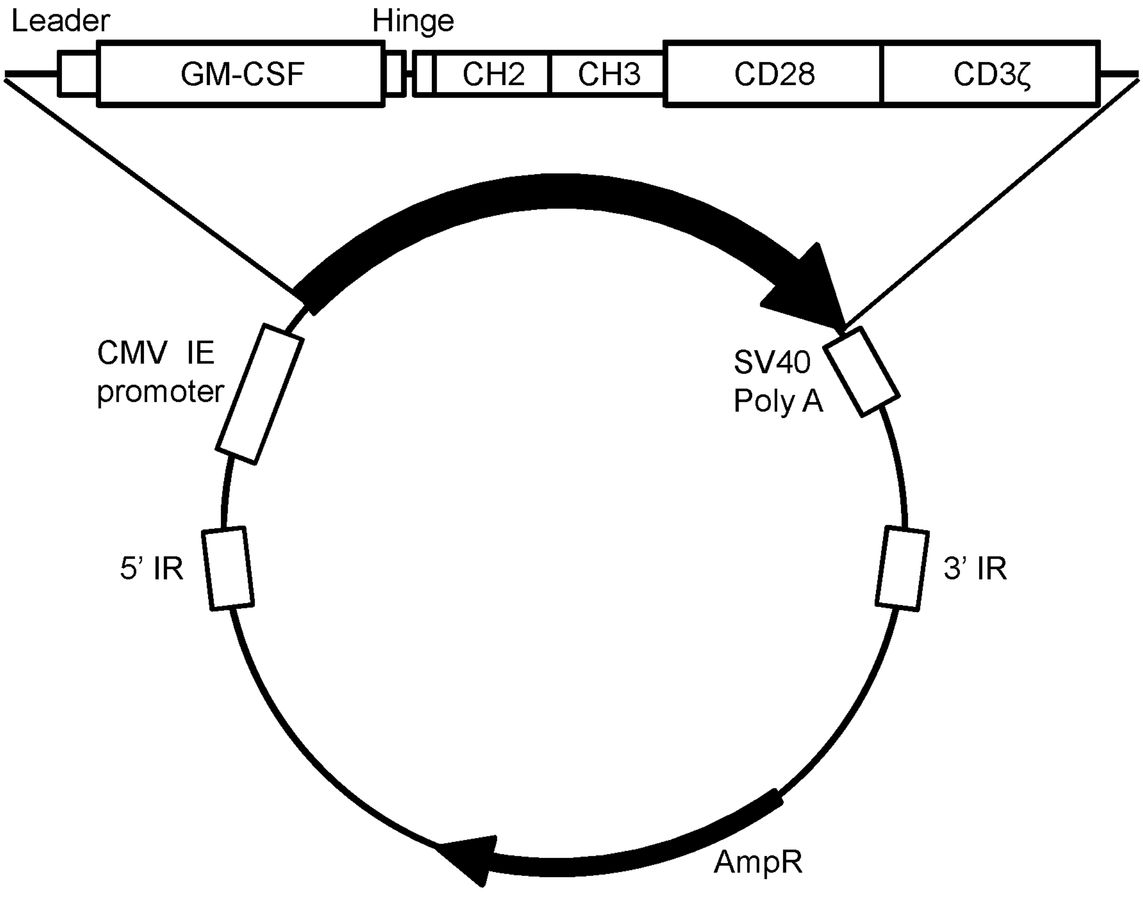
Leader
GM-CSF
Hinge
CH2
CH3
CD28
CD3ζ
CMV IE promoter
SV40 Poly A
5' IR
3' IR
AmpR

Fig. 2

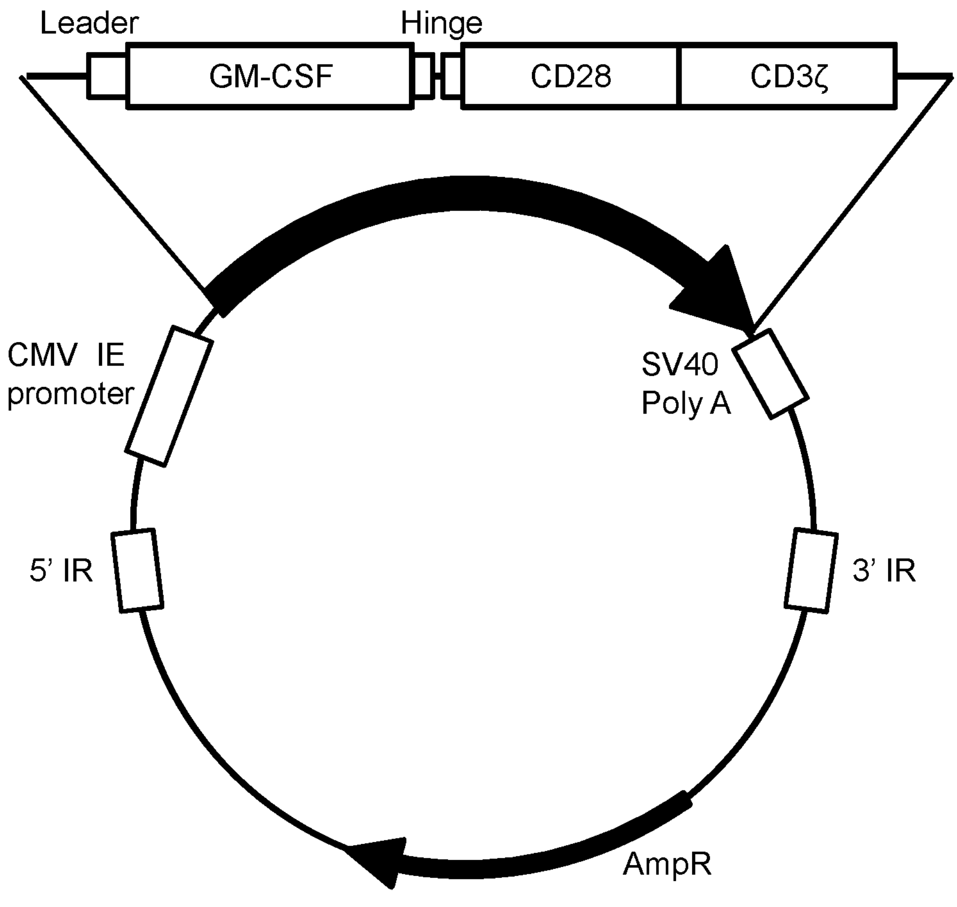
Leader
Hinge
GM-CSF
CD28
CD3ζ
CMV IE promoter
SV40 Poly A
5' IR
3' IR
AmpR

Fig. 3

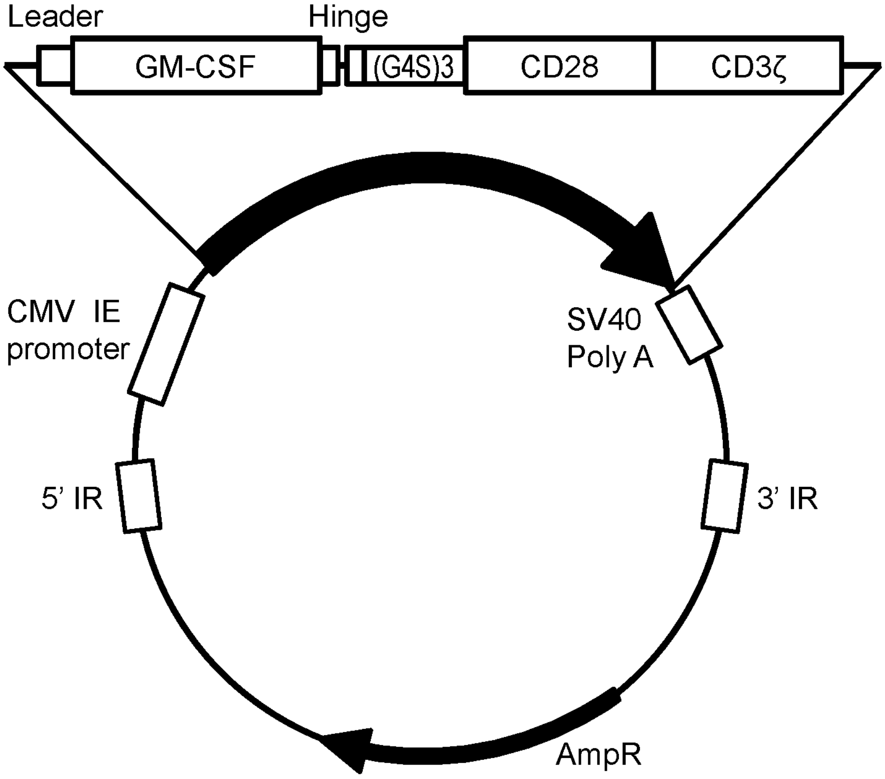
Leader
Hinge
GM-CSF
(G4S)3
CD28
CD3ζ
CMV IE promoter
SV40 Poly A
5' IR
3' IR
AmpR

Fig. 4

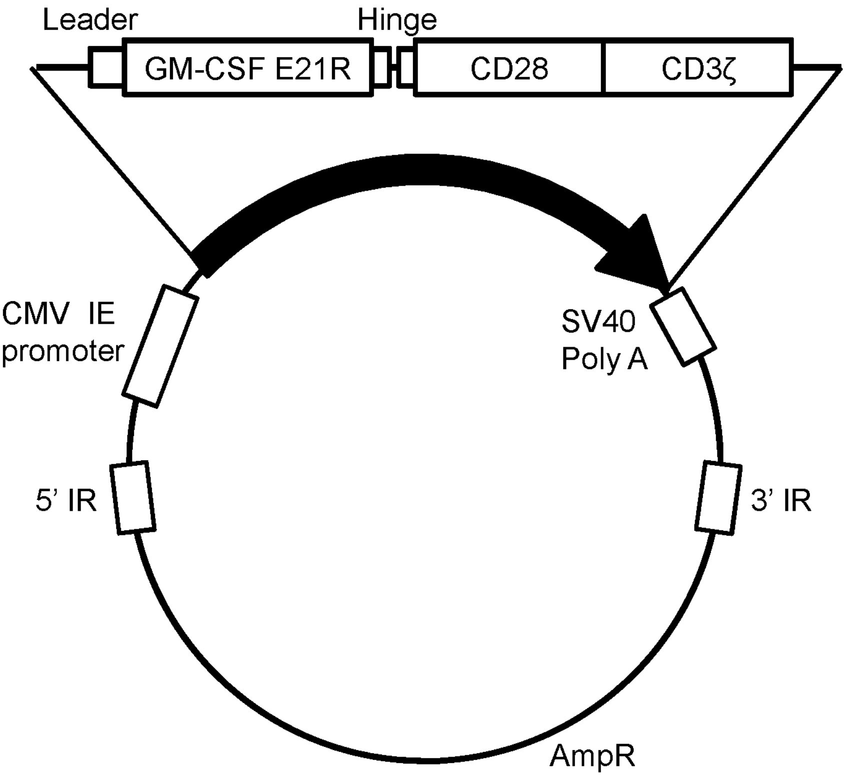
Leader
Hinge
GM-CSF E21R
CD28
CD3ζ
CMV IE promoter
SV40 Poly A
5' IR
3' IR
AmpR

Fig. 5

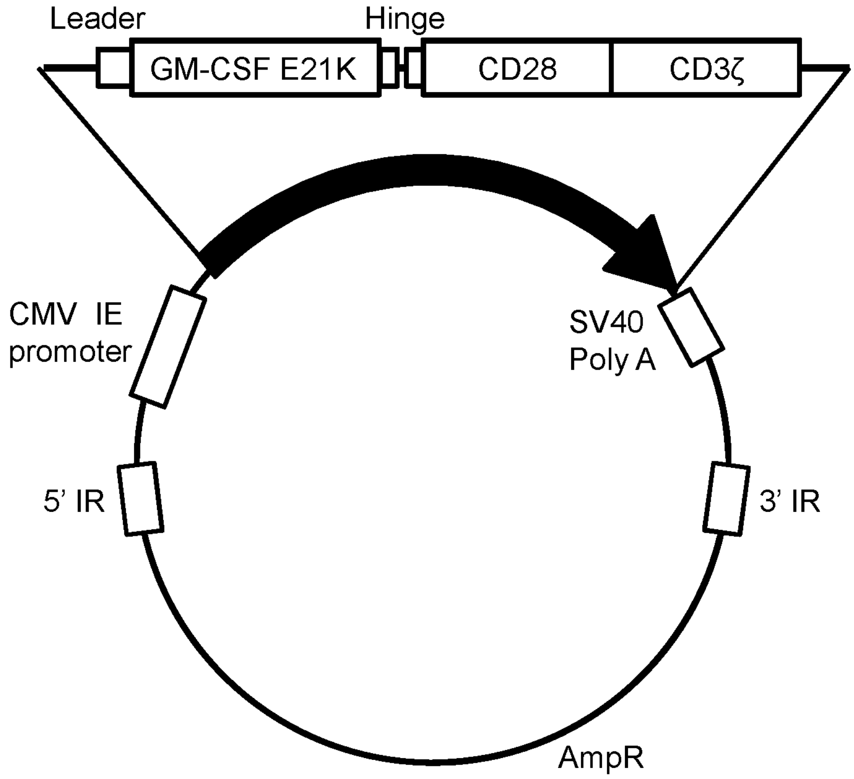
Leader
Hinge
GM-CSF E21K
CD28
CD3ζ
CMV IE
promoter
SV40
Poly A
5' IR
3' IR
AmpR

Fig. 6

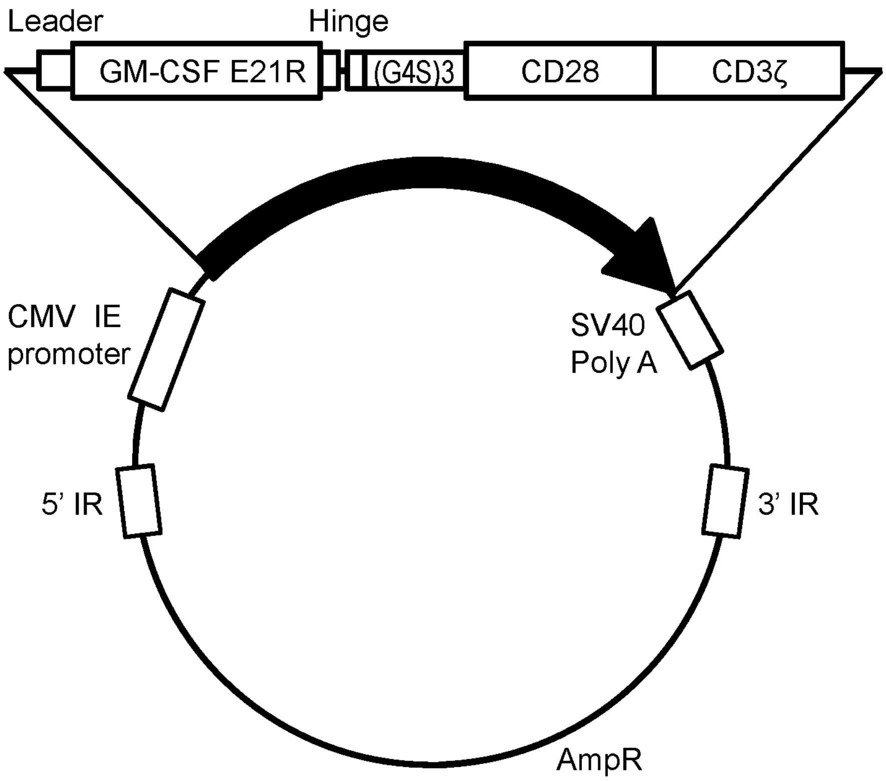
Leader
Hinge
GM-CSF E21R
(G4S)3
CD28
CD3ζ
CMV IE
promoter
SV40
Poly A
5' IR
3' IR
AmpR

Fig. 7

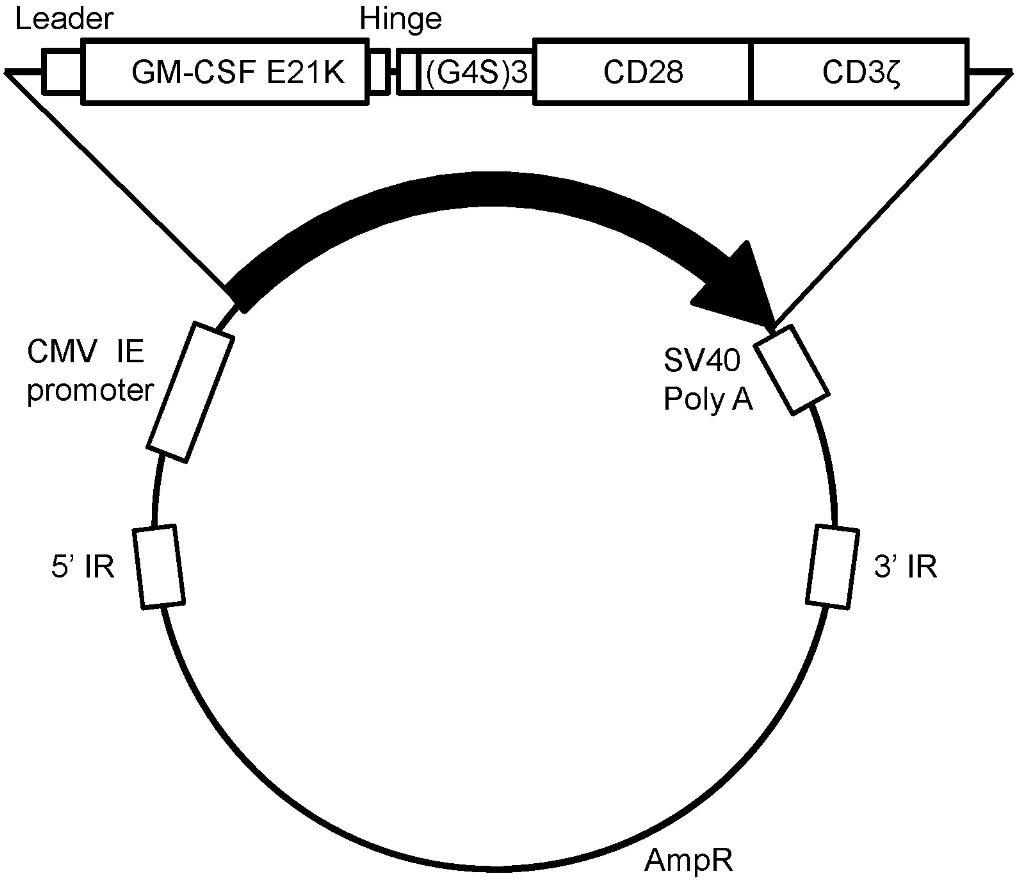
Leader
GM-CSF E21K
Hinge
(G4S)3
CD28
CD3ζ
CMV IE promoter
SV40 Poly A
5' IR
3' IR
AmpR

Fig. 8

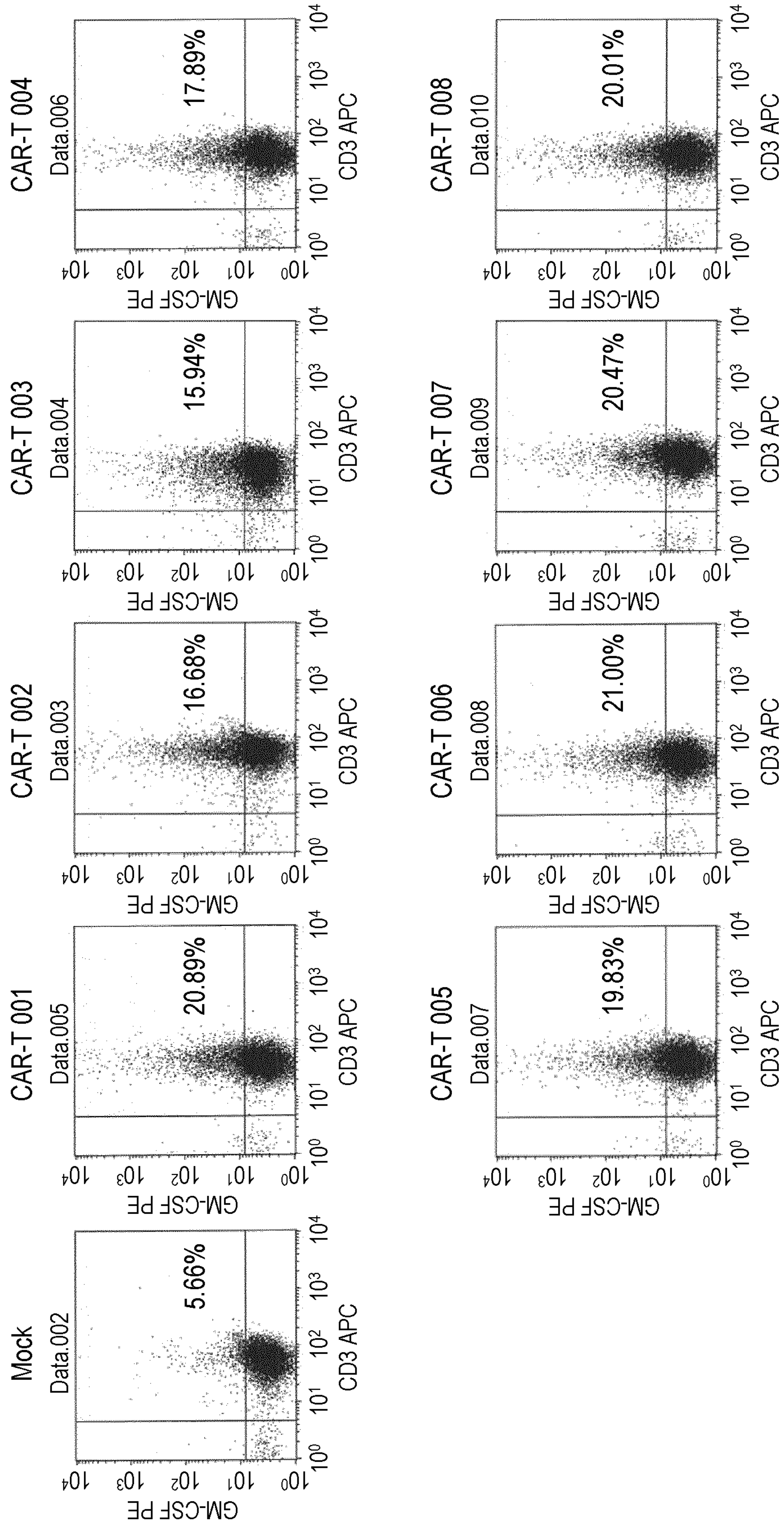
Mock
Data.002
5.66%
CAR-T 001
Data.005
20.89%
CAR-T 002
Data.003
16.68%
CAR-T 003
Data.004
15.94%
CAR-T 004
Data.006
17.89%
CAR-T 005
Data.007
19.83%
CAR-T 006
Data.008
21.00%
CAR-T 007
Data.009
20.47%
CAR-T 008
Data.010
20.01%
GM-CSF PE
CD3 APC

Fig. 9

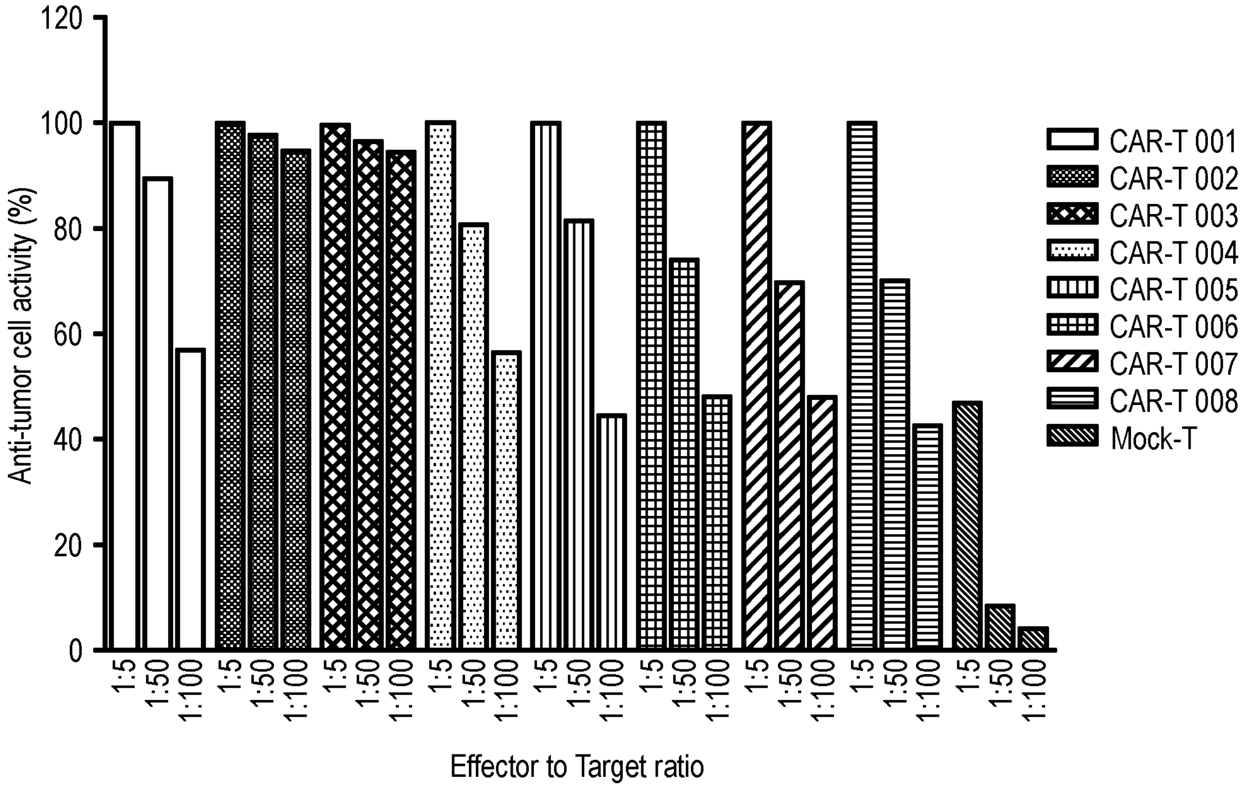
120
100
80
60
40
20
0
Anti-tumor cell activity (%)
1:5
1:50
1:100
Effector to Target ratio
CAR-T 001
CAR-T 002
CAR-T 003
CAR-T 004
CAR-T 005
CAR-T 006
CAR-T 007
CAR-T 008
Mock-T

Fig. 10

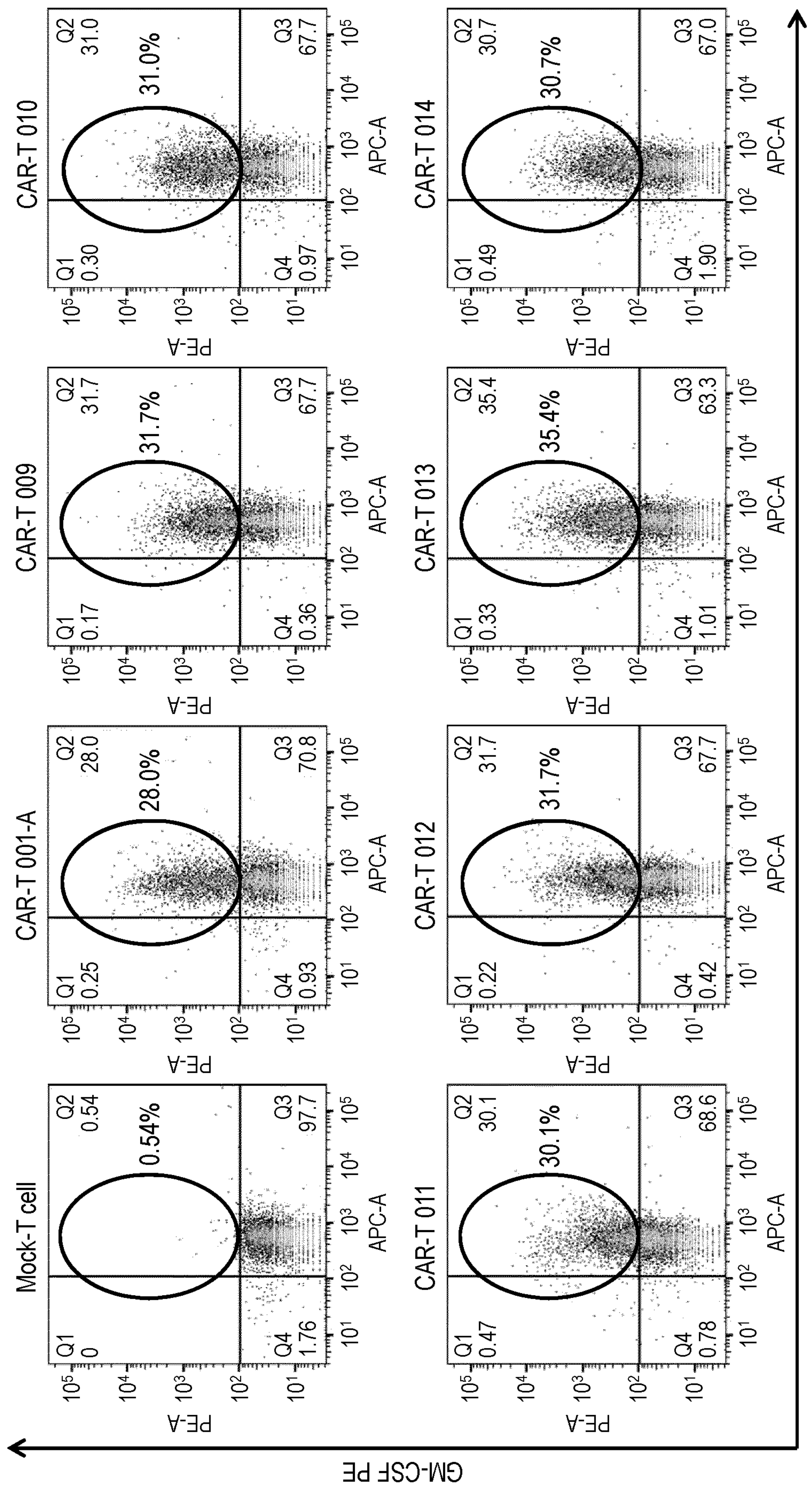
Mock-T cell
Q1 0
Q2 0.54
Q3 97.7
Q4 1.76
0.54%
CAR-T 001-A
Q1 0.25
Q2 28.0
Q3 70.8
Q4 0.93
28.0%
CAR-T 009
Q1 0.17
Q2 31.7
Q3 67.7
Q4 0.36
31.7%
CAR-T 010
Q1 0.30
Q2 31.0
Q3 67.7
Q4 0.97
31.0%
CAR-T 011
Q1 0.47
Q2 30.1
Q3 68.6
Q4 0.78
30.1%
CAR-T 012
Q1 0.22
Q2 31.7
Q3 67.7
Q4 0.42
31.7%
CAR-T 013
Q1 0.33
Q2 35.4
Q3 63.3
Q4 1.01
35.4%
CAR-T 014
Q1 0.49
Q2 30.7
Q3 67.0
Q4 1.90
30.7%
PE-A
APC-A
CD3 APC
GM-CSF PE

Fig. 11

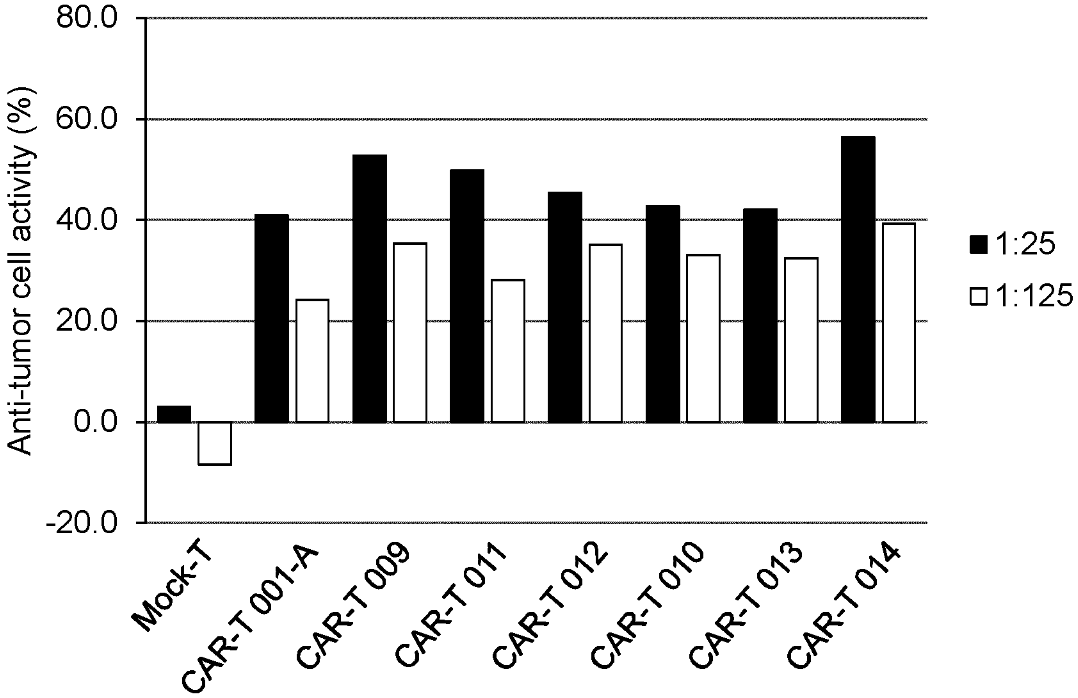
80.0
60.0
40.0
20.0
0.0
-20.0
Anti-tumor cell activity (%)
Mock-T
CAR-T 001-A
CAR-T 009
CAR-T 011
CAR-T 012
CAR-T 010
CAR-T 013
CAR-T 014
1:25
1:125

Fig. 12

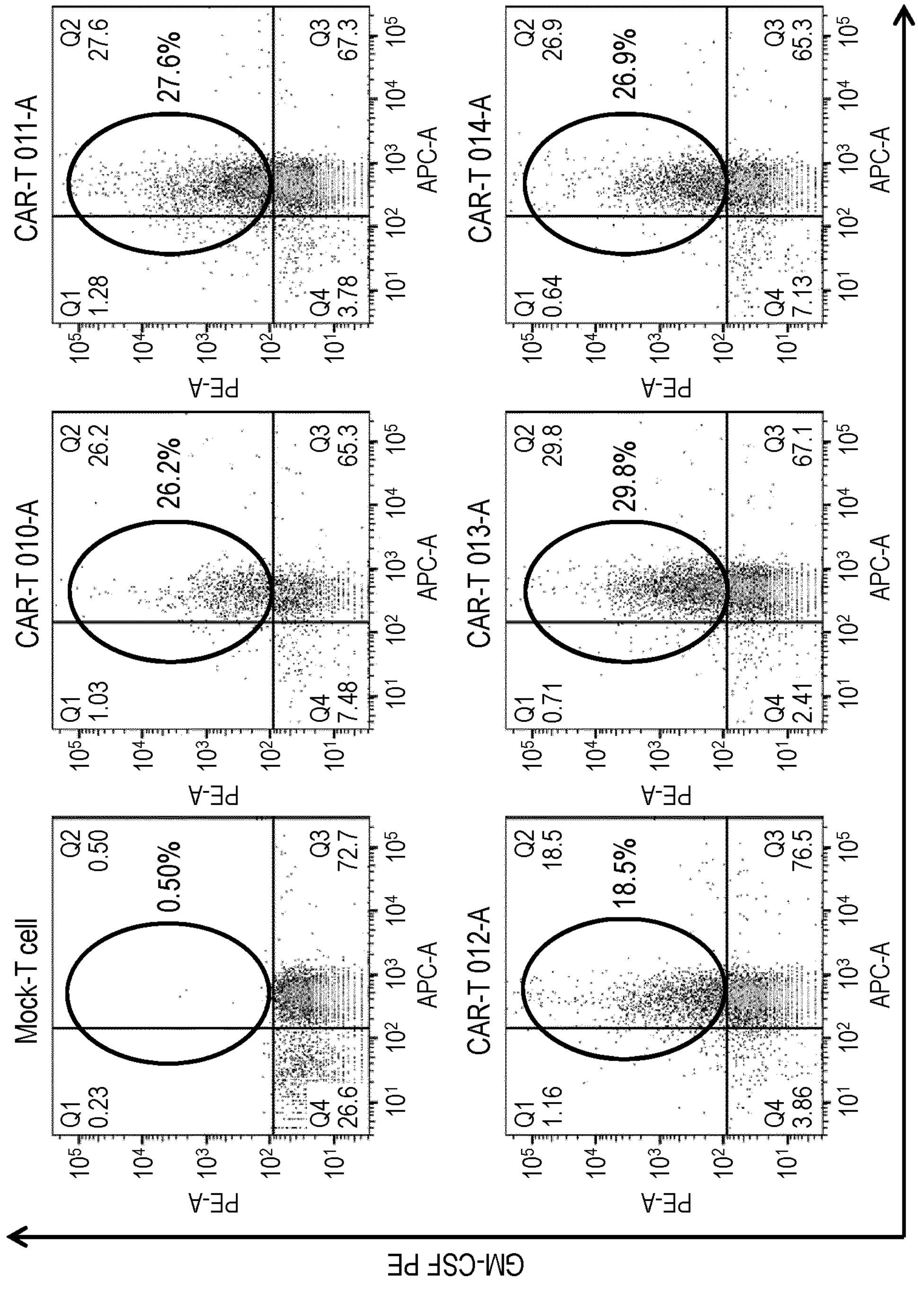
Mock-T cell
Q1 0.23
Q2 0.50
Q3 72.7
Q4 26.6
0.50%
CAR-T 010-A
Q1 1.03
Q2 26.2
Q3 65.3
Q4 7.48
26.2%
CAR-T 011-A
Q1 1.28
Q2 27.6
Q3 67.3
Q4 3.78
27.6%
CAR-T 012-A
Q1 1.16
Q2 18.5
Q3 76.5
Q4 3.86
18.5%
CAR-T 013-A
Q1 0.71
Q2 29.8
Q3 67.1
Q4 2.41
29.8%
CAR-T 014-A
Q1 0.64
Q2 26.9
Q3 65.3
Q4 7.13
26.9%
PE-A
APC-A
CD3 APC
GM-CSF PE

Fig. 13

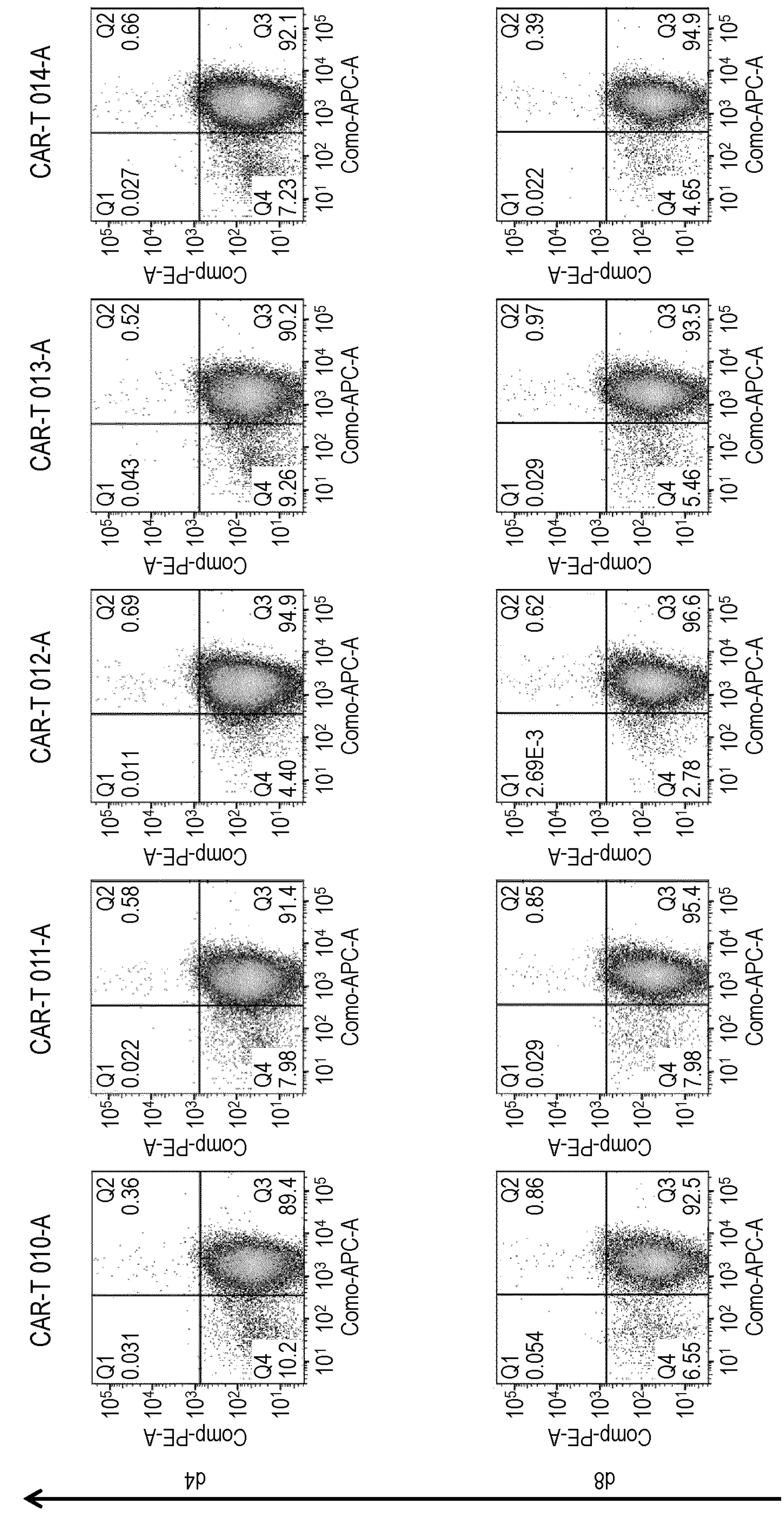
d4
d8
CAR-T 010-A
CAR-T 011-A
CAR-T 012-A
CAR-T 013-A
CAR-T 014-A
Comp-PE-A
Como-APC-A
d4 CAR-T 010-A: Q1 0.031, Q2 0.36, Q3 89.4, Q4 10.2
d4 CAR-T 011-A: Q1 0.022, Q2 0.58, Q3 91.4, Q4 7.98
d4 CAR-T 012-A: Q1 0.011, Q2 0.69, Q3 94.9, Q4 4.40
d4 CAR-T 013-A: Q1 0.043, Q2 0.52, Q3 90.2, Q4 9.26
d4 CAR-T 014-A: Q1 0.027, Q2 0.66, Q3 92.1, Q4 7.23
d8 CAR-T 010-A: Q1 0.054, Q2 0.86, Q3 92.5, Q4 6.55
d8 CAR-T 011-A: Q1 0.029, Q2 0.85, Q3 95.4, Q4 7.98
d8 CAR-T 012-A: Q1 2.69E-3, Q2 0.62, Q3 96.6, Q4 2.78
d8 CAR-T 013-A: Q1 0.029, Q2 0.97, Q3 93.5, Q4 5.46
d8 CAR-T 014-A: Q1 0.022, Q2 0.39, Q3 94.9, Q4 4.65

Fig. 13 (cont.)

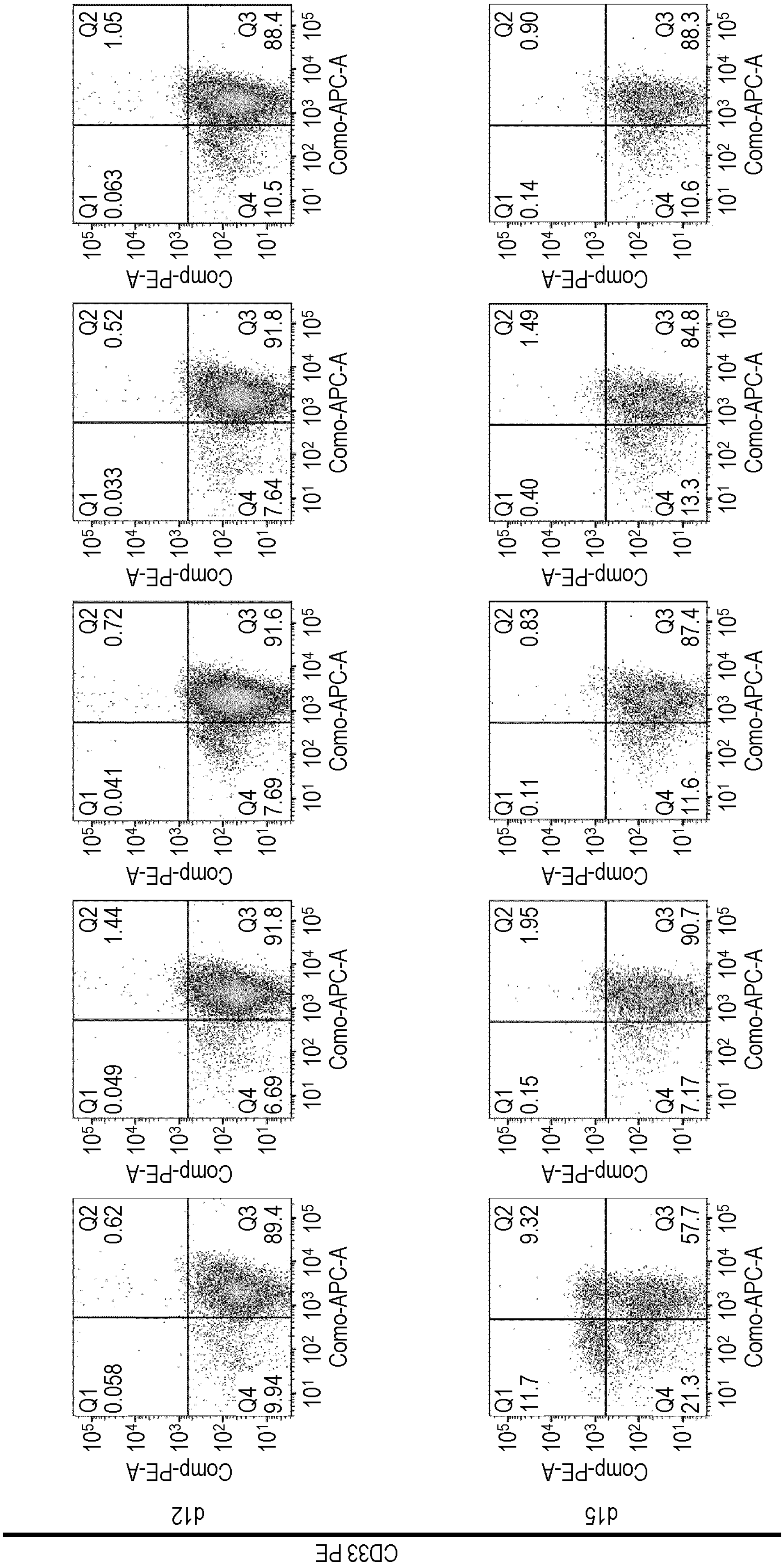
CD33 PE
d12
d15
Comp-PE-A
Como-APC-A
Q1 0.058
Q2 0.62
Q3 89.4
Q4 9.94
Q1 0.049
Q2 1.44
Q3 91.8
Q4 6.69
Q1 0.041
Q2 0.72
Q3 91.6
Q4 7.69
Q1 0.033
Q2 0.52
Q3 91.8
Q4 7.64
Q1 0.063
Q2 1.05
Q3 88.4
Q4 10.5
Q1 11.7
Q2 9.32
Q3 57.7
Q4 21.3
Q1 0.15
Q2 1.95
Q3 90.7
Q4 7.17
Q1 0.11
Q2 0.83
Q3 87.4
Q4 11.6
Q1 0.40
Q2 1.49
Q3 84.8
Q4 13.3
Q1 0.14
Q2 0.90
Q3 88.3
Q4 10.6

Fig. 13 (cont.)

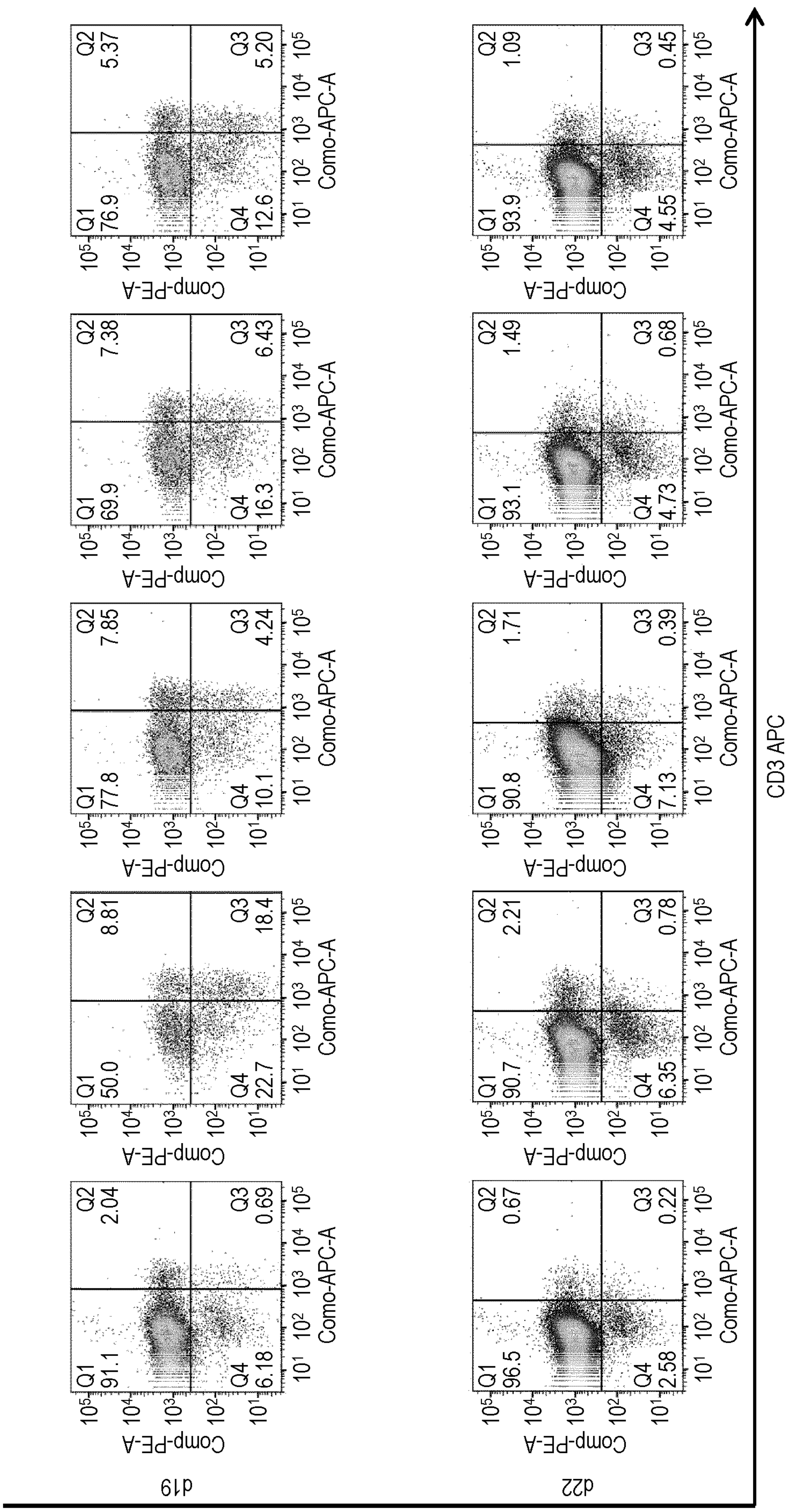
d19
d22
CD3 APC
Comp-PE-A
Como-APC-A
Q1 91.1
Q2 2.04
Q3 0.69
Q4 6.18
Q1 50.0
Q2 8.81
Q3 18.4
Q4 22.7
Q1 77.8
Q2 7.85
Q3 4.24
Q4 10.1
Q1 69.9
Q2 7.38
Q3 6.43
Q4 16.3
Q1 76.9
Q2 5.37
Q3 5.20
Q4 12.6
Q1 96.5
Q2 0.67
Q3 0.22
Q4 2.58
Q1 90.7
Q2 2.21
Q3 0.78
Q4 6.35
Q1 90.8
Q2 1.71
Q3 0.39
Q4 7.13
Q1 93.1
Q2 1.49
Q3 0.68
Q4 4.73
Q1 93.9
Q2 1.09
Q3 0.45
Q4 4.55

Fig. 14

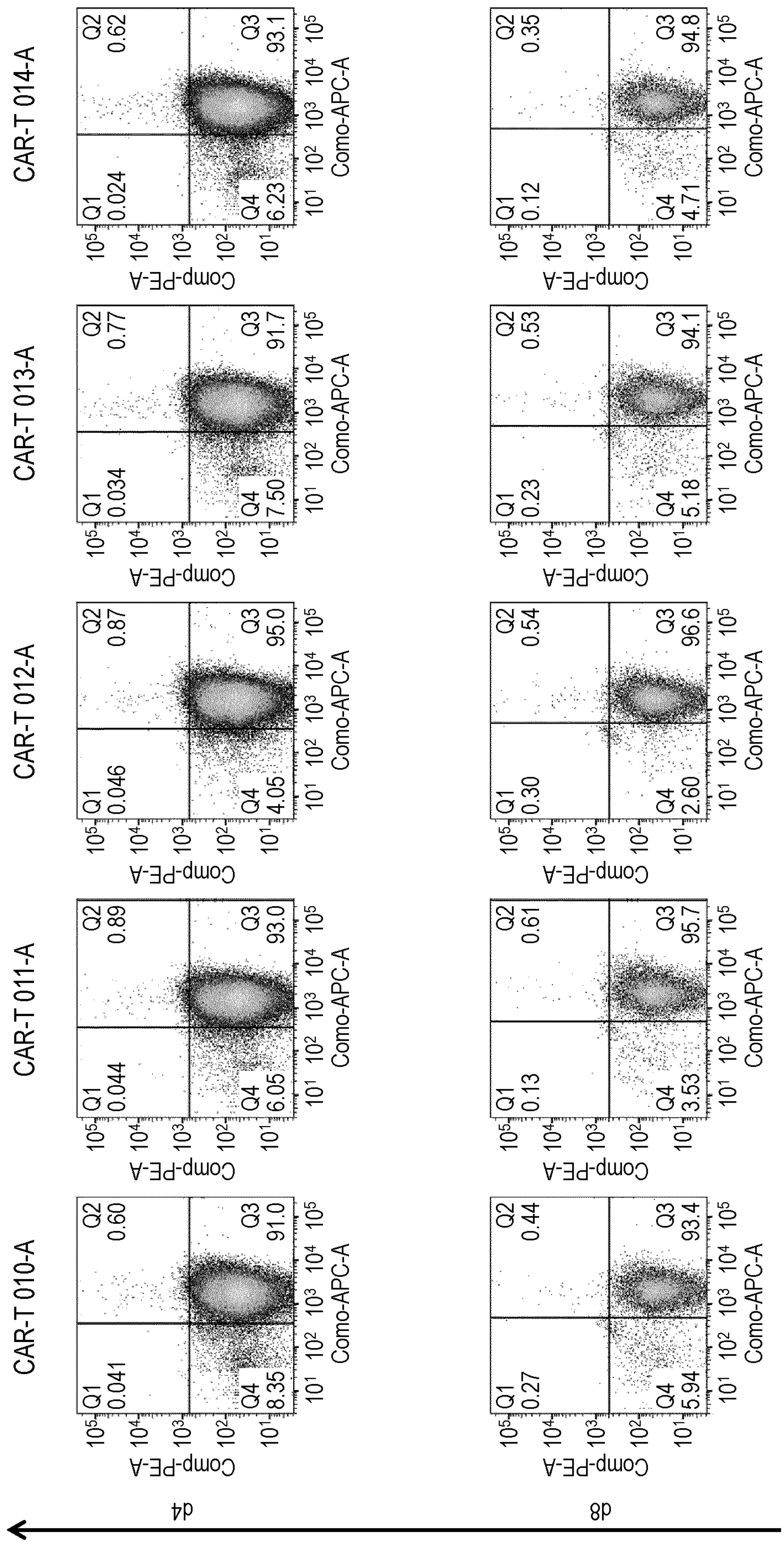
d4
d8
CAR-T 010-A
CAR-T 011-A
CAR-T 012-A
CAR-T 013-A
CAR-T 014-A
Comp-PE-A
Como-APC-A
Q1 0.041
Q2 0.60
Q3 91.0
Q4 8.35
Q1 0.044
Q2 0.89
Q3 93.0
Q4 6.05
Q1 0.046
Q2 0.87
Q3 95.0
Q4 4.05
Q1 0.034
Q2 0.77
Q3 91.7
Q4 7.50
Q1 0.024
Q2 0.62
Q3 93.1
Q4 6.23
Q1 0.27
Q2 0.44
Q3 93.4
Q4 5.94
Q1 0.13
Q2 0.61
Q3 95.7
Q4 3.53
Q1 0.30
Q2 0.54
Q3 96.6
Q4 2.60
Q1 0.23
Q2 0.53
Q3 94.1
Q4 5.18
Q1 0.12
Q2 0.35
Q3 94.8
Q4 4.71

Fig. 14 (cont.)

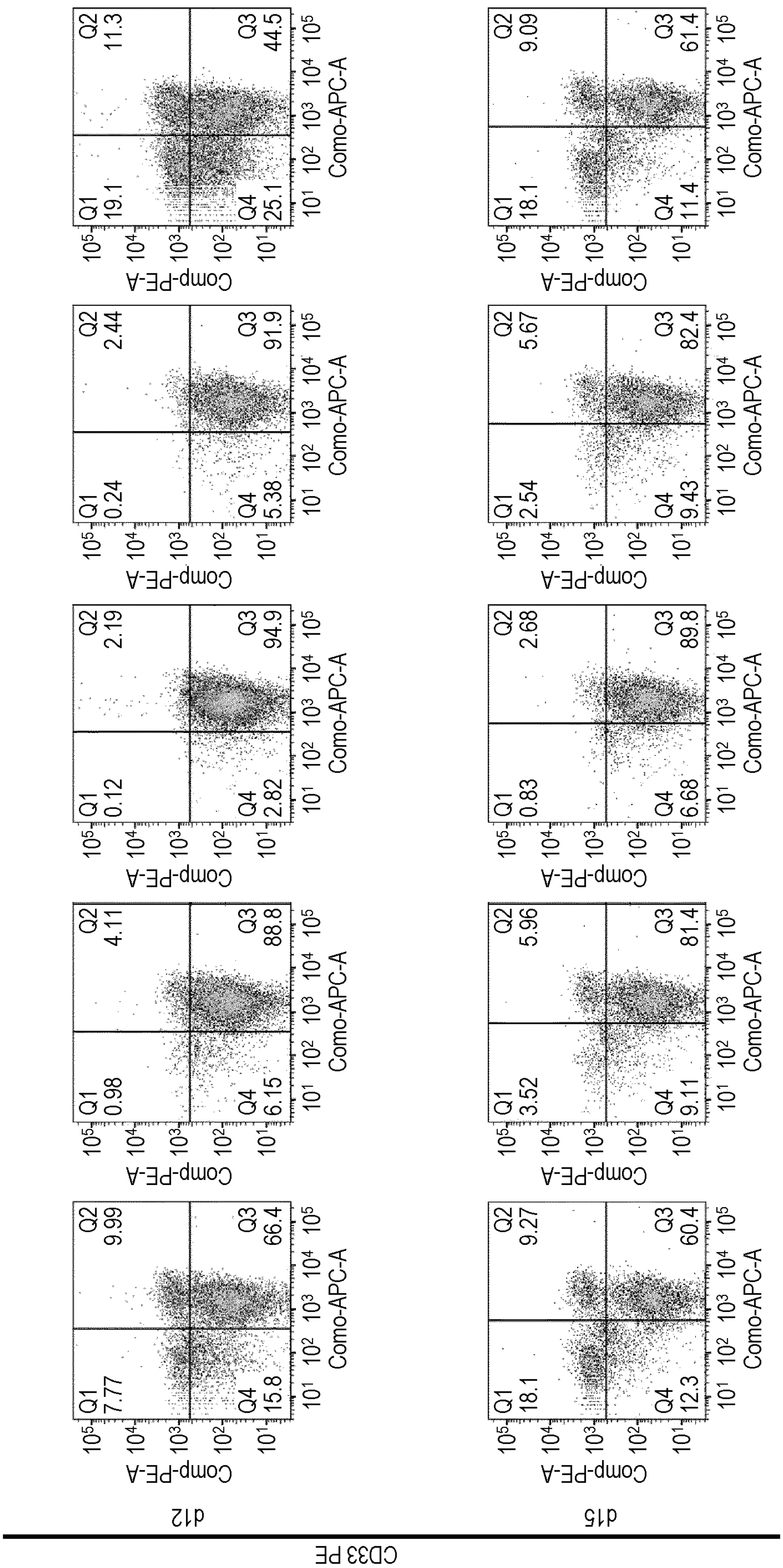
CD33 PE
d12
d15
Comp-PE-A
Como-APC-A
Q1 7.77 Q2 9.99 Q3 66.4 Q4 15.8
Q1 0.98 Q2 4.11 Q3 88.8 Q4 6.15
Q1 0.12 Q2 2.19 Q3 94.9 Q4 2.82
Q1 0.24 Q2 2.44 Q3 91.9 Q4 5.38
Q1 19.1 Q2 11.3 Q3 44.5 Q4 25.1
Q1 18.1 Q2 9.27 Q3 60.4 Q4 12.3
Q1 3.52 Q2 5.96 Q3 81.4 Q4 9.11
Q1 0.83 Q2 2.68 Q3 89.8 Q4 6.68
Q1 2.54 Q2 5.67 Q3 82.4 Q4 9.43
Q1 18.1 Q2 9.09 Q3 61.4 Q4 11.4

Fig. 14 (cont.)

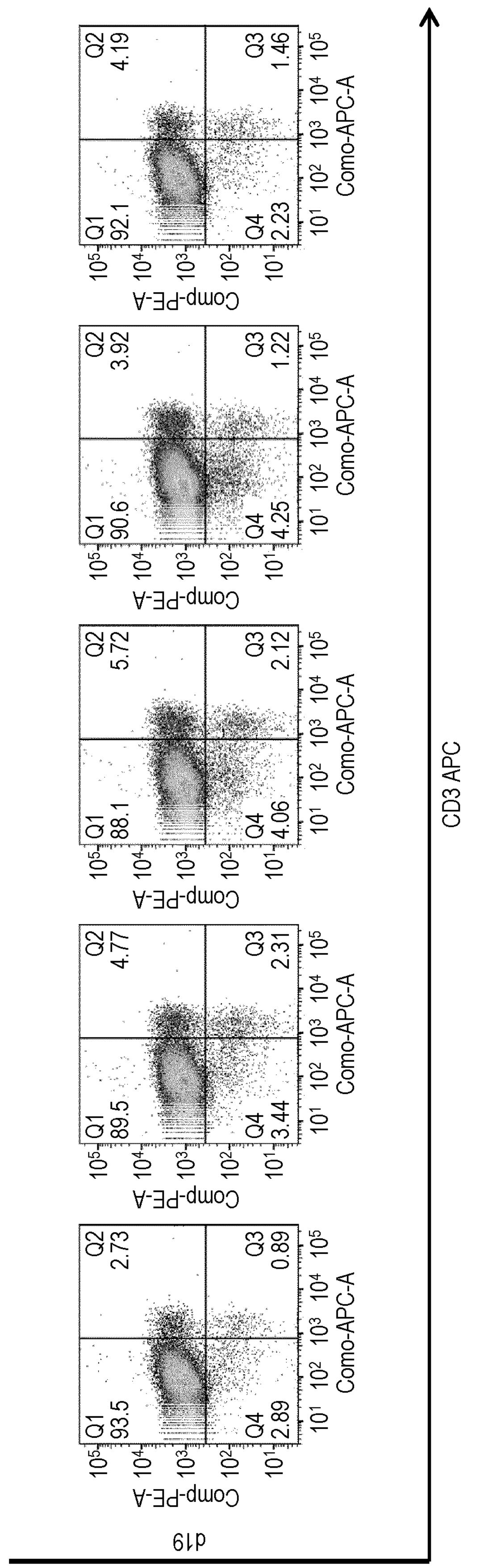

d19
CD3 APC
Comp-PE-A
Como-APC-A
Q1 93.5
Q2 2.73
Q3 0.89
Q4 2.89
Q1 89.5
Q2 4.77
Q3 2.31
Q4 3.44
Q1 88.1
Q2 5.72
Q3 2.12
Q4 4.06
Q1 90.6
Q2 3.92
Q3 1.22
Q4 4.25
Q1 92.1
Q2 4.19
Q3 1.46
Q4 2.23

Fig. 15

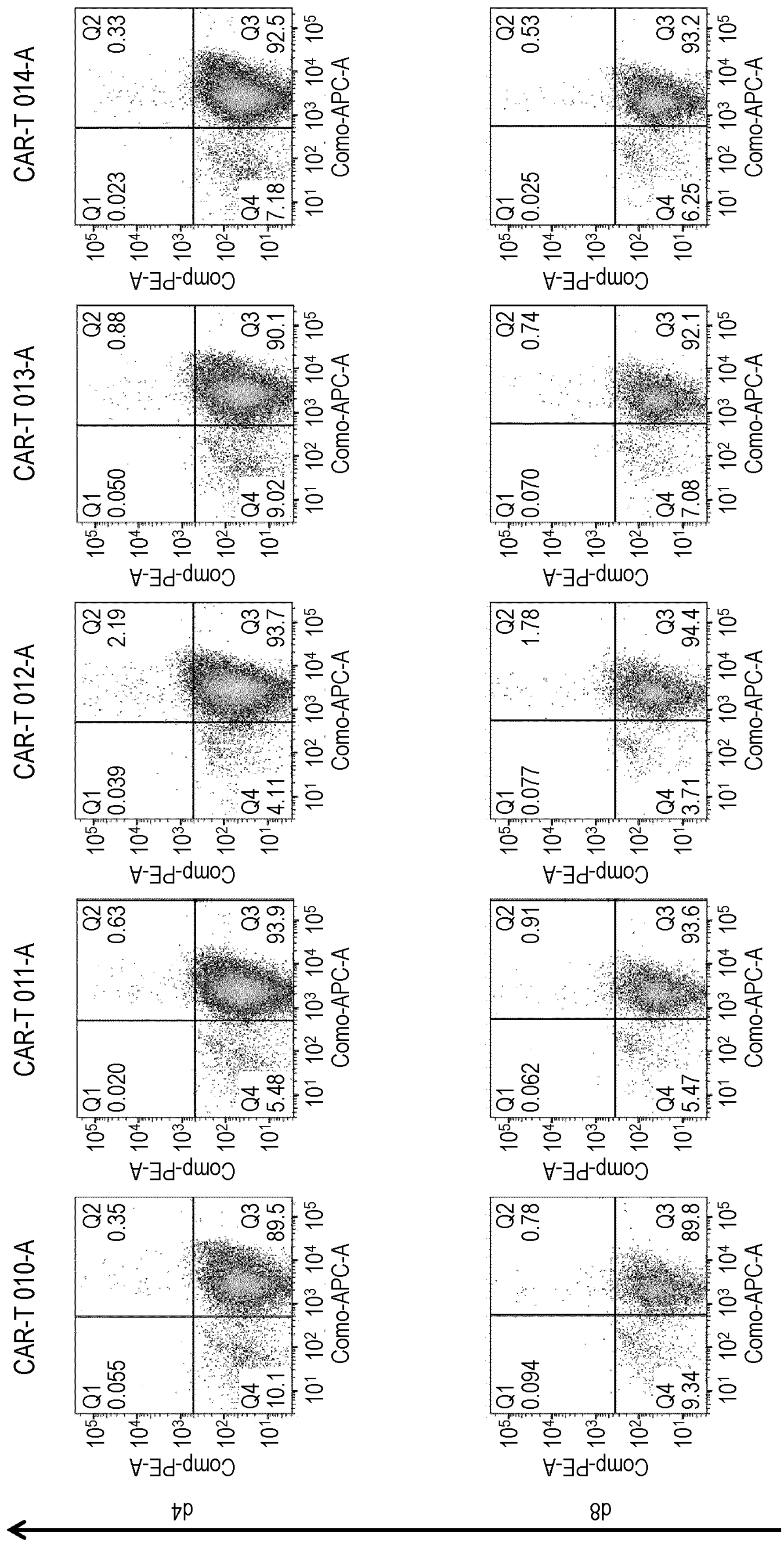
CAR-T 010-A
CAR-T 011-A
CAR-T 012-A
CAR-T 013-A
CAR-T 014-A
d4
d8
Comp-PE-A
Como-APC-A
Q1 0.055
Q2 0.35
Q3 89.5
Q4 10.1
Q1 0.020
Q2 0.63
Q3 93.9
Q4 5.48
Q1 0.039
Q2 2.19
Q3 93.7
Q4 4.11
Q1 0.050
Q2 0.88
Q3 90.1
Q4 9.02
Q1 0.023
Q2 0.33
Q3 92.5
Q4 7.18
Q1 0.094
Q2 0.78
Q3 89.8
Q4 9.34
Q1 0.062
Q2 0.91
Q3 93.6
Q4 5.47
Q1 0.077
Q2 1.78
Q3 94.4
Q4 3.71
Q1 0.070
Q2 0.74
Q3 92.1
Q4 7.08
Q1 0.025
Q2 0.53
Q3 93.2
Q4 6.25

Fig. 15 (cont.)

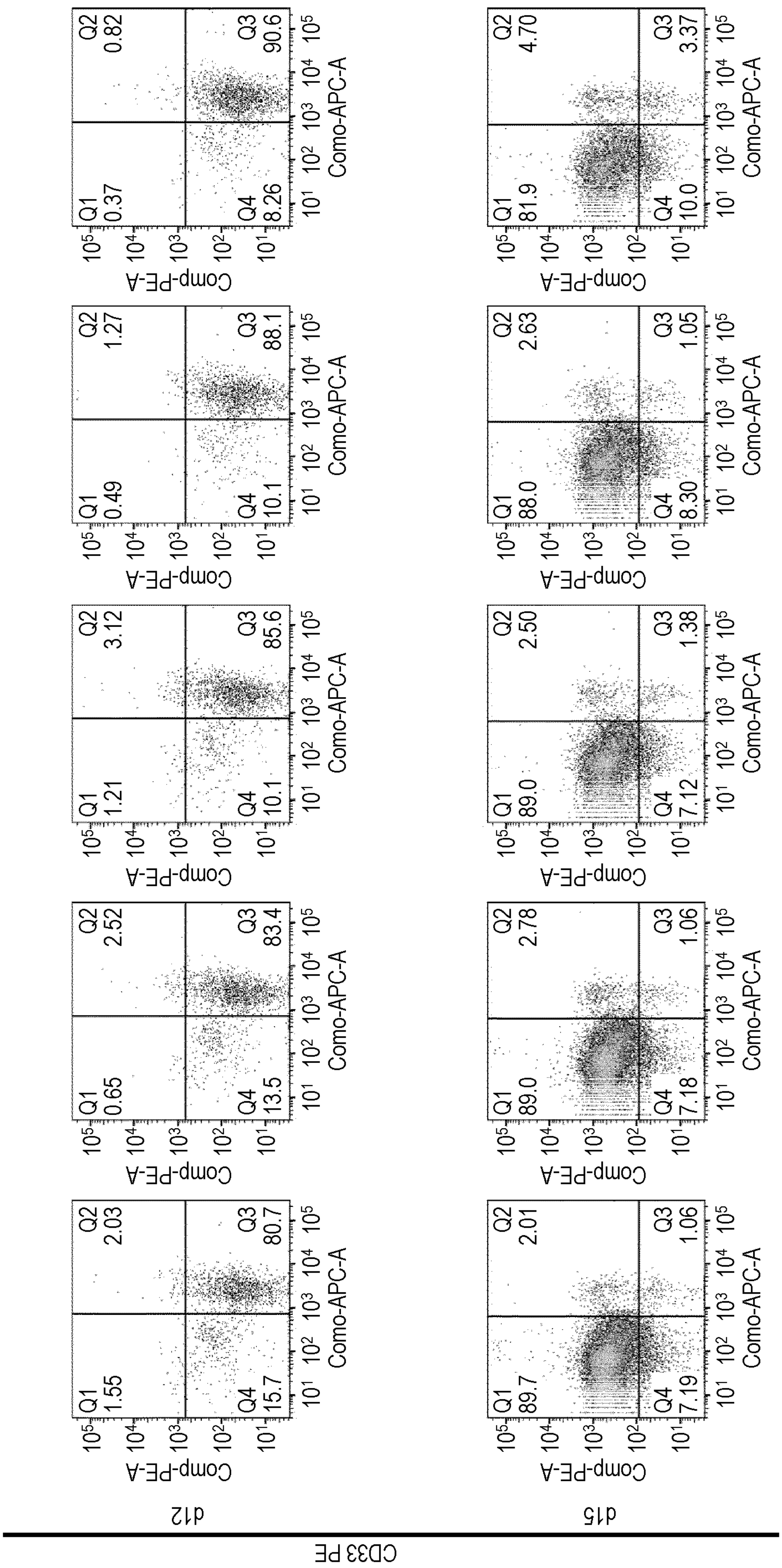
CD33 PE
d12
d15
Comp-PE-A
Como-APC-A
Q1 1.55
Q2 2.03
Q3 80.7
Q4 15.7
Q1 0.65
Q2 2.52
Q3 83.4
Q4 13.5
Q1 1.21
Q2 3.12
Q3 85.6
Q4 10.1
Q1 0.49
Q2 1.27
Q3 88.1
Q4 10.1
Q1 0.37
Q2 0.82
Q3 90.6
Q4 8.26
Q1 89.7
Q2 2.01
Q3 1.06
Q4 7.19
Q1 89.0
Q2 2.78
Q3 1.06
Q4 7.18
Q1 89.0
Q2 2.50
Q3 1.38
Q4 7.12
Q1 88.0
Q2 2.63
Q3 1.05
Q4 8.30
Q1 81.9
Q2 4.70
Q3 3.37
Q4 10.0

Fig. 15 (cont.)

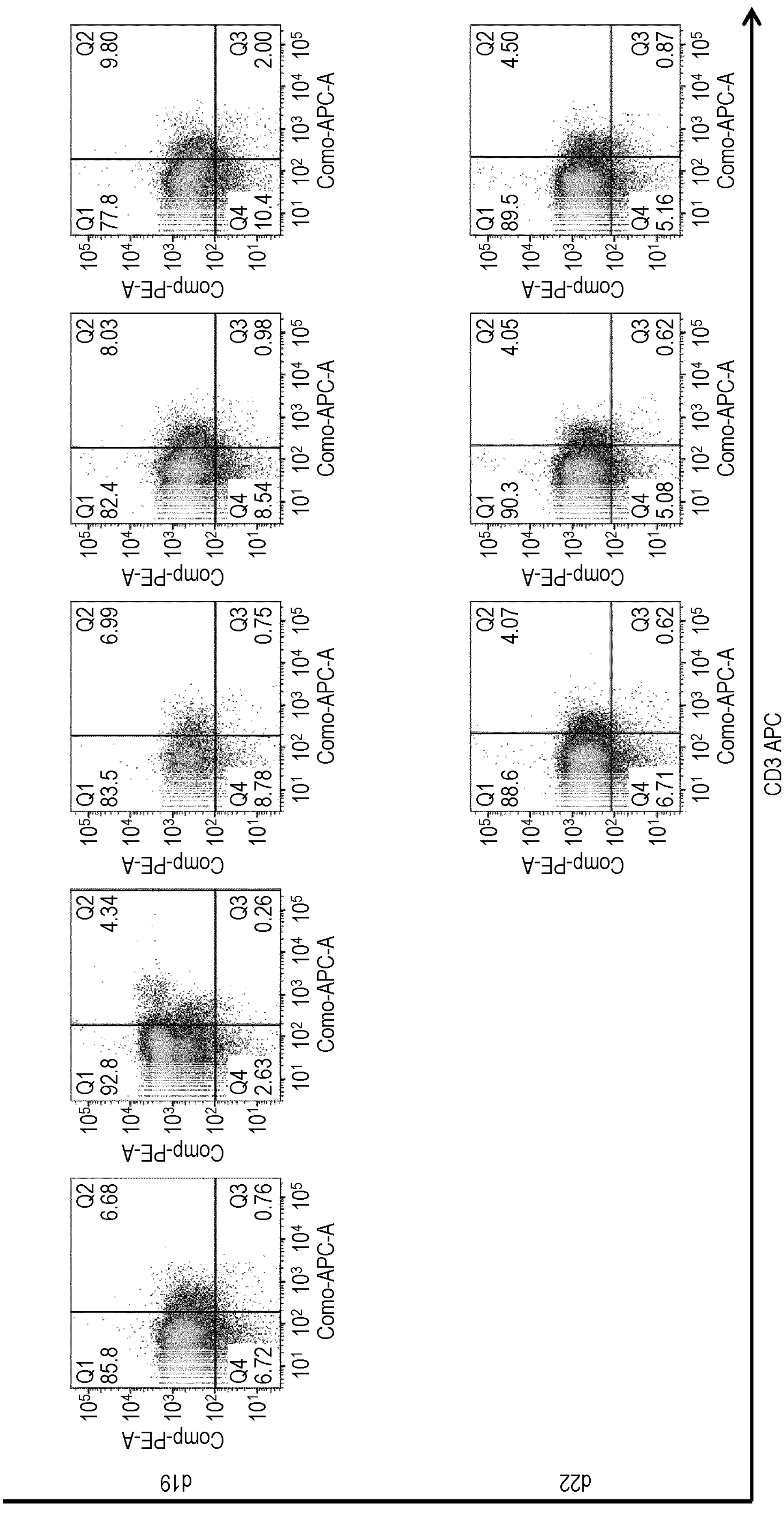
d19
d22
CD3 APC
Comp-PE-A
Como-APC-A
Q1 85.8
Q2 6.68
Q3 0.76
Q4 6.72
Q1 92.8
Q2 4.34
Q3 0.26
Q4 2.63
Q1 83.5
Q2 6.99
Q3 0.75
Q4 8.78
Q1 82.4
Q2 8.03
Q3 0.98
Q4 8.54
Q1 77.8
Q2 9.80
Q3 2.00
Q4 10.4
Q1 88.6
Q2 4.07
Q3 0.62
Q4 6.71
Q1 90.3
Q2 4.05
Q3 0.62
Q4 5.08
Q1 89.5
Q2 4.50
Q3 0.87
Q4 5.16

Fig. 16

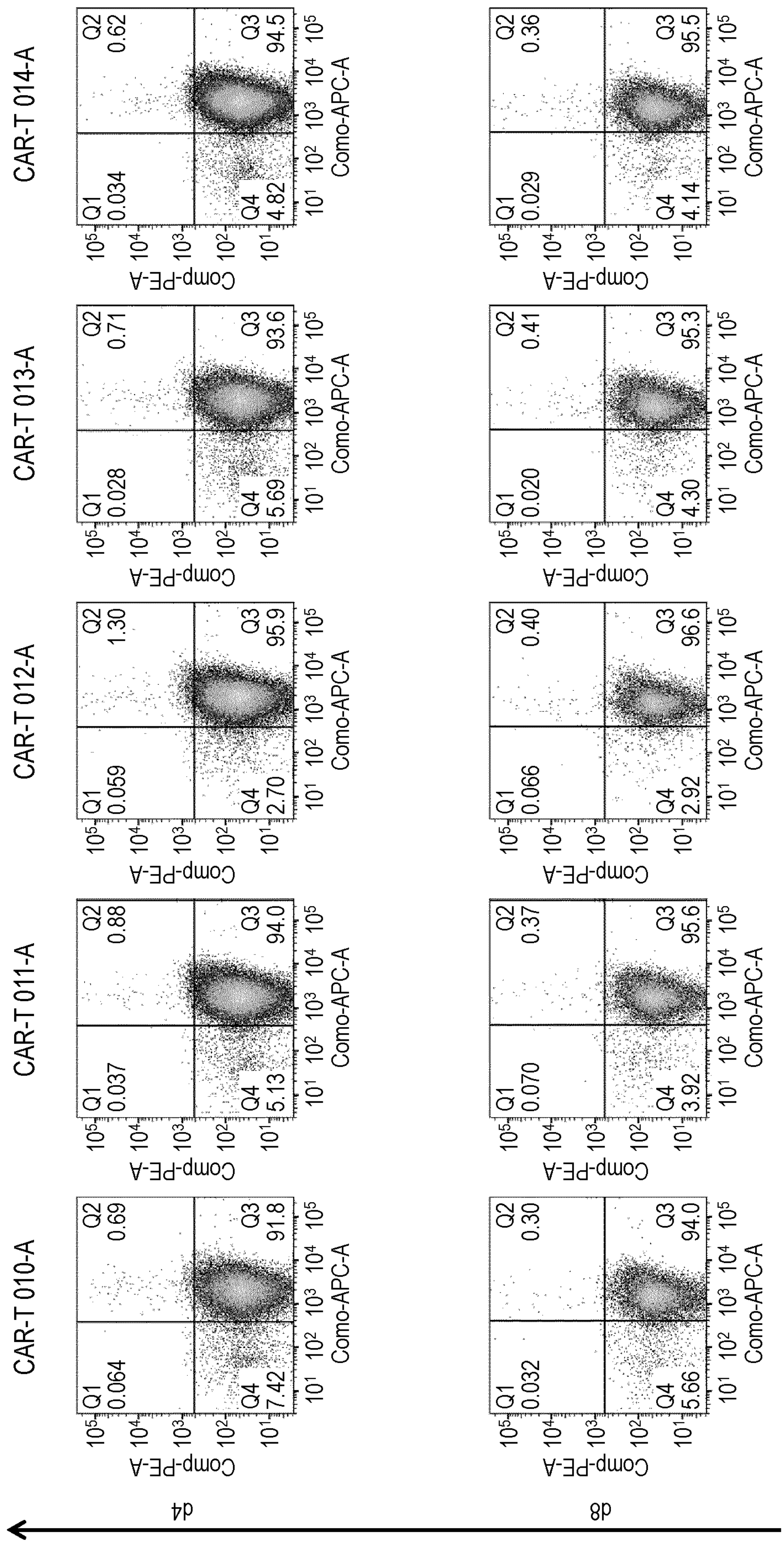
CAR-T 010-A
CAR-T 011-A
CAR-T 012-A
CAR-T 013-A
CAR-T 014-A
d4
d8
Comp-PE-A
Como-APC-A
Q1 0.064
Q2 0.69
Q3 91.8
Q4 7.42
Q1 0.037
Q2 0.88
Q3 94.0
Q4 5.13
Q1 0.059
Q2 1.30
Q3 95.9
Q4 2.70
Q1 0.028
Q2 0.71
Q3 93.6
Q4 5.69
Q1 0.034
Q2 0.62
Q3 94.5
Q4 4.82
Q1 0.032
Q2 0.30
Q3 94.0
Q4 5.66
Q1 0.070
Q2 0.37
Q3 95.6
Q4 3.92
Q1 0.066
Q2 0.40
Q3 96.6
Q4 2.92
Q1 0.020
Q2 0.41
Q3 95.3
Q4 4.30
Q1 0.029
Q2 0.36
Q3 95.5
Q4 4.14

Fig. 16 (cont.)

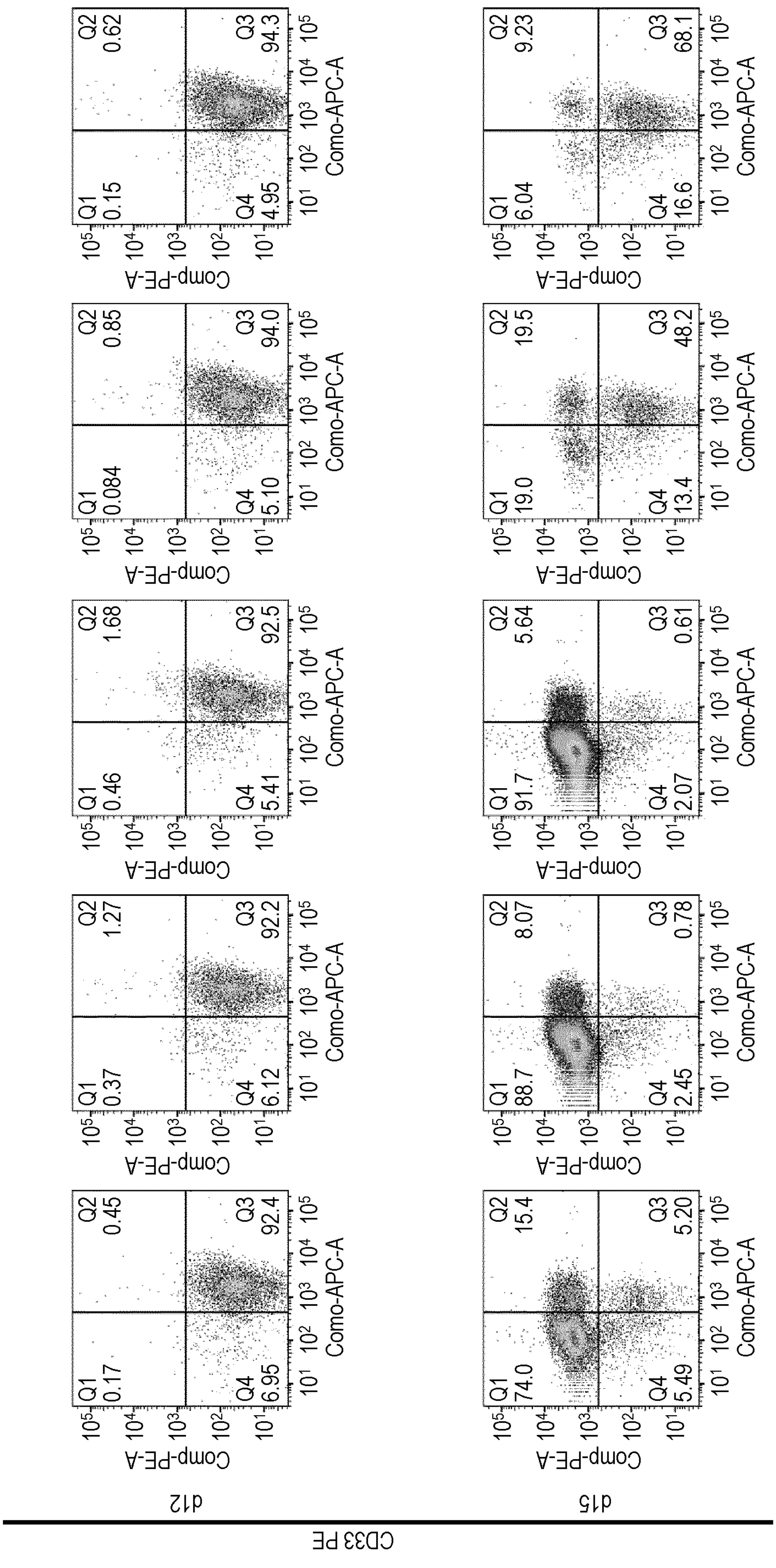
CD33 PE
d12
d15
Comp-PE-A
Como-APC-A
Q1 0.17
Q2 0.45
Q3 92.4
Q4 6.95
Q1 0.37
Q2 1.27
Q3 92.2
Q4 6.12
Q1 0.46
Q2 1.68
Q3 92.5
Q4 5.41
Q1 0.084
Q2 0.85
Q3 94.0
Q4 5.10
Q1 0.15
Q2 0.62
Q3 94.3
Q4 4.95
Q1 74.0
Q2 15.4
Q3 5.20
Q4 5.49
Q1 88.7
Q2 8.07
Q3 0.78
Q4 2.45
Q1 91.7
Q2 5.64
Q3 0.61
Q4 2.07
Q1 19.0
Q2 19.5
Q3 48.2
Q4 13.4
Q1 6.04
Q2 9.23
Q3 68.1
Q4 16.6

Fig. 16 (cont.)

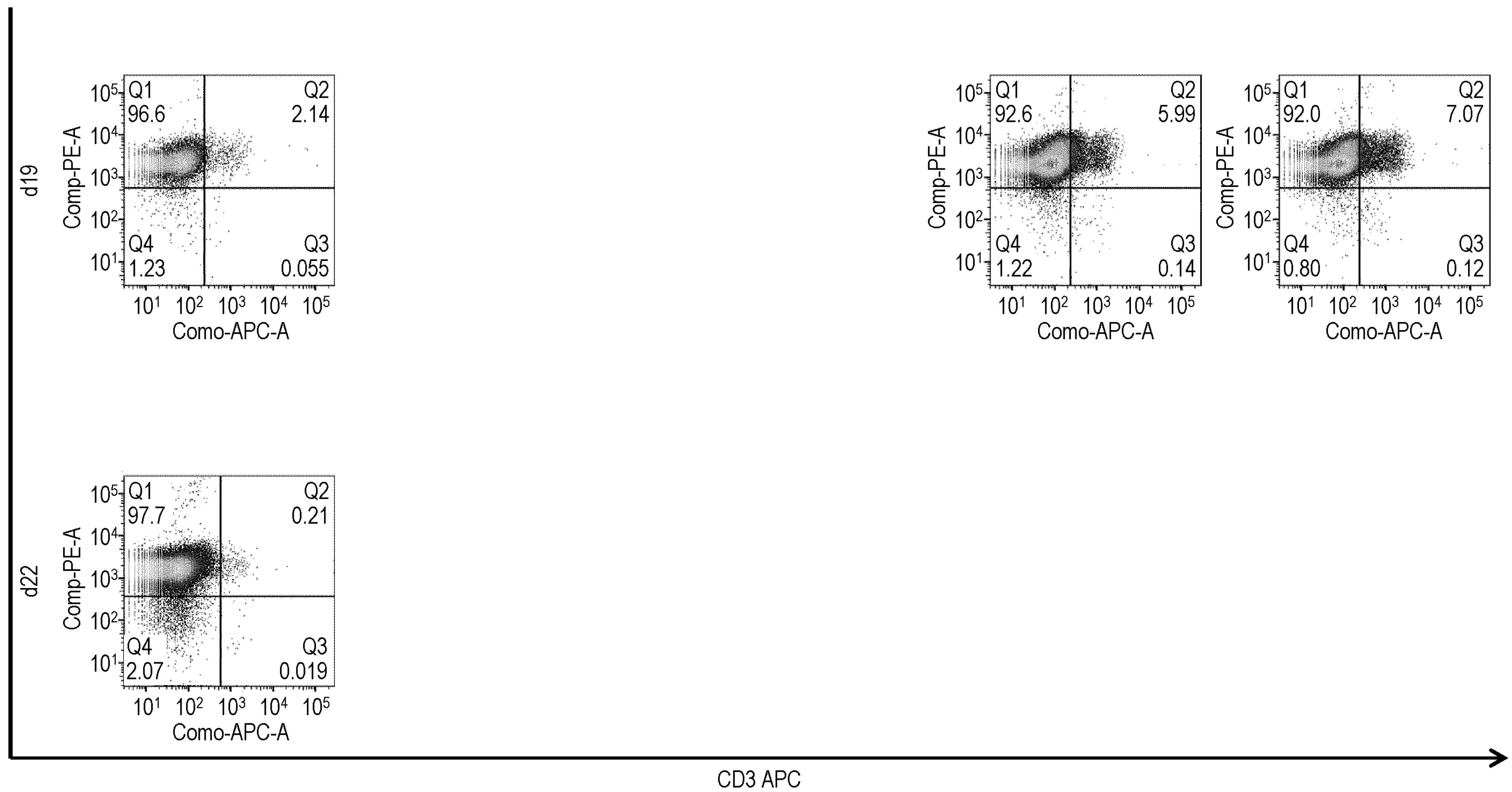
d19
d22
Comp-PE-A
Como-APC-A
Q1
96.6
Q2
2.14
Q3
0.055
Q4
1.23
Q1
97.7
Q2
0.21
Q3
0.019
Q4
2.07
Q1
92.6
Q2
5.99
Q3
0.14
Q4
1.22
Q1
92.0
Q2
7.07
Q3
0.12
Q4
0.80
CD3 APC

# Fig. 17

| THP-1 | term1 | | | term2 | | | term3 | | | term4 | | | term5 | | | term6 | |
|---|---|---|---|---|---|---|---|---|---|---|---|---|---|---|---|---|---|
| Day | 0 | 4 | | 4 | 8 | | 8 | 12 | | 12 | 15 | | 15 | 19 | | 19 | 22 |
| No effector | 250000 | 1345650 | | 250000 | 1103630 | | 250000 | 1039230 | | 250000 | 982760 | | 250000 | 1090100 | | 250000 | 852460 |
| CAR-T 010-A | 250000 | 1640 | | 250820 | 2850 | | 251425 | 820 | | 250410 | 18660 | | 259330 | 249670 | | 374835 | 698910 |
| CAR-T 011-A | 250000 | 2790 | | 251395 | 3060 | | 251530 | 2420 | | 251210 | 1670 | | 250835 | 28450 | | 264225 | 482070 |
| CAR-T 012-A | 250000 | 3820 | | 251910 | 2300 | | 251150 | 1850 | | 250925 | 840 | | 250420 | 111920 | | 305960 | 649930 |
| CAR-T 013-A | 250000 | 2380 | | 251190 | 3480 | | 251740 | 1020 | | 250510 | 1450 | | 250725 | 64530 | | 282265 | 508120 |
| CAR-T 014-A | 250000 | 4290 | | 252145 | 1350 | | 250675 | 1940 | | 250970 | 840 | | 250420 | 103320 | | 301660 | 589420 |

| MV4-11 | term1 | | | term2 | | | term3 | | | term4 | | | term5 | |
|---|---|---|---|---|---|---|---|---|---|---|---|---|---|---|
| Day | 0 | 4 | | 4 | 8 | | 8 | 12 | | 12 | 15 | | 15 | 19 |
| No effector | 250000 | 1264350 | | 250000 | 702720 | | 250000 | 637010 | | 250000 | 911130 | | 250000 | 900690 |
| CAR-T 010-A | 250000 | 3900 | | 251950 | 1340 | | 250670 | 22030 | | 261015 | 26440 | | 263220 | 321520 |
| CAR-T 011-A | 250000 | 5940 | | 252970 | 1380 | | 250690 | 6080 | | 253040 | 8830 | | 254415 | 312460 |
| CAR-T 012-A | 250000 | 6330 | | 253165 | 2050 | | 251025 | 3490 | | 251745 | 3260 | | 251630 | 367020 |
| CAR-T 013-A | 250000 | 4780 | | 252390 | 1450 | | 250725 | 2340 | | 251170 | 6630 | | 253315 | 485380 |
| CAR-T 014-A | 250000 | 5500 | | 252750 | 810 | | 250405 | 56490 | | 278245 | 26140 | | 263070 | 281690 |

| Kasumi-1 | term1 | | | term2 | | | term3 | | | term4 | | | term5 | | | term6 | |
|---|---|---|---|---|---|---|---|---|---|---|---|---|---|---|---|---|---|
| Day | 0 | 4 | | 4 | 8 | | 8 | 12 | | 12 | 15 | | 15 | 19 | | 19 | 22 |
| No effector | 250000 | 463440 | | 250000 | 422670 | | 250000 | 395600 | | 250000 | 349860 | | 250000 | 467130 | | 250000 | 443720 |
| CAR-T 010-A | 250000 | 960 | | 250480 | 830 | | 250415 | 970 | | 250485 | 193650 | | 346825 | 374300 | | | |
| CAR-T 011-A | 250000 | 2250 | | 251125 | 1250 | | 250625 | 880 | | 250440 | 175410 | | 337705 | 507240 | | | |
| CAR-T 012-A | 250000 | 6940 | | 253470 | 2170 | | 251085 | 1250 | | 250625 | 137370 | | 318685 | 98790 | | 299395 | 476690 |
| CAR-T 013-A | 250000 | 2580 | | 251290 | 920 | | 250460 | 470 | | 250235 | 122040 | | 311020 | 301750 | | 400875 | 427070 |
| CAR-T 014-A | 250000 | 1240 | | 250620 | 870 | | 250435 | 420 | | 250210 | 81520 | | 290760 | 238280 | | 369140 | 427700 |

| shinAML-1 | term1 | | | term2 | | | term3 | | | term4 | | | term5 | | | term6 | |
|---|---|---|---|---|---|---|---|---|---|---|---|---|---|---|---|---|---|
| Day | 0 | 4 | | 4 | 8 | | 8 | 12 | | 12 | 15 | | 15 | 19 | | 19 | 22 |
| No effector | 250000 | 1100600 | | 250000 | 1082550 | | 250000 | 1355330 | | 250000 | 1451470 | | 250000 | 1339470 | | 250000 | 1159230 |
| CAR-T 010-A | 250000 | 2840 | | 251420 | 730 | | 250365 | 540 | | 250270 | 150920 | | 325460 | 270650 | | 385325 | 968690 |
| CAR-T 011-A | 250000 | 4490 | | 252245 | 890 | | 250445 | 1120 | | 250560 | 369220 | | | | | | |
| CAR-T 012-A | 250000 | 6700 | | 253350 | 840 | | 250420 | 1450 | | 250725 | 539190 | | | | | | |
| CAR-T 013-A | 250000 | 3210 | | 251605 | 1060 | | 250530 | 780 | | 250390 | 16930 | | 258465 | 613270 | | | |
| CAR-T 014-A | 250000 | 4080 | | 252040 | 1080 | | 250540 | 830 | | 250415 | 6170 | | 253085 | 539910 | | | |

Fig. 18

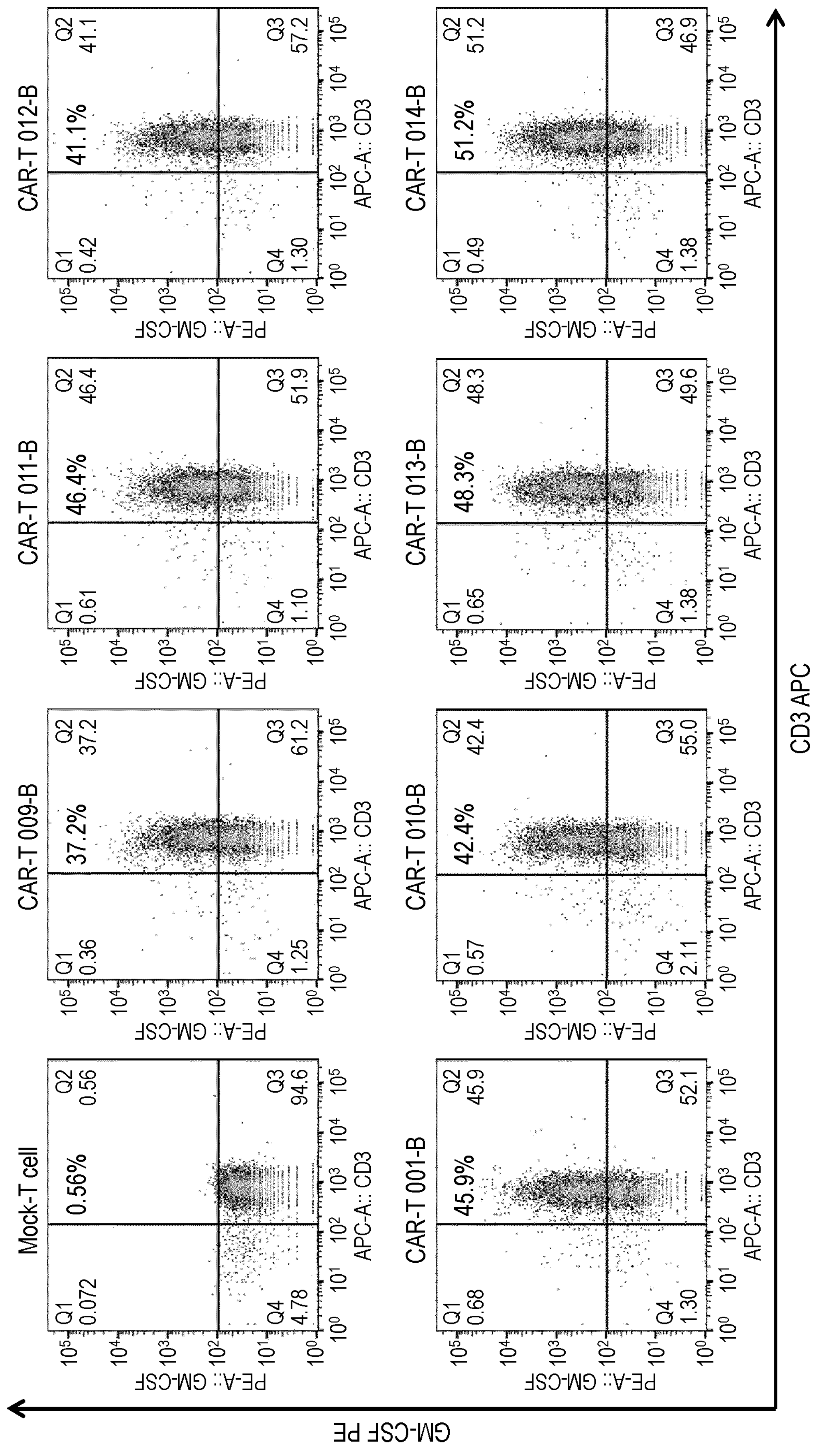

Mock-T cell
0.56%
Q1 0.072
Q2 0.56
Q3 94.6
Q4 4.78
CAR-T 009-B
37.2%
Q1 0.36
Q2 37.2
Q3 61.2
Q4 1.25
CAR-T 011-B
46.4%
Q1 0.61
Q2 46.4
Q3 51.9
Q4 1.10
CAR-T 012-B
41.1%
Q1 0.42
Q2 41.1
Q3 57.2
Q4 1.30
CAR-T 001-B
45.9%
Q1 0.68
Q2 45.9
Q3 52.1
Q4 1.30
CAR-T 010-B
42.4%
Q1 0.57
Q2 42.4
Q3 55.0
Q4 2.11
CAR-T 013-B
48.3%
Q1 0.65
Q2 48.3
Q3 49.6
Q4 1.38
CAR-T 014-B
51.2%
Q1 0.49
Q2 51.2
Q3 46.9
Q4 1.38
PE-A:: GM-CSF
APC-A:: CD3
CD3 APC
GM-CSF PE

Fig. 19

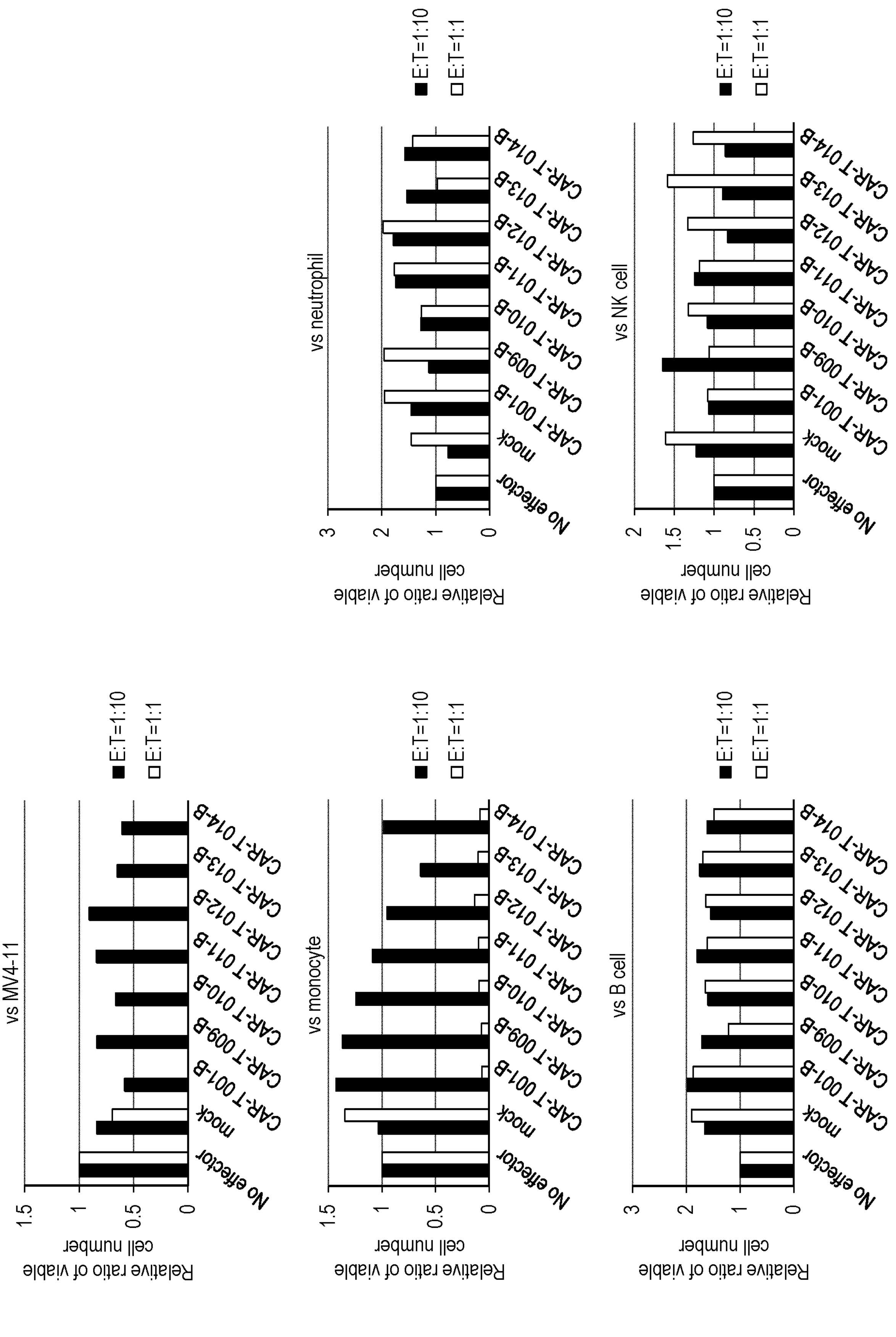
vs MV4-11
vs monocyte
vs B cell
vs neutrophil
vs NK cell
Relative ratio of viable cell number
■ E:T=1:10
□ E:T=1:1
No effector
mock
CAR-T 001-B
CAR-T 009-B
CAR-T 010-B
CAR-T 011-B
CAR-T 012-B
CAR-T 013-B
CAR-T 014-B

Fig. 20

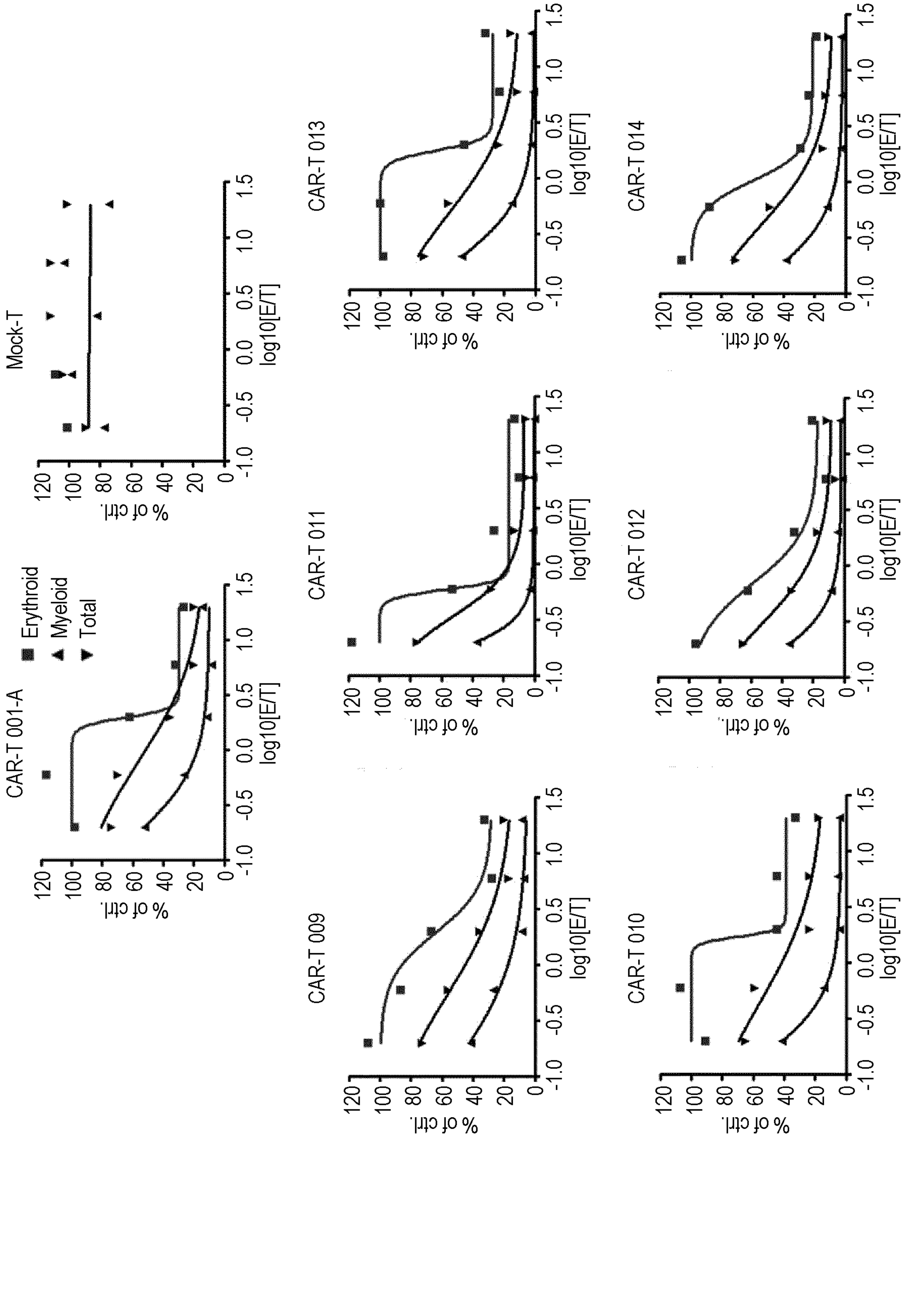

CAR-T 001-A
Erythroid
Myeloid
Total
Mock-T
CAR-T 009
CAR-T 011
CAR-T 013
CAR-T 010
CAR-T 012
CAR-T 014
% of ctrl.
log10[E/T]

Fig. 21

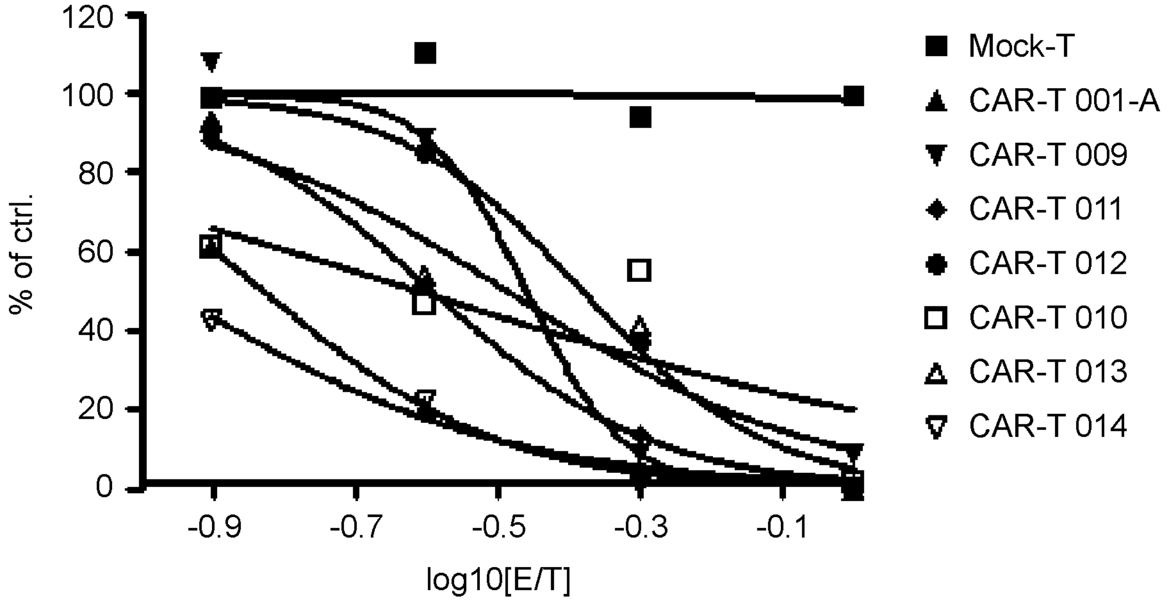


Fig. 22

| name | IC50 | | | EC50 |
|---|---|---|---|---|
| | Erythroid | Myeloid | Total | |
| CAR-T 001-A | 1.97 | 0.18 | 0.80 | 0.15 |
| CAR-T 009 | 1.96 | 0.12 | 0.54 | 0.35 |
| CAR-T 011 | 0.59 | 0.17 | 0.34 | 0.26 |
| CAR-T 012 | 1.77 | 0.18 | 0.50 | 0.42 |
| CAR-T 010 | 1.71 | 0.15 | 0.42 | 0.24 |
| CAR-T 013 | 0.70 | 0.14 | 0.29 | 0.33 |
| CAR-T 014 | 1.01 | 0.14 | 0.42 | 0.11 |

| Margin of safety (=LC50/EC50) | | |
|---|---|---|
| Erythroid | Myeloid | Total |
| 13.31 | 1.24 | 5.42 |
| 5.65 | 0.35 | 1.55 |
| 2.29 | 0.65 | 1.31 |
| 4.25 | 0.44 | 1.20 |
| 7.03 | 0.61 | 1.71 |
| 2.16 | 0.42 | 0.90 |
| 9.33 | 1.29 | 3.86 |

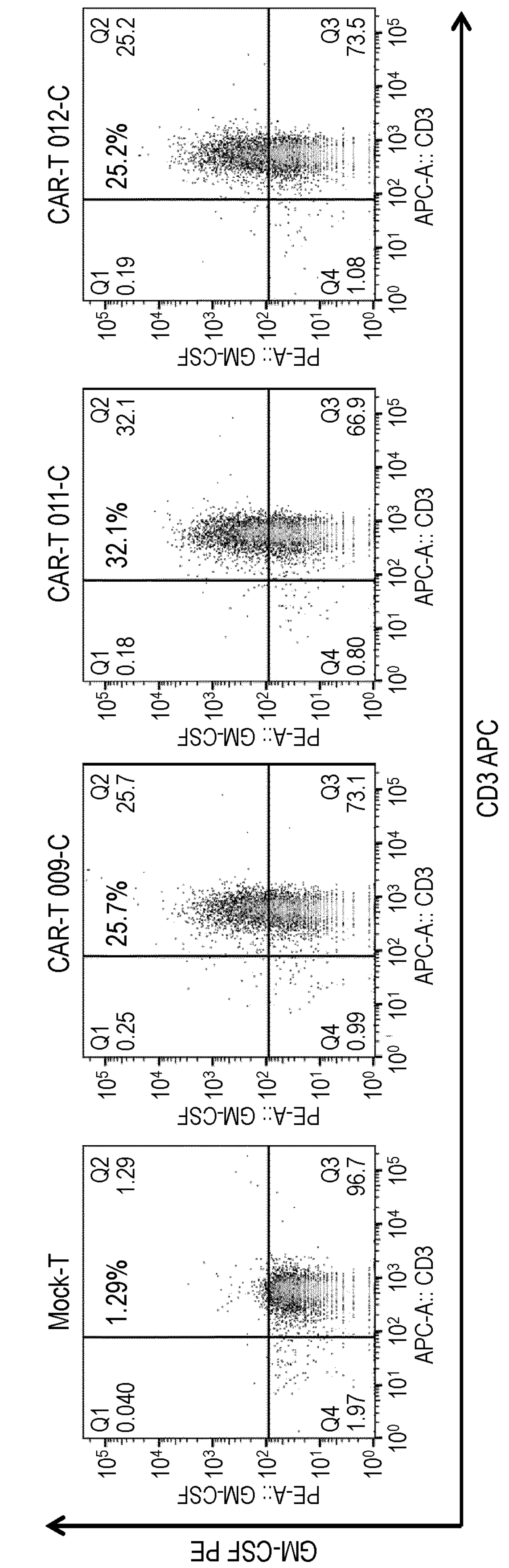

Mock-T
1.29%
Q1 0.040
Q2 1.29
Q3 96.7
Q4 1.97
CAR-T 009-C
25.7%
Q1 0.25
Q2 25.7
Q3 73.1
Q4 0.99
CAR-T 011-C
32.1%
Q1 0.18
Q2 32.1
Q3 66.9
Q4 0.80
CAR-T 012-C
25.2%
Q1 0.19
Q2 25.2
Q3 73.5
Q4 1.08
PE-A:: GM-CSF
APC-A:: CD3
CD3 APC
GM-CSF PE


Fig. 23

Fig. 24

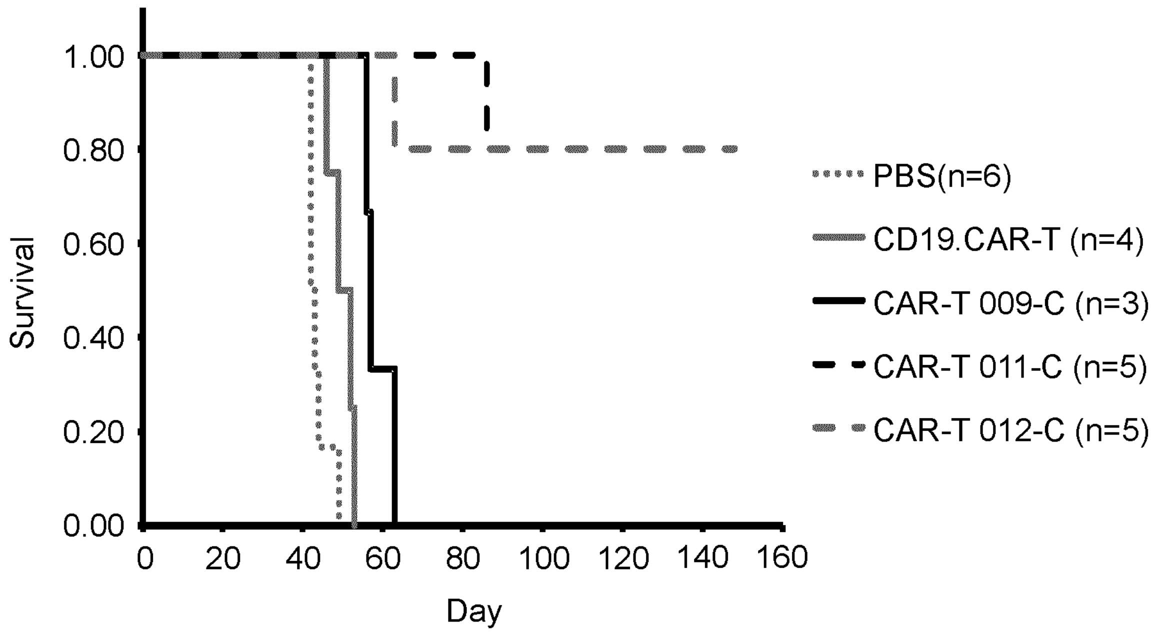


P value

| | CAR-T 009-C | CAR-T 011-C | CAR-T 012-C |
|---|---|---|---|
| CD19 | 0.0086(**) | 0.0011(**) | 0.0011(**) |
| PBS | 0.0177(*) | 0.0027(**) | 0.0027(**) |

P value

| | CAR-T 011-C | CAR-T 012-C |
|---|---|---|
| CAR-T 009-C | 0.0042(**) | 0.0136(*) |

Fig. 25

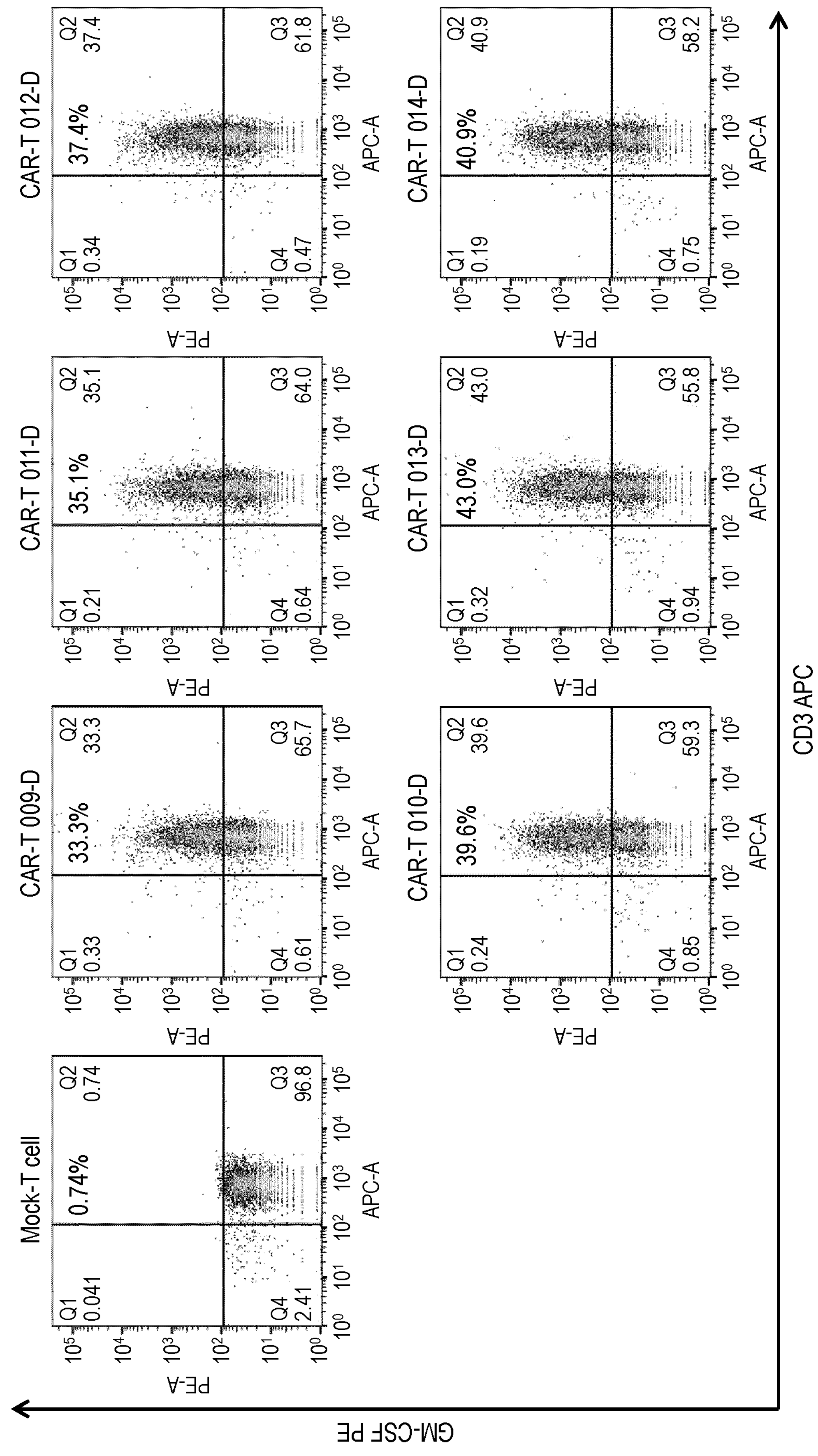
Mock-T cell
0.74%
Q1 0.041
Q2 0.74
Q3 96.8
Q4 2.41
CAR-T 009-D
33.3%
Q1 0.33
Q2 33.3
Q3 65.7
Q4 0.61
CAR-T 011-D
35.1%
Q1 0.21
Q2 35.1
Q3 64.0
Q4 0.64
CAR-T 012-D
37.4%
Q1 0.34
Q2 37.4
Q3 61.8
Q4 0.47
CAR-T 010-D
39.6%
Q1 0.24
Q2 39.6
Q3 59.3
Q4 0.85
CAR-T 013-D
43.0%
Q1 0.32
Q2 43.0
Q3 55.8
Q4 0.94
CAR-T 014-D
40.9%
Q1 0.19
Q2 40.9
Q3 58.2
Q4 0.75
PE-A
APC-A
CD3 APC
GM-CSF PE

Fig. 26

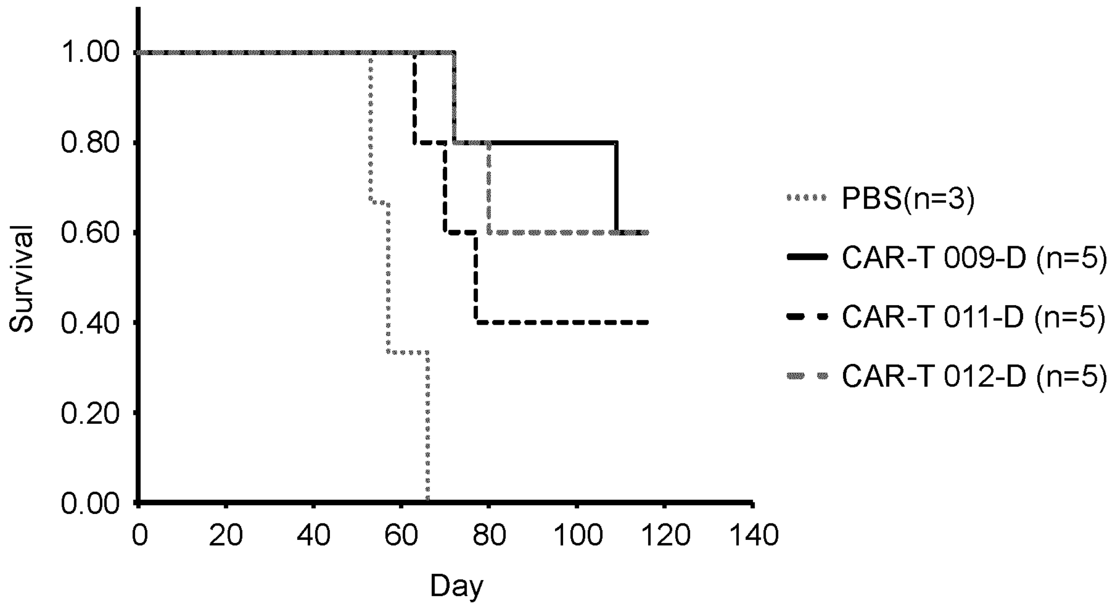


P value

| | CAR-T 009-D | CAR-T 011-D | CAR-T 012-D |
|---|---|---|---|
| PBS | **(0.0042) | *(0.0216) | **(0.0042) |

Fig. 27

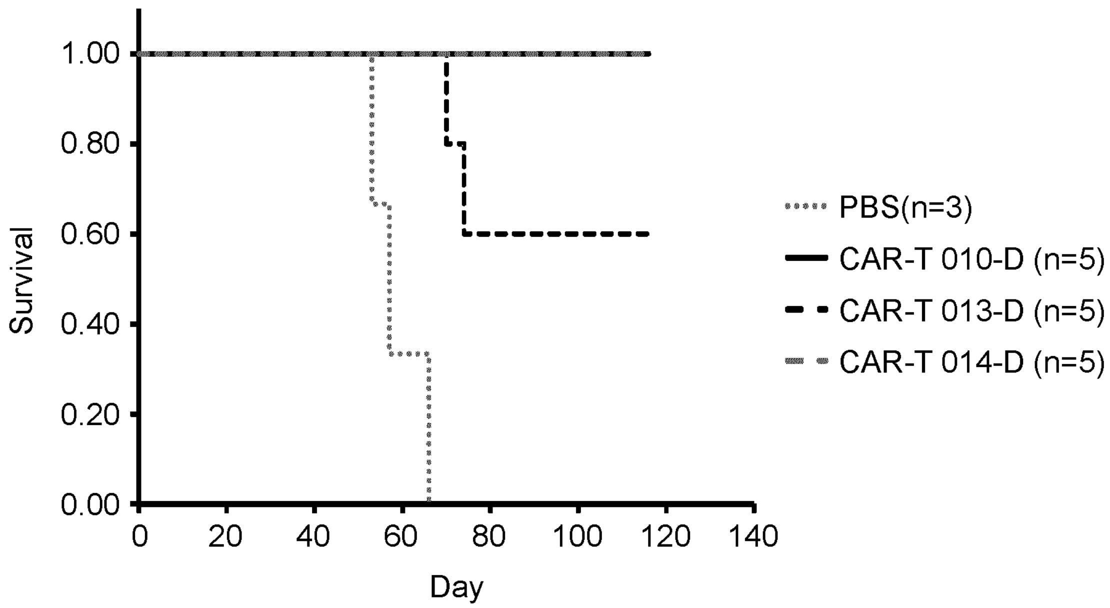


P value

| | CAR-T 010-D | CAR-T 013-D | CAR-T 014-D |
|---|---|---|---|
| PBS | **(0.0042) | **(0.0042) | **(0.0042) |

Fig. 28

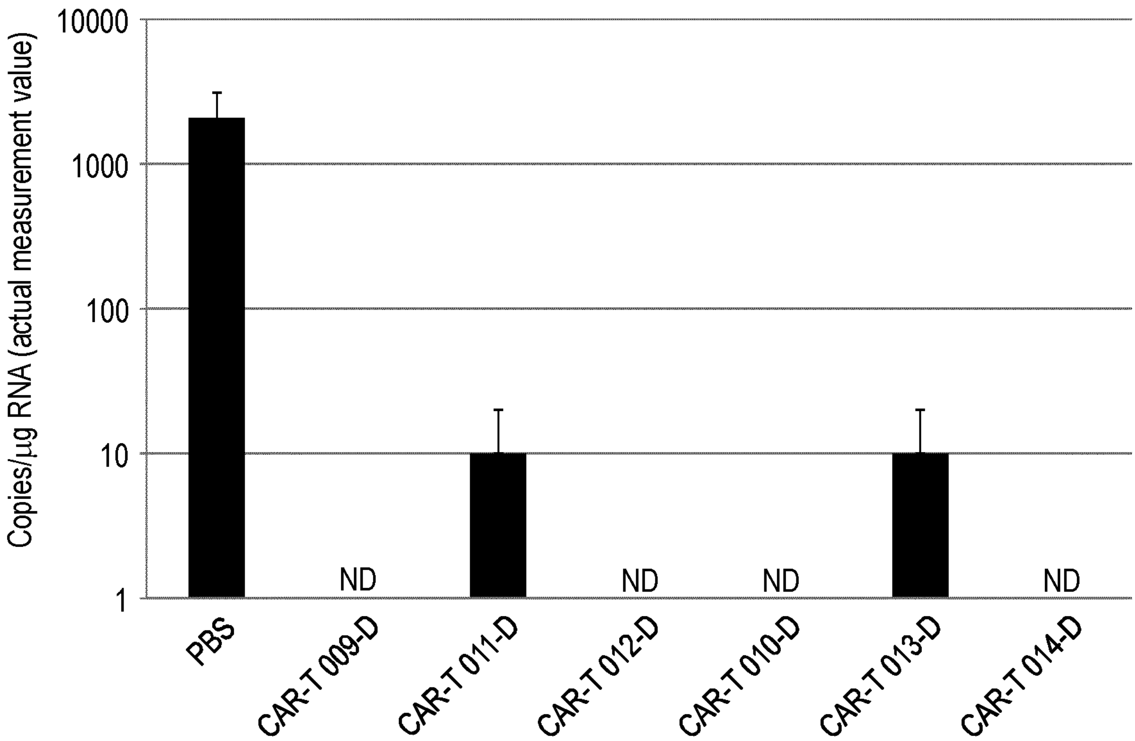

Copies/μg RNA (actual measurement value)
10000
1000
100
10
1
ND
ND
ND
ND
PBS
CAR-T 009-D
CAR-T 011-D
CAR-T 012-D
CAR-T 010-D
CAR-T 013-D
CAR-T 014-D

Fig. 29

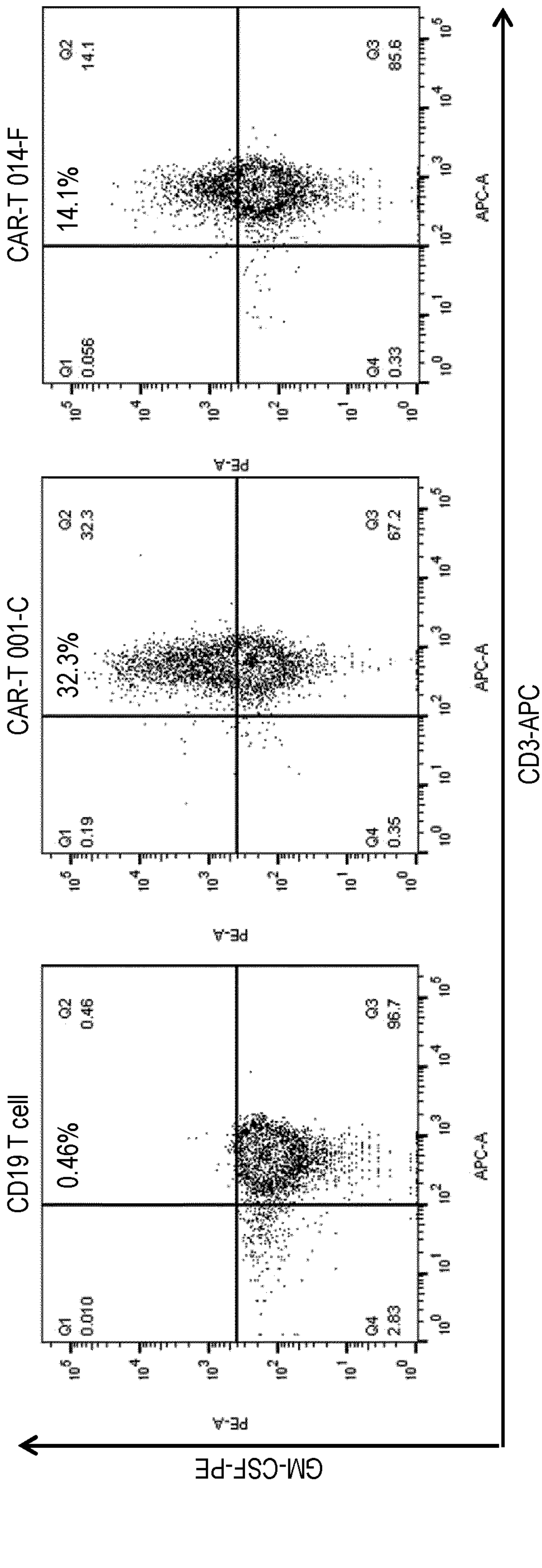
CD19 T cell
0.46%
Q1 0.010
Q2 0.46
Q3 96.7
Q4 2.83
CAR-T 001-C
32.3%
Q1 0.19
Q2 32.3
Q3 67.2
Q4 0.35
CAR-T 014-F
14.1%
Q1 0.056
Q2 14.1
Q3 85.6
Q4 0.33
PE-A
APC-A
CD3-APC
GM-CSF-PE

Fig. 30

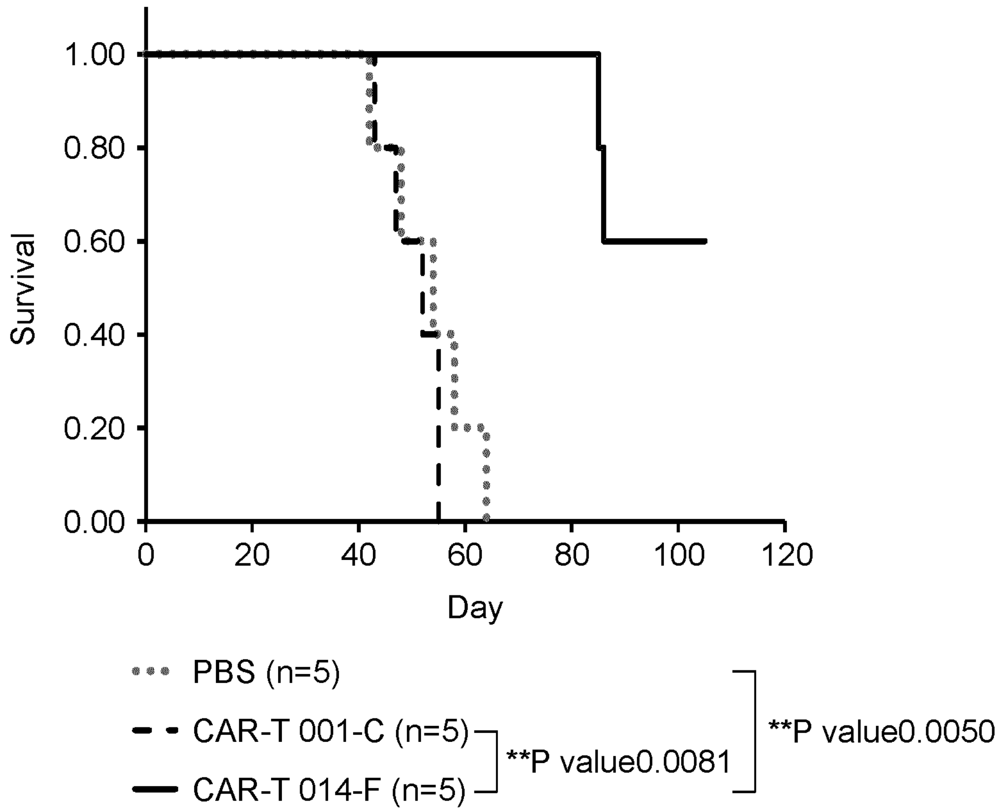
1.00
0.80
0.60
0.40
0.20
0.00
Survival
0
20
40
60
80
100
120
Day
PBS (n=5)
CAR-T 001-C (n=5)
CAR-T 014-F (n=5)
**P value0.0081
**P value0.0050

Fig. 31

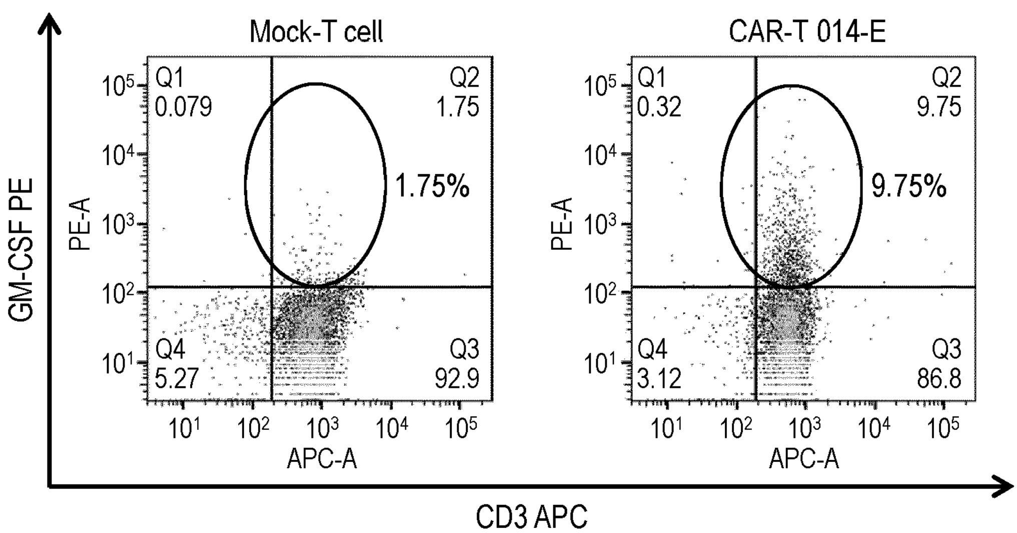
Mock-T cell
CAR-T 014-E
GM-CSF PE
PE-A
Q1
0.079
Q2
1.75
1.75%
Q4
5.27
Q3
92.9
Q1
0.32
Q2
9.75
9.75%
Q4
3.12
Q3
86.8
APC-A
CD3 APC

## INTERNATIONAL SEARCH REPORT

International application No.
PCT/JP2019/010854

**A. CLASSIFICATION OF SUBJECT MATTER**

Int.Cl. C12N15/62(2006.01)i, A61K35/12(2015.01)i, A61P35/02(2006.01)i, C12N5/10(2006.01)i, C12N15/12(2006.01)i, C12N15/13(2006.01)i, C12N15/27(2006.01)i, C12N15/85(2006.01)i

According to International Patent Classification (IPC) or to both national classification and IPC

**B. FIELDS SEARCHED**

Minimum documentation searched (classification system followed by classification symbols)
Int.Cl. C12N15/62, A61K35/12, A61P35/02, C12N5/10, C12N15/12, C12N15/13, C12N15/27, C12N15/85

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CiNii, UniProt/GeneSeq, CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| A | NAKAZAWA, Y. et al., "Anti-proliferative effects of T cells expressing a ligand-based chimeric antigen receptor against CD116 on CD34+ cells of juvenile myelomonocytic leukemia.", Journal of Hematology & Oncology, 27 September 2016, abstract, fig. 1, 3, page 8, section "Conclusions" | 1-12 |
| A | IVERSEN, P. O. et al., "Apoptosis of hemopoietic cells by the human granulocyte-macrophage colony-stimulating factor mutant E21R.", Proc. Natl. Acad. Sci. USA, 1996, vol. 93, pp. 2785-2789, abstract, fig. 1-3 | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 May 2019 (21.05.2019) | 28 May 2019 (28.05.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/010854

C (Continuation). DOCUMENTS CONSIDERED TO BE RELEVANT

| Category* | Citation of document, with indication, where appropriate, of the relevant passages | Relevant to claim No. |
|---|---|---|
| A | ALTMANN, S. W. et al., "Rational Design of a Mouse Granulocyte Macrophage-Colony-stimulating Factor Receptor Antagonist.", J. Biol. Chem., 1995, vol. 270, no. 5, pp. 2233-2240, abstract, page 2233, lines 9-11, page 2235, right column, paragraph [0004], fig. 1, 3, table III | 1-12 |
| A | WO 2015/066262 A1 (TRUSTEES OF DARTMOUTH COLLEGE) 07 May 2015, claims, paragraphs [0044]-[0046] (Family: none) | 1-12 |
| P, A | WO 2018/052142 A1 (KISSEI PHARMACEUTICAL CO., LTD.) 22 March 2018 (Family: none) | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018050266 A


### Non-patent literature cited in the description


- *Journal of Hematology & Oncology,* 2016, vol. 9, 27
- *The 22nd Annual Meeting JSGCT2016: Japan Society of Gene and Cell Therapy Program and Abstracts,* 13 July 2016, 0-70
- *Journal of Hematology & Oncology,* 2017, vol. 10, 151
- *Blood,* 2013, vol. 122 (18), 3138-3148
- *Blood Cancer Journal,* 2016, vol. 6, e458
- *Oncoimmunology,* 2016, vol. 5 (12), e1253656
- **YANG XU et al.** *Blood,* 2014, vol. 123, 3750-3759
- **HUANG X ; GUO H et al.** *Mol Ther.,* 2008, vol. 16, 580-9
- **SINGH H ; MANURI PR et al.** *Cancer Res.,* 2008, vol. 68, 2961-71
- **DENIGER DC ; YU J et al.** *PLoS One,* 2015, vol. 10, e0128151
- **SINGH H ; MOYES JS et al.** *Cancer Gene Ther.,* 2015, vol. 22, 95-100
- **HOU X ; DU Y et al.** *Cancer Biol Ther.,* 2015, vol. 16, 8-16
- **SINGH H ; HULS H et al.** *Immunol Rev.,* 2014, vol. 257, 181-90
- **MAITI SN ; HULS H et al.** *J Immunother.,* 2013, vol. 36, 112-23
- **NAKAZAWA Y ; HUYE LE et al.** *J Immunother.,* 2009, vol. 32, 826-36
- **GALVAN DL ; NAKAZAWA Y et al.** *J Immunother.,* 2009, vol. 32, 837-44
- **NAKAZAWA Y ; HUYE LE et al.** *Mol Ther.,* 2011, vol. 19, 2133-43
- **HUYE LE ; NAKAZAWA Y et al.** *Mol Ther.,* 2011, vol. 19, 2239-48
- **SAHA S ; NAKAZAWA Y et al.** *J Vis Exp.,* 2012, vol. 69, e4235
- **NAKAZAWA Y ; SAHA S et al.** *J Immunother.,* 2013, vol. 36, 3-10
- **SAITO S ; NAKAZAWA Y et al.** *Cytotherapy,* 2014, vol. 16, 1257-69
- **ZOLA H. et al.** *Immunol Cell Biol.,* 1991, vol. 69, 411-412
- **SAVOLDO et al.** *J Clin Invest,* 2011, vol. 121 (5), 1822-1826
- **HUYE et al.** *Mol Ther,* 2011, vol. 19 (12), 2239-2248
- **MORITA et al.** *Molecular Therapy: Methods & Clinical Development,* March 2018, vol. 8

SEQUENCE LISTING

<110> KISSEI PHARMACEUTICAL CO., LTD.
SHINSHU UNIVERSITY

<120> Gene-Engineered Cells And Method For Producing Said Cells

<130> PH-7741-PCT

<150> JP 2018-050266
<151> 2018-03-16

<160> 34

<170> PatentIn version 3.5

<210> 1
<211> 127
<212> PRT
<213> Homo sapiens

<400> 1

```
Ala Pro Ala Arg Ser Pro Ser Pro Ser Thr Gln Pro Trp Glu His Val
1               5                   10                  15

Asn Ala Ile Gln Glu Ala Arg Arg Leu Leu Asn Leu Ser Arg Asp Thr
            20                  25                  30

Ala Ala Glu Met Asn Glu Thr Val Glu Val Ile Ser Glu Met Phe Asp
        35                  40                  45

Leu Gln Glu Pro Thr Cys Leu Gln Thr Arg Leu Glu Leu Tyr Lys Gln
    50                  55                  60

Gly Leu Arg Gly Ser Leu Thr Lys Leu Lys Gly Pro Leu Thr Met Met
65                  70                  75                  80

Ala Ser His Tyr Lys Gln His Cys Pro Pro Thr Pro Glu Thr Ser Cys
                85                  90                  95

Ala Thr Gln Ile Ile Thr Phe Glu Ser Phe Lys Glu Asn Leu Lys Asp
            100                 105                 110

Phe Leu Leu Val Ile Pro Phe Asp Cys Trp Glu Pro Val Gln Glu
        115                 120                 125
```

<210> 2
<211> 127
<212> PRT
<213> Artificial

<220>
<223> GM-CSF E21R mutant polypeptide

<400> 2

Ala Pro Ala Arg Ser Pro Ser Pro Ser Thr Gln Pro Trp Glu His Val
1 5 10 15

Asn Ala Ile Gln Arg Ala Arg Arg Leu Leu Asn Leu Ser Arg Asp Thr
20 25 30

Ala Ala Glu Met Asn Glu Thr Val Glu Val Ile Ser Glu Met Phe Asp
35 40 45

Leu Gln Glu Pro Thr Cys Leu Gln Thr Arg Leu Glu Leu Tyr Lys Gln
50 55 60

Gly Leu Arg Gly Ser Leu Thr Lys Leu Lys Gly Pro Leu Thr Met Met
65 70 75 80

Ala Ser His Tyr Lys Gln His Cys Pro Pro Thr Pro Glu Thr Ser Cys
85 90 95

Ala Thr Gln Ile Ile Thr Phe Glu Ser Phe Lys Glu Asn Leu Lys Asp
100 105 110

Phe Leu Leu Val Ile Pro Phe Asp Cys Trp Glu Pro Val Gln Glu
115 120 125

<210> 3
<211> 127
<212> PRT
<213> Artificial

<220>
<223> GM-CSF E21K mutant polypeptide

<400> 3

Ala Pro Ala Arg Ser Pro Ser Pro Ser Thr Gln Pro Trp Glu His Val
1 5 10 15

Asn Ala Ile Gln Lys Ala Arg Arg Leu Leu Asn Leu Ser Arg Asp Thr
20 25 30

Ala Ala Glu Met Asn Glu Thr Val Glu Val Ile Ser Glu Met Phe Asp
35 40 45

Leu Gln Glu Pro Thr Cys Leu Gln Thr Arg Leu Glu Leu Tyr Lys Gln
50 55 60

Gly Leu Arg Gly Ser Leu Thr Lys Leu Lys Gly Pro Leu Thr Met Met
65 70 75 80

Ala Ser His Tyr Lys Gln His Cys Pro Pro Thr Pro Glu Thr Ser Cys
85 90 95

Ala Thr Gln Ile Ile Thr Phe Glu Ser Phe Lys Glu Asn Leu Lys Asp
100 105 110

Phe Leu Leu Val Ile Pro Phe Asp Cys Trp Glu Pro Val Gln Glu
115 120 125

<210> 4
<211> 127
<212> PRT
<213> Artificial

<220>
<223> GM-CSF E21H mutant polypeptide

<400> 4

Ala Pro Ala Arg Ser Pro Ser Pro Ser Thr Gln Pro Trp Glu His Val
1 5 10 15

Asn Ala Ile Gln His Ala Arg Arg Leu Leu Asn Leu Ser Arg Asp Thr
20 25 30

Ala Ala Glu Met Asn Glu Thr Val Glu Val Ile Ser Glu Met Phe Asp
35 40 45

Leu Gln Glu Pro Thr Cys Leu Gln Thr Arg Leu Glu Leu Tyr Lys Gln
50 55 60

Gly Leu Arg Gly Ser Leu Thr Lys Leu Lys Gly Pro Leu Thr Met Met
65 70 75 80

Ala Ser His Tyr Lys Gln His Cys Pro Pro Thr Pro Glu Thr Ser Cys
85 90 95

Ala Thr Gln Ile Ile Thr Phe Glu Ser Phe Lys Glu Asn Leu Lys Asp
100 105 110

Phe Leu Leu Val Ile Pro Phe Asp Cys Trp Glu Pro Val Gln Glu
115 120 125

<210> 5
<211> 127
<212> PRT
<213> Artificial

<220>
<223> GM-CSF E21D mutant polypeptide

<400> 5

Ala Pro Ala Arg Ser Pro Ser Pro Ser Thr Gln Pro Trp Glu His Val
1 5 10 15

Asn Ala Ile Gln Asp Ala Arg Arg Leu Leu Asn Leu Ser Arg Asp Thr
20 25 30

Ala Ala Glu Met Asn Glu Thr Val Glu Val Ile Ser Glu Met Phe Asp
35 40 45

Leu Gln Glu Pro Thr Cys Leu Gln Thr Arg Leu Glu Leu Tyr Lys Gln
50 55 60

Gly Leu Arg Gly Ser Leu Thr Lys Leu Lys Gly Pro Leu Thr Met Met
65 70 75 80

Ala Ser His Tyr Lys Gln His Cys Pro Pro Thr Pro Glu Thr Ser Cys
85 90 95

Ala Thr Gln Ile Ile Thr Phe Glu Ser Phe Lys Glu Asn Leu Lys Asp
100 105 110

Phe Leu Leu Val Ile Pro Phe Asp Cys Trp Glu Pro Val Gln Glu
115 120 125

<210> 6
<211> 127
<212> PRT
<213> Artificial

<220>
<223> GM-CSF E21S mutant polypeptide

<400> 6

Ala Pro Ala Arg Ser Pro Ser Pro Ser Thr Gln Pro Trp Glu His Val
1 5 10 15

Asn Ala Ile Gln Ser Ala Arg Arg Leu Leu Asn Leu Ser Arg Asp Thr
20 25 30

Ala Ala Glu Met Asn Glu Thr Val Glu Val Ile Ser Glu Met Phe Asp
35 40 45

Leu Gln Glu Pro Thr Cys Leu Gln Thr Arg Leu Glu Leu Tyr Lys Gln
50 55 60

Gly Leu Arg Gly Ser Leu Thr Lys Leu Lys Gly Pro Leu Thr Met Met
65 70 75 80

Ala Ser His Tyr Lys Gln His Cys Pro Pro Thr Pro Glu Thr Ser Cys
85 90 95

Ala Thr Gln Ile Ile Thr Phe Glu Ser Phe Lys Glu Asn Leu Lys Asp
100 105 110

Phe Leu Leu Val Ile Pro Phe Asp Cys Trp Glu Pro Val Gln Glu
115 120 125

<210> 7
<211> 127
<212> PRT
<213> Artificial

<220>
<223> GM-CSF E21A mutant polypeptide

<400> 7

Ala Pro Ala Arg Ser Pro Ser Pro Ser Thr Gln Pro Trp Glu His Val
1 5 10 15

Asn Ala Ile Gln Ala Ala Arg Arg Leu Leu Asn Leu Ser Arg Asp Thr
20 25 30

Ala Ala Glu Met Asn Glu Thr Val Glu Val Ile Ser Glu Met Phe Asp
35 40 45

Leu Gln Glu Pro Thr Cys Leu Gln Thr Arg Leu Glu Leu Tyr Lys Gln
50 55 60

Gly Leu Arg Gly Ser Leu Thr Lys Leu Lys Gly Pro Leu Thr Met Met
65 70 75 80

Ala Ser His Tyr Lys Gln His Cys Pro Pro Thr Pro Glu Thr Ser Cys
85 90 95

Ala Thr Gln Ile Ile Thr Phe Glu Ser Phe Lys Glu Asn Leu Lys Asp
100 105 110

Phe Leu Leu Val Ile Pro Phe Asp Cys Trp Glu Pro Val Gln Glu
115 120 125

<210> 8
<211> 127
<212> PRT
<213> Artificial

<220>
<223> GM-CSF E21F mutant polypeptide

<400> 8

Ala Pro Ala Arg Ser Pro Ser Pro Ser Thr Gln Pro Trp Glu His Val
1               5                   10                  15

Asn Ala Ile Gln Phe Ala Arg Arg Leu Leu Asn Leu Ser Arg Asp Thr
            20                  25                  30

Ala Ala Glu Met Asn Glu Thr Val Glu Val Ile Ser Glu Met Phe Asp
        35                  40                  45

Leu Gln Glu Pro Thr Cys Leu Gln Thr Arg Leu Glu Leu Tyr Lys Gln
    50                  55                  60

Gly Leu Arg Gly Ser Leu Thr Lys Leu Lys Gly Pro Leu Thr Met Met
65                  70                  75                  80

Ala Ser His Tyr Lys Gln His Cys Pro Pro Thr Pro Glu Thr Ser Cys
                85                  90                  95

Ala Thr Gln Ile Ile Thr Phe Glu Ser Phe Lys Glu Asn Leu Lys Asp
            100                 105                 110

Phe Leu Leu Val Ile Pro Phe Asp Cys Trp Glu Pro Val Gln Glu
        115                 120                 125

<210> 9
<211> 87
<212> DNA
<213> Homo sapiens

<400> 9
gagcccaaat cttgtgacaa aactcacaca tgcccaccgt gtgatcccgc cgagcccaaa 60

tctcctgaca aaactcacac atgccca 87

<210> 10
<211> 29
<212> PRT
<213> Homo sapiens

<400> 10

Glu Pro Lys Ser Cys Asp Lys Thr His Thr Cys Pro Pro Cys Asp Pro
1               5                   10                  15

Ala Glu Pro Lys Ser Pro Asp Lys Thr His Thr Cys Pro
            20                  25

<210> 11
<211> 339

<212> DNA
<213> Homo sapiens

<400> 11
ccgtgcccag cacctgaact cctgggggga ccgtcagtct tcctcttccc cccaaaaccc 60

aaggacaccc tcatgatctc ccggacccct gaggtcacat gcgtggtggt ggacgtgagc 120

cacgaagacc ctgaggtcaa gttcaactgg tacgtggacg gcgtggaggt gcataatgcc 180

aagacaaagc cgcgggagga gcagtacaac agcacgtacc gtgtggtcag cgtcctcacc 240

gtcctgcacc aggactggct gaatggcaag gagtacaagt gcaaggtctc caacaaagcc 300

ctcccagccc ccatcgagaa aaccatctcc aaagccaaa 339

<210> 12
<211> 113
<212> PRT
<213> Homo sapiens

<400> 12

Pro Cys Pro Ala Pro Glu Leu Leu Gly Gly Pro Ser Val Phe Leu Phe
1 5 10 15

Pro Pro Lys Pro Lys Asp Thr Leu Met Ile Ser Arg Thr Pro Glu Val
20 25 30

Thr Cys Val Val Val Asp Val Ser His Glu Asp Pro Glu Val Lys Phe
35 40 45

Asn Trp Tyr Val Asp Gly Val Glu Val His Asn Ala Lys Thr Lys Pro
50 55 60

Arg Glu Glu Gln Tyr Asn Ser Thr Tyr Arg Val Val Ser Val Leu Thr
65 70 75 80

Val Leu His Gln Asp Trp Leu Asn Gly Lys Glu Tyr Lys Cys Lys Val
85 90 95

Ser Asn Lys Ala Leu Pro Ala Pro Ile Glu Lys Thr Ile Ser Lys Ala
100 105 110

Lys

<210> 13
<211> 321
<212> DNA
<213> Homo sapiens

<400> 13
gggcagcccc gagaaccaca ggtgtacacc ctgcccccat cccgggatga gctgaccaag 60

aaccaggtca gcctgacctg cctggtcaaa ggcttctatc ccagcgacat cgccgtggag 120

tgggagagca atgggcaacc ggagaacaac tacaagacca cgcctcccgt gctggactcc 180

gacggctcct tcttcctcta cagcaagctc accgtggaca agagcaggtg gcagcagggg 240

aacgtcttct catgctccgt gatgcatgag gctctgcaca accactacac gcagaagagc 300

ctctccctgt ctccgggtaa a 321

<210> 14
<211> 107
<212> PRT
<213> Homo sapiens

<400> 14

Gly Gln Pro Arg Glu Pro Gln Val Tyr Thr Leu Pro Pro Ser Arg Asp
1 5 10 15

Glu Leu Thr Lys Asn Gln Val Ser Leu Thr Cys Leu Val Lys Gly Phe
20 25 30

Tyr Pro Ser Asp Ile Ala Val Glu Trp Glu Ser Asn Gly Gln Pro Glu
35 40 45

Asn Asn Tyr Lys Thr Thr Pro Pro Val Leu Asp Ser Asp Gly Ser Phe
50 55 60

Phe Leu Tyr Ser Lys Leu Thr Val Asp Lys Ser Arg Trp Gln Gln Gly
65 70 75 80

Asn Val Phe Ser Cys Ser Val Met His Glu Ala Leu His Asn His Tyr
85 90 95

Thr Gln Lys Ser Leu Ser Leu Ser Pro Gly Lys
100 105

<210> 15
<211> 15
<212> PRT
<213> Artificial

<220>
<223> (G4S)3 sequence

<400> 15

Gly Gly Gly Gly Ser Gly Gly Gly Gly Ser Gly Gly Gly Gly Ser
1 5 10 15

<210> 16
<211> 81

<212> DNA
<213> Homo sapiens

<400> 16
ttttgggtgc tggtggtggt tggtggagtc ctggcttgct atagcttgct agtaacagtg 60

gcctttatta ttttctgggt g 81

<210> 17
<211> 27
<212> PRT
<213> Homo sapiens

<400> 17

Phe Trp Val Leu Val Val Val Gly Gly Val Leu Ala Cys Tyr Ser Leu
1 5 10 15

Leu Val Thr Val Ala Phe Ile Ile Phe Trp Val
20 25

<210> 18
<211> 123
<212> DNA
<213> Homo sapiens

<400> 18
aggagtaaga ggagcaggct cctgcacagt gactacatga acatgactcc ccgccgcccc 60

gggcccaccc gcaagcatta ccagccctat gccccaccac gcgacttcgc agcctatcgc 120

tcc 123

<210> 19
<211> 41
<212> PRT
<213> Homo sapiens

<400> 19

Arg Ser Lys Arg Ser Arg Leu Leu His Ser Asp Tyr Met Asn Met Thr
1 5 10 15

Pro Arg Arg Pro Gly Pro Thr Arg Lys His Tyr Gln Pro Tyr Ala Pro
20 25 30

Pro Arg Asp Phe Ala Ala Tyr Arg Ser
35 40

<210> 20
<211> 339
<212> DNA
<213> Homo sapiens

<400> 20
agagtgaagt tcagcaggag cgcagacgcc cccgcgtacc agcagggcca gaaccagctc 60

tataacgagc tcaatctagg acgaagagag gagtacgatg ttttggacaa gagacgtggc 120

cgggaccctg agatgggggg aaagccgaga aggaagaacc ctcaggaagg cctgtacaat 180

gaactgcaga aagataagat ggcggaggcc tacagtgaga ttgggatgaa aggcgagcgc 240

cggaggggca aggggcacga tggcctttac cagggtctca gtacagccac caaggacacc 300

tacgacgccc ttcacatgca ggccctgccc cctcgctaa 339

<210> 21
<211> 112
<212> PRT
<213> Homo sapiens

<400> 21

Arg Val Lys Phe Ser Arg Ser Ala Asp Ala Pro Ala Tyr Gln Gln Gly
1 5 10 15

Gln Asn Gln Leu Tyr Asn Glu Leu Asn Leu Gly Arg Arg Glu Glu Tyr
20 25 30

Asp Val Leu Asp Lys Arg Arg Gly Arg Asp Pro Glu Met Gly Gly Lys
35 40 45

Pro Arg Arg Lys Asn Pro Gln Glu Gly Leu Tyr Asn Glu Leu Gln Lys
50 55 60

Asp Lys Met Ala Glu Ala Tyr Ser Glu Ile Gly Met Lys Gly Glu Arg
65 70 75 80

Arg Arg Gly Lys Gly His Asp Gly Leu Tyr Gln Gly Leu Ser Thr Ala
85 90 95

Thr Lys Asp Thr Tyr Asp Ala Leu His Met Gln Ala Leu Pro Pro Arg
100 105 110

<210> 22
<211> 800
<212> DNA
<213> Homo sapiens

<220>
<221> CDS
<222> (33)..(467)

<220>
<221> sig_peptide
<222> (33)..(83)

<220>
<221> mat_peptide

<222> (84)..(464)

<400> 22

```
acacagagag aaaggctaaa gttctctgga gg atg tgg ctg cag agc ctg ctg        53
                                    Met Trp Leu Gln Ser Leu Leu
                                            -15

ctc ttg ggc act gtg gcc tgc agc atc tct gca ccc gcc cgc tcg ccc       101
Leu Leu Gly Thr Val Ala Cys Ser Ile Ser Ala Pro Ala Arg Ser Pro
-10                 -5                  -1  1               5

agc ccc agc acg cag ccc tgg gag cat gtg aat gcc atc cag gag gcc       149
Ser Pro Ser Thr Gln Pro Trp Glu His Val Asn Ala Ile Gln Glu Ala
            10                  15                  20

cgg cgt ctc ctg aac ctg agt aga gac act gct gct gag atg aat gaa       197
Arg Arg Leu Leu Asn Leu Ser Arg Asp Thr Ala Ala Glu Met Asn Glu
        25                  30                  35

aca gta gaa gtc atc tca gaa atg ttt gac ctc cag gag ccg acc tgc       245
Thr Val Glu Val Ile Ser Glu Met Phe Asp Leu Gln Glu Pro Thr Cys
    40                  45                  50

cta cag acc cgc ctg gag ctg tac aag cag ggc ctg cgg ggc agc ctc       293
Leu Gln Thr Arg Leu Glu Leu Tyr Lys Gln Gly Leu Arg Gly Ser Leu
55                  60                  65                  70

acc aag ctc aag ggc ccc ttg acc atg atg gcc agc cac tac aag cag       341
Thr Lys Leu Lys Gly Pro Leu Thr Met Met Ala Ser His Tyr Lys Gln
                75                  80                  85

cac tgc cct cca acc ccg gaa act tcc tgt gca acc cag att atc acc       389
His Cys Pro Pro Thr Pro Glu Thr Ser Cys Ala Thr Gln Ile Ile Thr
            90                  95                  100

ttt gaa agt ttc aaa gag aac ctg aag gac ttt ctg ctt gtc atc ccc       437
Phe Glu Ser Phe Lys Glu Asn Leu Lys Asp Phe Leu Leu Val Ile Pro
        105                 110                 115

ttt gac tgc tgg gag cca gtc cag gag tgagaccggc cagatgaggc             484
Phe Asp Cys Trp Glu Pro Val Gln Glu
    120                 125

tggccaagcc ggggagctgc tctctcatga aacaagagct agaaactcag gatggtcatc     544

ttggagggac caaggggtgg gccacagcca tggtgggagt ggcctggacc tgccctgggc     604

cacactgacc ctgatacagg catggcagaa gaatgggaat attttatact gacagaaatc     664

agtaatattt atatatttat atttttaaaa tatttattta tttatttatt taagttcata     724

ttccatattt attcaagatg ttttaccgta ataattatta ttaaaaatat gcttctactt     784

gaaaaaaaaa aaaaaa                                                     800
```

<210> 23
<211> 28
<212> DNA
<213> Artificial

<220>
<223> forward primer for producing E21R

<400> 23
agagcccggc gtctcctgaa cctgagta 28

<210> 24
<211> 28
<212> DNA
<213> Artificial

<220>
<223> forward primer for producing E21H

<400> 24
cacgcccggc gtctcctgaa cctgagta 28

<210> 25
<211> 28
<212> DNA
<213> Artificial

<220>
<223> forward primer for producing E21S

<400> 25
agcgcccggc gtctcctgaa cctgagta 28

<210> 26
<211> 30
<212> DNA
<213> Artificial

<220>
<223> forward primer for producing E21A

<400> 26
gccgcccggc gtctcctgaa cctgagtaga 30

<210> 27
<211> 25
<212> DNA
<213> Artificial

<220>
<223> reverse primer for mutation

<400> 27
ctggatggca ttcacatcga gggtc 25

<210> 28
<211> 30
<212> DNA
<213> Artificial

<220>
<223> forward primer for constructing GMR.CAR dCH2CH3

<400> 28
ttttgggtgc tggtggtggt tggtggagtc 30

<210> 29
<211> 28
<212> DNA
<213> Artificial

<220>
<223> reverse primer for constructing GMR.CAR dCH2CH3

<400> 29
tgggcatgtg tgagttttgt caggagat 28

<210> 30
<211> 70
<212> DNA
<213> Artificial

<220>
<223> forward primer for constructing GMR.CAR dCH2+G4S3

<400> 30
ggtggtggtg gatccggcgg cggcggctcc ggtggtggtg gttctaaaga tcccaaattt 60

tgggtgctgg 70

<210> 31
<211> 34
<212> DNA
<213> Artificial

<220>
<223> reverse primer for constructing GMR.CAR dCH2+G4S3

<400> 31
tgggcatgtg tgagttttgt caggagattt gggc 34

<210> 32
<211> 24
<212> DNA
<213> Artificial

<220>
<223> forwared primer for producing E21R

<400> 32
agagcccggc gtctcctgaa cctg 24

<210> 33
<211> 27
<212> DNA
<213> Artificial

<220>
<223> reverse primer for producing E21R/E21K

<400> 33
ctggatggca ttcacatgct cccaggg 27

<210> 34
<211> 24
<212> DNA
<213> Artificial

<220>
<223> forwared primer for producing E21K

<400> 34
aaggcccggc gtctcctgaa cctg 24